(19) Europäisches Patentamt / European Patent Office / Office européen des brevets

(11) **EP 2 569 632 B1**

(12) **EUROPEAN PATENT SPECIFICATION**

(45) Date of publication and mention
of the grant of the patent:
**15.08.2018 Bulletin 2018/33**

(21) Application number: **11781379.0**

(22) Date of filing: **13.05.2011**

(51) Int Cl.:
*G01N 33/569* (2006.01)   *C07K 16/14* (2006.01)

(86) International application number:
**PCT/US2011/036518**

(87) International publication number:
**WO 2011/143613 (17.11.2011 Gazette 2011/46)**

(54) **YEAST CELL WALL COMPONENTS AND DETECTION THEREOF**

HEFE-ZELLWAND-KOMPONENTEN UND DEREN ERKENNUNG

COMPOSANTS DE PAROI CELLULAIRE DE LEVURES ET PROCÉDÉ DE DÉTECTION DE CEUX-CI

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.05.2010 US 334995 P**

(43) Date of publication of application:
**20.03.2013 Bulletin 2013/12**

(73) Proprietor: **Alltech, Inc.
Nicholasville, KY 40345 (US)**

(72) Inventors:
• **MORAN, Colm**
**13600 La Ciotat (FR)**
• **KWIATKOWSKI, Stefan**
**Lexington, KY 40511 (US)**
• **YIANNIKOURIS, Alexandros**
**Lexington, Kentucky 40513 (US)**
• **THIELEN, Ursula Anne**
**Lexington, Kentucky 40503 (US)**

(74) Representative: **Grünecker Patent- und
Rechtsanwälte
PartG mbB
Leopoldstraße 4
80802 München (DE)**

(56) References cited:
• **KWIATKOWSKI ET AL.: "A Study of
Saccharomyces cerevisiae Cell Wall Glucans.", J
INST BREWING, vol. 115, no. 2, 2009, pages
151-158, XP055082042,**
• **KWIATKOWSKI ET AL.: 'A Study of
Saccharomyces cerevisiae Cell Wall Glucans.' J
INST BREWING vol. 115, no. 2, 2009, pages 151 -
158, XP055082042**
• **BRUSSAARD: 'Novel ingredients protect animals
from mycotoxin.' FEED MIX vol. 16, no. 2, 2008,
pages 22 - 25, XP008167435**
• **OHNO ET AL.: 'Solubilization of yeast cell-wall
beta-(1->3)-D-glucan by sodium hypochlorite
oxidation and dimethyl sulfoxide extraction.'
CARBOHYDR RES vol. 316, no. 1-4, 31 March
1999, pages 161 - 172, XP004171909**
• **ZEKOVIC ET AL.: 'Mild Pfitzner--Moffat oxidation
of the (1->3)-beta-D-glucan from Saccharomyces
cerevisiae.' CHEMICAL PAPERS vol. 60, no. 3,
2006, pages 243 - 248, XP008167288**

**EP 2 569 632 B1**

## Description

This Application claims priority to United States Provisional Patent Application Serial No. 61/334,995 filed May 14, 2010.

## FIELD OF THE INVENTION

The invention relates to methods for the immunological detection of yeast cell components.

## BACKGROUND OF THE INVENTION

In the field of animal nutrition, a wide variety of additives and supplements have been employed to improve livestock performance. In some cases, yeast cell wall components find use as animal feed additives. One example is the feed additive MYCOSORB(manufactured by ALLTECH, Inc.). MYCOSORB binds mycotoxin contaminants in food and feed, thereby sequestering the mycotoxins in an indigestible state. Such mycotoxin contaminants commonly occur due to both field and postharvest fungal growth in grains, grain byproducts, and maize silage used as feedstock components. While raw and finished feed components sold commercially undergo required monitoring for mycotoxins, no monitoring system is 100% effective. Worldwide, it is estimated that approximately 25% of all crops are affected by mycotoxin contamination (Council for Agricultural Science and Technology (1989) "Mycotoxins: Economics and Health Risks", Task Force Report No. 116, Ames, IA), and incidences where contaminated materials enter the livestock and companion animal feed supply chain are therefore nearly inevitable. Consequences of livestock consumption of mycotoxin-contaminated feed include depressed appetite, reduced growth, reduced reproductive function and milk output, suppressed immune system, impaired digestion, and in severe cases death. Therefore, mycotoxin sequestration has proven to be a useful strategy for reducing the hazards of naturally occurring toxins that adversely affect animal and human health.

Development of robust, specific analytical methods and kits for qualitative and quantitative detection of feed additives are complicated by the presence of many interfering compounds, and by the relatively low concentration of feed additives used in, feed ready for animal consumption. Kwiatkowski et al., Journal of the Institute of Brewing, vol. 115, no. 2, pages 151-158, 2009, describes various all wall glucans of Saccharomyces cerevisiae.

## SUMMARY OF THE INVENTION

The subject-matter of the invention is as defined in the appended claims.

The present invention relates to methods for the immunological detection of yest celle wall components. In some embodiments, a method of detecting a yeast cell wall component and/or fragment thereof in a sample (e.g., feed samples, food samples, or extracts thereof) is provided. Such a method finds use, for example, in determining the presence of food and feed additives comprised of yeast cell wall components (e.g., yeast glucan, yeast $(1\rightarrow4)$-$\alpha$-D-glucan, yeast $(1\rightarrow6)$-$\beta$-D-glucan, yeast $(1\rightarrow4)$-$\alpha$-/$(1\rightarrow6)$-$\beta$-D-glucan, MYCOSORB feed additive, BIOMOSS feed additive, ACTIGENfeed additive, and the like) in feed products (e.g., livestock feed, companion animal feed, or manufacturing intermediates thereof). Accordingly, this invention relates to a method for detecting a yeast cell wall in a sample, comprising:

- providing a sample;
- exposing said sample to a primary antibody capable of binding to an antigen selected from the group consisting of $(I\rightarrow4)$-$\alpha$-D-glucan, $(I\rightarrow6)$-$\beta$-D-glucan, and $(I\rightarrow4)$-$\alpha$-/$(I\rightarrow6)$-$\beta$-D-glucan thereby forming a primary antibody-antigen complex; and
- detecting the degree of binding between said primary antibody and said antigen using a secondary antibody.

In some disclosures, the present application relates to methods for extracting yeast cell wall components (e.g., yeast glucan, yeast $(1\rightarrow4)$-$\alpha$-D-glucan, yeast $(1\rightarrow6)$-$\beta$-D-glucan, yeast $(1\rightarrow4)$-$\alpha$-/$(1\rightarrow6)$-$\beta$-D-glucan, MYCOSORB feed additive, and the like) from feed products (e.g., livestock feed, companion animal feed, or manufacturing intermediates thereof). The application discloses antigens that find use in raising antibodies to specific yeast cell wall components (e.g., yeast $(1\rightarrow4)$-$\alpha$-D-glucan, yeast $(1\rightarrow6)$-$\beta$-D-glucan, yeast $(1\rightarrow4)$-$\alpha$-/$(1\rightarrow6)$-$\beta$-D-glucan, and/or conjugates thereof). In some disclosures, such antigens are conjugated to a carrier, e.g., a protein carrier (e.g., bovine serum albumin (BSA)). In some embodiments, the application discloses provided methods for activation of carbohydrates (e.g., carbohydrates comprising glucopyranose rings, glucans, yeast $(1\rightarrow4)$-$\alpha$-/$(1\rightarrow6)$-$\beta$-D-glucan) at C6-OH position(s). In some disclosures, such activation comprises mild oxidation (e.g., using dimethylsulfoxide/acetic anhydride). In some embodiments, the application discloses antibodies capable of recognizing yeast cell wall antigens (e.g., monoclonal or polyclonal antibodies capable of recognizing yeast (1→4)-α-D-glucan, yeast (1→6)-β-D-glucan, yeast (1→4)-α-/(1→6)-β-D-glucan, or conjugates thereof; monoclonal antibodies 513A161.1 or 513A431.1 raised against (1→4)-α-D-glucan/(1,6)-β-D-glucan/BSA antigen; polyclonal antibodies raised against (1→4)-α-D-glucan/(1→6)-β-D-glucan/BSA antigen; or purified, diluted, conjugated, and/or monospecific forms thereof). Further disclosures are kits comprising reagents for the detection of yeast cell wall components (e.g., yeast glucan, yeast (1→4)-α-D-glucan, yeast (1→6)-β-D-glucan, yeast (1→4)-α-/(1→6)-β-D-glucan, MYCOSORB feed additive, and the like) in samples (e.g., feed samples, food samples, or extracts thereof).

The sample is or is derived from a food or feed product. A food or feed product comprises any material(s) that are consumed (e.g., by an animal) that contributes energy and/or nutrients to the diet. Examples of feedstuffs include, but are not limited to, Total Mixed Ration (TMR), forage(s), pellet(s), concentrate(s), premix(es) coproduct(s), grain(s), distiller grain(s), molasses, fiber(s), fodder(s), grass(es), hay, kernel(s), leaves, meal, soluble(s), and supplement(s). Feed and foodstuffs are not limited by their physical form. Feed and foodstuffs may be processed to smaller particle size (e.g., chipped, chopped, ground, milled, or the like); made into liquid form (e.g., extracted, boiled, concentrated, converted to syrup or other viscous form); or processed into larger size (e.g., baled, consolidated, compressed, or formed into composite shapes, e.g., pellets, blocks, squares, flakes, and the like).

The present invention is not limited by the type or nature of extraction method used to obtain samples for analysis. In some embodiments, original material to be analyzed (e.g., a food or feed product; a feedstuff) is subjected to an extraction technique to obtain a sample for analysis. Extraction techniques include but are not limited to acid extraction, alkali extraction, extraction with organic solvent, extraction with buffer, extraction with salt, extraction with detergent, physical extraction (e.g., boiling, steam extraction, low-temperature extraction, grinding, and the like), or a combination thereof. In some embodiments, material to be analyzed (e.g., a food or feed product; a feedstuff) is extracted using a combination of organic solvent and acid solution. In some embodiments, material to be analyzed (e.g., a food or feed product; a feedstuff) is extracted using a solution of dimethylsulfoxide (DMSO) and hydrochloric acid (HCl).

The present invention is not limited by the amount of analyte (e.g., antigen, yeast cell wall component, yeast glucan, yeast (1→4)-α-D-glucan, yeast (1→6)-β-D-glucan, yeast (1→4)-α-(1→6)-β-D-glucan, MYCOSORB feed additive, and the like) present in the original sample. The amount of analyte may be less than 0.005 mg, 0.005-0.05 mg, 0.05-0.5 mg, 0.5-1 mg, 1-5 mg, 5-10 mg, 10-25 mg, 25 mg or more. The present invention is not limited by the amount of analyte (e.g., antigen, yeast cell wall component, yeast glucan, yeast (1→4)-α-D-glucan, yeast (1→6)-β-D-glucan, yeast (1→4)-α-/(1→6)-β-D-glucan, MYCOSORB feed additive, and the like) present in the original material to be tested. The amount of analyte detected may be less than 0.05 kg per ton, 0.05-0.5 kg per ton, 0.5-1 kg per ton, 1.0-2.0 kg per ton, 2.0-3.0 kg per ton, 3.0-4.0 kg per ton, 4.0-5.0 kg per ton, 5.0-6.0 kg per ton, 6.0-10.0 kg per ton, 10 kg per ton or more.

The present invention is not limited by the working concentration of antibodies used to detect an analyte (e.g., e.g., antigen, yeast cell wall component, yeast glucan, yeast (1-4)-α-D-glucan, yeast (1→6)-β-D-glucan, yeast (1→4)-α-/(1→6)-β-D-glucan, MYCOSORB feed additive, and the like). The working dilution of the antibodies (e.g., monoclonal or polyclonal antibodies capable of recognizing yeast (1→4)-α-D-glucan, yeast (1→6)-β-D-glucan, yeast 1→4)-α-/(1→6)-β-D-glucan, or conjugates thereof; monoclonal antibodies 513A161.1 or 513A431.1 raised against (1→4)-α-D-glucan/(1→6)-β-D-glucan/BSA antigen; polyclonal antibodies raised against (1→4)-α-D-glucan/(1→6)-β-D-glucan/BSA antigen; or purified, diluted, conjugated, and/or monospecific forms thereof) may be more dilute than 1:100,000; 1:50,000 to 1:100,000; 1:20,000 to 1:50,000; 1:10,000 to 1:20,000; 1:5,000 to 1:10,000; 1:1,000 to 1:5,000; 1:500 to 1:1,000; 1:100 to 1:500; 1:50 to 1:100; 1:10-1:50; 1:1 to 1:10; 2:1 to 1:1; or more concentrated than 2:1.

Further disclosesd is an immunogenic composition comprising yeast cell wall components. In some disclosures a yeast cell wall component comprises yeast glucan. In some disclosures, the glucan comprises yeast (1→4)-α-D-glucan, yeast (1→6)-β-D-glucan, yeast (1→4)-α-(1→6)-β-D-glucan, or conjugates or derivatives thereof. In some embodiments, the present invention provides yeast (1→6)-β-D-glucan, yeast (1→4)-α-/(1→6)-β-D-glucan conjugated to a carrier (e.g., a protein carrier). Carriers may facilitate immunogenicity and/or stability in the host animal. Examples of carriers include but are not limited to bovine serum albumin (BSA), keyhole limpet hemocyanin (KLH), ovalbumin (OVA), bovine thyroglobulin (THY), and duck hepatitis B core antigen (DHBcAg) (Gathuru et al. (2005) Vaccine 23:4727-4733). The disclosure is not limited by the position of linkage between the antigen and to a carrier. In some disclosures, the antigen (e.g., carbohydrate antigen, glucan, carbohydrate comprising glucopyranose ring(s), yeast (1→4)-α-D-glucan, yeast (1→6)-β-D-glucan, yeast (1→4)-α-/(1→6)-β-D-glucan) is conjugated to a carrier at one or more O-6 position(s) with respect to the antigen. In some disclosures, the antigen is yeast (1→4)-α-/(1→6)-β-D-glucan conjugated to BSA at a C6 position.

Antibodies used in the method of the present invention are not limited by the host species in which they are raised. Host species may be rat, mouse, guinea pig, hamster, rabbit, goat, sheep, chicken, donkey, horse, bovine, canine, feline, porcine, simian, human, or any other species. Antibodies used in a method of the present invention are not limited by the inoculation regime, antigen preparation method, or antigen delivery method used to raise the antibodies. Antigen may be presented in presence or absence of immune-stimulating agents (e.g., adjuvants), buffers, salts, solvents, solubility-enhancing compounds, or the like. The host species may be immunized with the antigen once; twice; three times; four times; five times, 5-10 times; 10-20 times; or more than 20 times. Antibodies used in a method of the present invention are not limited by clonality (e.g., monoclonal, polyclonal). Antibodies may be utilized in crude or purified form. Antibodies may be polyspecific or monospecific. In preferred embodiments, antibodies used in the method of the invention are capable of specific recognition of the antigen used to raise them. In some preferred embodiments, antibodies used in a method of the present invention are capable of specific recognition of $(1\rightarrow4)$-$\alpha$-D-glucan/$(1\rightarrow6)$-$\beta$-D-glucan extracted from yeast (e.g., *Saccharomyces cerevisiae*) cell walls. In some preferred embodiments, antibodies used in a method of the present invention comprise polyclonal antibodies. In some embodiments, monoclonal or polyclonal antibodies used in a method of the invention are capable of recognizing yeast $(1\rightarrow4)$-$\alpha$-D-glucan, yeast $(1\rightarrow6)$-$\beta$-D-glucan, yeast $(1\rightarrow4)$-$\alpha$-/$(1\rightarrow6)$-$\beta$-D-glucan, or fragments, variants, or conjugates thereof. In some disclosures, antibody compositions bind to yeast cell wall $(1\rightarrow4)$-$\alpha$-D-glucan, but not $(1\rightarrow4)$-$\alpha$-D-glucan-containing moieties present in substances other than yeast cell wall $(1\rightarrow4)$-$\alpha$-D-glucan-containing polymers (e.g., yeast cell wall $(1\rightarrow4)$-$\alpha$-D-glucan, yeast cell wall $(1\rightarrow4)$-$\alpha$-/$(1\rightarrow6)$-$\beta$-D-glucan, or fragments, variants, or conjugates thereof). Further disclosed are, antibody compositions that bind to yeast cell wall $(1\rightarrow6)$-$\beta$-D-glucan, but not $(1\rightarrow6)$-$\beta$-D-glucan-containing moieties present in substances other than yeast cell wall $(1\rightarrow6)$-$\beta$-D-glucan-containing polymers (e.g., yeast cell wall yeast cell wall $(1\rightarrow6)$-$\beta$-D-gluean, yeast cell wall $(1\rightarrow4)$-$\alpha$-/$(1\rightarrow6)$-$\beta$-D-glucan, or fragments, variants, or conjugates thereof). In some disclosures, antibody compositions bind to yeast cell wall $(1\rightarrow4)$-$\alpha$-/$(1\rightarrow6)$-$\beta$-D-glucan, but not $(1\rightarrow4)$-$\alpha$-/$(1\rightarrow6)$-$\beta$-D-glucan-containing moieties present in substances other than yeast cell wall (e.g., yeast cell wall $(1\rightarrow4)$-$\alpha$-/$(1\rightarrow6)$-$\beta$-D-glucan, or fragments, variants, or conjugates thereof).

Some antigen detection methods of the present invention comprise immunodetection methods. In some preferred embodiments, immunodetection methods comprise ELISA. In some embodiments, an ELISA is conducted using a sample derived from an original material to be analyzed (e.g., a food or feed product; a feedstuff; an extract thereof) and a primary antibody (e.g., an antibody capable of recognizing a yeast cell wall component; an antibody capable of recognizing a yeast glucan; an antibody capable of recognizing yeast $(1\rightarrow4)$-$\alpha$-D-glucan, yeast $(1\rightarrow6)$-$\beta$-D-glucan, and/or yeast $(1\rightarrow4)$-$\alpha$-/$(1\rightarrow6)$-$\beta$-D-glucan. In some embodiments, the antibody is directly linked to a substance capable of generating a detectible signal (e.g., a chromogenic substance, a fluorogenic substance, a radiolabelled isotope, etc.). In some embodiments, the antibody directly or indirectly associates with another agent capable of generating a detectable signal (e.g., a chromogenic substance, a fluorogenic substance, a radio label, an isotope, etc.).

The application discloses kits for the detection of an analyte (e.g., an antigen, a yeast cell wall component, a yeast glucan, yeast $(1\rightarrow4)$-$\alpha$-D-glucan, yeast $(1\rightarrow6)$-$\beta$-D-glucan, and/or yeast $(1\rightarrow4)$-$\alpha$-/$(1\rightarrow6)$-$\beta$-D-glucan) in a sample (e.g., a food or feed product, a feedstuff, an extract thereof). Kit components may include, but are not limited to, reagents, extraction buffers, solvents, detergents, blocking agents, tubes, antibodies, standards, instructions, and any combination thereof.

In some dislosures, the application provides services in which information about the concentration of an analyte (e.g., an antigen, a yeast cell wall component, a yeast glucan, yeast $(1\rightarrow4)$-$\alpha$-D-glucan, yeast $(1\rightarrow6)$-$\beta$-D-glucan, and/or yeast $(1\rightarrow4)$-$\alpha$-/$(1\rightarrow6)$-$\beta$-D-glucan) is obtained for an original material to be analyzed (e.g., a food or feed product, a feedstuff, an extract thereof). In some disclosures, the analysis is conducted by an end user (e.g., a farmer, a livestock owner, a livestock handler, a livestock breeder, a companion animal owner, a companion animal handler, a companion animal breeder, a veterinarian, a feed or food product manufacturer, a feed or food product distributor, a regulatory official). In some disclosures, sample or original material is submitted to a third party by an end user (e.g., a farmer, a livestock owner, a livestock handler, a livestock breeder, a companion animal owner, a companion animal handler, a companion animal breeder, a veterinarian, a feed or food product manufacturer, a feed or food product distributor, a regulatory official) and the third party conducts the analysis. In some disclosures, the third party communicates the information regarding the analysis results to an end user (e.g., a farmer, a livestock owner, a livestock handler, a livestock breeder, a companion animal owner, a companion animal handler, a companion animal breeder, a veterinarian, a feed or food product manufacturer, a feed or food product distributor, a regulatory official). In some disclosures, the third party transmits information about the results of the analysis to a fourth party.

Additional embodiments and disclosures will be apparent to persons skilled in the relevant art based on the teachings contained herein.

## DESCRIPTION OF THE DRAWINGS

Figure 1 shows a schematic diagram of the production of the glucan fractions P1, P2, P3 and S1, S2, and S3 (see, e.g., Example 1).

Figure 2 shows the response of the original rabbit antisera and four supernatants of affinity-purified antisera. Affinity-purified antisera was obtained from separation of $\beta$-$(1,6)$-glucan specific (Gab 1-Gab4) antibodies to micro-well plates coated with BSA (see, e.g., Example 3).

Figure 3 shows a calibration curve constructed with the use of purified anti-$(1\rightarrow4)$-$\alpha$-/$(1\rightarrow6)$-$\beta$-D-glucan-BSA poly-

clonal rabbit antibodies generated using methods described herein (see, e.g., Examples 2 and 3).

Figure 4 shows an average standard curve from 5 assays performed with 6 inclusion levels of MYCOSORB in dairy feed material. The secondary y-axis is highlighting the Within-day (% CV intra) and Between-day (% CV $_{inter}$) repeatability precisions for the standard curves.

Figure 5 shows an average standard curve from 5 assays performed with 6 inclusion levels of MYCOSORB in chicken feed material. The secondary y-axis is highlighting the Within-day (% CV $_{intra}$) and Between-day (% CV $_{inter}$) repeatability precisions for the standard curves.

Figure 6 shows an average standard curve from 5 assays performed with 6 inclusion levels of MYCOSORB in pig feed material. The secondary y-axis is highlighting the Within-day (% CV $_{intra}$) and Between-day (% CV $_{inter}$) repeatability precisions for the standard curves.

Figure 7 shows average optical density reading ($\Delta OD_{450}$) differences of the interfering product subtracted from the MYCOSORB product signal obtained in chicken feed for a multiple microtiter plate distribution (Example 7). The interferential products were added at 50, 100, 200% (w/w) in addition to the MYCOSORB product (100%, 1.0 kg/T).

Figure 8 shows average optical density reading ($\Delta OD_{450}$) differences of the interfering product subtracted from the MYCOSORB product signal obtained in chicken feed for a single microtiter plate distribution (Example 7). The interferential products were added at 50, 100, 200% (w/w) in addition to the MYCOSORB product (100%, 1.0 kg/T).

Figure 9 shows ELISA assay results indicating cross-reactivity of monoclonal antibody 513A161.1 at a concentration of 1:100 (top) or 1:200 (bottom).

Figure 10 shows ELISA assay results indicating cross-reactivity of monoclonal antibody 513A161.1 at a concentration of 1:500 (top) or 1:1000 (bottom).

Figure 11 shows ELISA assay results indicating cross-reactivity of monoclonal antibody 513A161.1 at a concentration of 1:1500 (top) or 1:2000 (bottom).

Figure 12 shows ELISA assay results indicating cross-reactivity of monoclonal antibody 513A431.1 at a concentration of 1:100 (top) or 1:200 (bottom).

Figure 13 shows ELISA assay results indicating cross-reactivity of monoclonal antibody 513A431.1 at a concentration of 1:500 (top) or 1:1000 (bottom).

Figure 14 shows ELISA assay results indicating cross-reactivity of monoclonal antibody 513A431.1 at a concentration of 1:1500 (top) or 1:2000 (bottom).

Figure 15 shows average ELISA assay absorbance readings and standard deviations of three yeast cell wall extract standards either plated undiluted or diluted 1:1 in PBS or PBS + 3% nonfat dry milk (Example 6).

Figure 16 shows a graph of feed extract standard curves for ELISA microplate coating time and temperature incubations described in Example 6.

## DEFINITIONS

To facilitate an understanding of the present invention, a number of terms and phrases are defined below:
As used herein, the terms "peptide," "polypeptide" and "protein" all refer to a primary sequence of amino acids that are joined by covalent "peptide linkages." In general, a peptide consists of a few amino acids, typically from 2-50 amino acids, and is shorter than a protein. The term "polypeptide" encompasses peptides and proteins. In some embodiments, the peptide, polypeptide or protein is synthetic, while in other embodiments, the peptide, polypeptide or protein are recombinant or naturally occurring. A synthetic peptide is a peptide that is produced by artificial means in vitro (i.e., was not produced in vivo).

The term "glycoprotein(s)" or "glycopeptides(s)" refers to a protein or peptide which contains one or more carbohydrate residues covalently attached to the polypeptide chain. The term "selenoprotein(s)" or "selenopeptide(s)" refers to a protein or peptide which contains one or more selenium atoms. Typically, selenium atoms are incorporated into proteins within selenuim-containing amino acids including selenocysteine and selenomethionine.

The term "selenoglycoprotein(s)," "selenoglycopeptide(s)" or "SGP(s)" refers to a glycoprotein or glycopeptides which incorporate one or more selenium atoms. Typically, "selenoglycoproteins" comprise one or more selenium-containing amino acids. "Selenoglycoproteins" may comprise a number of carbohydrates in any number of different forms.

The terms "sample" and "specimen" are used in their broadest sense and encompass samples or specimens obtained from any source. As used herein, the term "sample" is used to refer to biological samples obtained from animals (including humans), and encompasses fluids, solids, tissues, and gases. In some embodiments of this invention, samples include samples containing plant-derived matter (silage, grain, processed livestock feed, feed products in intermediate stages of manufacture). However, these examples are not to be construed as limiting the types of samples that find use with the present invention.

As used herein, the term "yeast" and "yeast cells" refers to eukaryotic microorganisms classified in the kingdom Fungi, having a cell wall, cell membrane and intracellular components. Yeasts do not form a specific taxonomic or phylogenetic grouping. Currently about 1,500 species are known; it is estimated that only 1% of all yeast species have been described. The term "yeast" is often taken as a synonym for *S. cerevisiae,* but the phylogenetic diversity of yeasts is shown by their placement in both divisions Ascomycota and Basidiomycota. The term "yeast" encompasses brewer's yeast, distillers yeast and bakers yeasts. The budding yeasts ("true yeasts") are classified in the order Saccharomycetales. Most species of yeast reproduce asexually by budding, although some reproduce by binary fission. Yeasts are unicellular, although some species become multicellular through the formation of a string of connected budding cells known as *pseudohyphae,* or *false hyphae.* Yeast size can vary greatly depending on the species, typically measuring 3 4 μm in diameter, although some yeast can reach over 40 μm. As used herein, the term "yeast cell wall" also referred to as "YCW" refers to the cell wall of a yeast organism that surrounds the plasmic membrane and the intracellular components of the yeast. Yeast cell wall includes both the outer layer (mainly mannan) and the inner layer (mainly glucan and chitin) of the yeast cell wall. A function of the cell wall is to provide structure and protect the yeast interior (its metabolic activity center). Signaling and recognition pathways take place in the yeast cell wall. The composition of yeast cell wall varies from strain to strain and according to growth conditions of yeast.

As used herein, the term "yeast cell wall extract" refers to the yeast cell wall of yeast that has been ruptured or "lysed" (e.g., during a rupture and lysing stage) and separated from the soluble intracellular components of the yeast cell.

As used herein, the term w/w (weight/weight) refers to the amount of a given substance in a composition on weight basis. For example, an animal feed comprising 0.02% w/w dietary feed supplement of the invention means that the mass of the dietary feed supplement is 0.02% of the total mass of the animal feed (e.g., 200 grams of dietary feed supplement composition of the invention in 907,200 grams of animal feed).

As used herein, the term "purified" or "to purify" refers to the removal of components from a sample. For example, yeast cell walls or yeast cell wall extracts are purified by removal of non-yeast cell wall components (e.g., plasma membrane and/or yeast intracellular components); they are also purified by the removal of contaminants or agents other than yeast cell wall. The removal of non-yeast cell wall components and/or non-yeast cell wall contaminants results in an increase in the percent of yeast cell wall or components thereof in a sample. At the molecular level, the term "purified" refers to molecules (e.g., carbohydrates, glycoproteins) that are removed from their natural environment, isolated or separated. An "isolated carbohydrate" may therefore be a purified carbohydrate. "Substantially purified" molecules are at least 60% free, preferably at least 75% free, and more preferably at least 90% free from other components with which they are naturally associated. As used herein, the term "purified" or "to purify" also refers to the removal of contaminants from a sample. The removal of contaminating proteins results in an increase in the percent of polypeptide of interest in the sample. Further, recombinant polypeptides (e.g., glycoproteins) are expressed in or purified from plant, bacterial, yeast, or mammalian host cells and the polypeptides are purified by the removal of host cell proteins; the percent of recombinant polypeptides is thereby increased in the sample. As used herein, the term "animal" refers to those of kingdom Animalia. This includes, but is not limited to livestock, farm animals, domestic animals, companion or pet animals, marine and freshwater animals, and wild animals.

As used herein the term, "livestock" also referred to as "livestock species" and also referred to "domestic livestock" and also referred to as "commercially raised animals" refers to a domesticated animal intentionally reared in an agricultural or aquaculture setting to produce food or fiber, or for its labor or companionship.

As used herein, the terms "food supplement" "dietary supplement" "dietary supplement composition" and the like refer to a food product formulated as a dietary or nutritional supplement to be used as part of a diet, e.g. as an addition to animal feed. Exemplary dietary supplement compositions are described herein.

As used herein, the term "kit" is used in reference to a combination of reagents and other materials. It is contemplated that the kit may include reagents such as extraction solutions, antibodies, and detection reagents. It is not intended that the term "kit" be limited to a particular combination of reagents and/or other materials.

As used herein, the term "toxic" refers to any detrimental or harmful effects on a subject, a cell, or a tissue as compared to the same cell or tissue prior to the administration of the toxicant.

As used herein, the term "freeze-drying" and the term "lyophilization" and the term "cryodesiccation" refer to the removal of a solvent from matter in a frozen state by sublimation. This is accomplished by freezing the material to be dried below its eutectic point and then providing the latent heat of sublimation. Precise control of heat input permits drying from the frozen state without product melt-back. In practical application, the process is accelerated and precisely controlled under reduced pressure conditions.

As used herein, the term "dry free flowing powder" refers to a free flowing dry powder, e.g. a powder that can be poured from a container, bag, vessel etc without hindrance of large clumps.

As used herein, the term "grinding" refers to reducing particle size by impact, shearing, or attrition.

As used herein, the term "washing" refers to the removal or cleansing (e.g., using any type of solute (e.g. distilled water, buffer, or solvent) or mixture) of impurities or soluble unwanted component of a preparation (e.g., a yeast cell wall extract may be washed to remove non-yeast cell wall components from the sample; a well of a microtiter plate may be washed to remove non-binding or non-specifically binding components).

The terms "antibody" or "immunoglobulin" refer to proteins that bind a specific antigen. Immunoglobulins include, but are not limited to, polyclonal, monoclonal, chimeric, and humanized antibodies, Fab Fragments, F(ab')$_2$ fragments,

and includes immunoglobulins of the following classes: IgG, IgA, IgM, IgD, IbE, and secreted immunoglobulins (sIg). Immunoglobulins generally comprise two identical heavy chains and two light chains. However, the terms "antibody" and "immunoglobulin" also encompass single chain antibodies and two chain antibodies. Antibodies may be produced by any of the known methodologies (See, e.g., Current Protocols in Immunology (1998) John Wiley and Sons, Inc., N.Y.).

The term "antigen" refers to a carbohydrate, protein, glycoprotein, lipoprotein, lipid or other substance that is reactive with an antibody specific for a portion of the molecule.

As used herein, the term "analyte" refers to an atom, a molecule, a grouping of atoms and/or molecules, a substance, or chemical constituent. An analyte, in and of itself cannot be measured, rather, aspects or properties (physical, chemical, biological, etc.) of the analyte can be determined using an analytical procedure, such as HPLC or NMR. For example, one cannot measure a "chair" (analyte-component) in and of itself, but, the height, width, etc. of a chair can be measured. Likewise, one cannot measure a mycotoxin but can measure the mycotoxin signal (e.g., chromogenic signal, fluorescence signal) that is related to its concentration.

The terms "immunoprecipitate," "immunopurify," and "affinity purify," and grammatical variations such as verbs and adjectives, refer to the use of an antibody to separate its antigen or a portion thereof from a mixture of other molecules.

The term "immunodetection" and grammatical variations refers to the use of an antibody to identify the presence of an antigen or a portion thereof from a mixture of other molecules.

The term "staining" refers to as any number of processes known to those in the field that are used to better visualize, distinguish or identify a specific component(s) and/or feature(s) of a material (e.g., sample, feed sample, extraction of feed sample, cell, cells).

The term "immunofluorescence" refers to a staining technique used to identify, mark, label, visualize or make readily apparent by procedures known to those practiced in the art, where a ligand (usually an antibody) is bound to a receptor (usually an antigen) and such ligand, if an antibody, is conjugated to a fluorescent molecule, or the ligand is then bound by an antibody specific for the ligand, and said antibody is conjugated to a fluorescent molecule, where said fluorescent molecule can be visualized with the appropriate instrument (e.g., a fluorescent microscope).

The term "antigenic determinant" refers to that portion of an antigen (e.g., an epitope) that makes contact with a particular antibody. When a protein or fragment of a protein is used to immunize a host animal, numerous regions of the protein may induce the production of antibodies that bind specifically to a given region or three-dimensional structure on the protein; these regions or structures are referred to as antigenic determinants. An antigenic determinant may compete with the intact antigen (the "immunogen" used to elicit the immune response) for binding to an antibody.

As used herein, the term "immunoassay" refers to a qualitative or quantitative test intended to allow detection of an antigen in a sample. Immunoassays typically utilize antigen-recognizing antibodies. The antigen-recognizing antibody may be directly or indirectly coupled to a visualization step, such as a chromogenic or fluorogenic marker or enzyme. Examples of immunoassays include but are not limited to enzyme-linked immunosorbant assays ("ELISA"), lateral flow tests, western blots, microparticle-based assays (e.g., Luminex® assays), magnetic immunoassays, dot blots, enzyme immunoassays (EIA), radioimmunoassay (RIA), chemiluminescent immunoassays (CLIA), counting immunoassays (CIA), and the like. Immunoassays may be competitive or noncompetitive.

As used herein, the term "enzyme-linked immunosorbant assay" or "ELISA," sometimes referred to as a "sandwich assay," refers to a specific type of immunoassay. Typically, an unknown amount of antigen is absorbed or immobilized on a solid surface and exposed to an antibody capable of recognizing it. The quantity of bound antibody is generally determined through direct or indirect linkage to a fluorogenic or chromogenic enzyme. Types of ELISAs include but are not limited to direct ELISA, indirect ELISA, sandwich ELISA, competitive ELISA, and reverse ELISA. As used herein, the term "ELISA" refers to enzyme-linked immunosorbent assay (or EIA). Numerous ELISA methods and applications are known in the art, and are described in many references (see, e.g., Crowther, "Enzyme-Linked Immunosorbent Assay (ELISA)," in Molecular Biomethods Handbook, Rapley et al. (eds.), pp. 595-617, Humana Press, Inc., Totowa, N.J. (1998); Harlow and Lane (eds.), Antibodies: A Laboratory Manual, Cold Spring Harbor Laboratory Press (1988); Ausubel et al. (eds.), Current Protocols in Molecular Biology, Ch. 11, John Wiley & Sons, Inc., New York (1994)). In addition, there are numerous commercially available ELISA test systems.As used herein, the terms "food," "foodstuffs," "feed," "feed product," "feedstuffs" and the like refer to any material(s) that is(are) consumed (e.g., by an animal), that contributes energy and/or nutrients to the diet. Examples include, but are not limited to, Total Mixed Ration (TMR), forage(s), pellet(s), concentrate(s), premix(es) coproduct(s), grain(s), distiller grain(s), molasses, fiber(s), fodder(s), grass(es), hay, kernel(s), leaves, meal, soluble(s), and supplement(s). Foodstuffs are not limited by their physical form. Foodstuffs may be processed to smaller particle size (e.g., chipped, chopped, ground, milled, or the like); made into liquid form (e.g., extracted, boiled, concentrated, converted to syrup or other viscous form); or processed into larger size (e.g., baled, consolidated, compressed, or formed into composite shapes, e.g., pellets, blocks, squares, flakes, and the like).

As used herein, when used as a noun, an "additive" or "supplement" refers to a substance or thing that is added to or otherwise included into another substance or thing. Further, as used herein, when used as a noun, the words "additive" or "supplement" can be used interchangeably when referencing "additives" or "supplements" that are mixed into animal feed before being fed to an animal.

As used herein, the term "glucan" refers to any glucose-containing carbohydrate polymer. In some embodiments, glucans are polymers of D-glucose without limitation to degree of polymerization, covalent or noncovalent association with other polymers, or exact carbohydrate composition. Throughout the length of a glucan polymer, D-glucose units may participate in $\alpha$-linkages, $\beta$-linkages, or both $\alpha$- and $\beta$-linkages

As used herein, the term "carbohydrate" is used to reference an organic compound with the general formula $C_m(H_2O)_n$. A carbohydrate consists only of a carbon, , a hydrogen and an oxygen atom whereby the hydrogen and oxygen atoms are in 2:1 atom ratio.

As used herein, the term "signal" is used generally in reference to any detectable process that indicates that a reaction has occurred, for example, binding of antibody to antigen. It is contemplated that signals in the form of radioactivity, fluorimetric or colorimetric products/reagents will all find use with the present invention. In various embodiments of the present invention, the signal is assessed qualitatively, while in alternative embodiments, the signal is assessed quantitatively.

As used herein, the term "solid support" is used in reference to any solid or stationary material to which reagents such as antibodies, antigens, and other test components are attached. For example, in an ELISA method, the wells of microtiter plates provide solid supports. Other examples of solid supports include microscope slides, coverslips, beads, particles, cell culture flasks, as well as many other suitable items.

The terms "specific binding" and "specifically binding" when used in reference to the interaction between an antibody and an antigen describe an interaction that is dependent upon the presence of a particular structure (i.e., the antigenic determinant or epitope) on the antigen. In other words, the antibody recognizes and binds to a protein structure unique to the antigen, rather than binding to all proteins in general (i.e., non-specific binding).

## DETAILED DESCRIPTION OF THE INVENTION

Yeast cell wall (YCW) comprises a complex structure that includes two major polysaccharide components: $(1\rightarrow2)(1\rightarrow3)(1\rightarrow6)$-$\alpha$-D-mannan, and $(1\rightarrow3)(1\rightarrow6)$-$\beta$-D-glucan. These polysaccharides are connected to the YCW proteins through O- and N-glycosidyl bonds, as discussed in a recently published review (Lesage et al. (2006) Microbiol. Mol. Biol. Rev. 70:317-343). On an industrial scale, YCW is typically separated, or extracted from from lysed yeast organisms by centrifugation and washing as reported by D.A. Howes and K.E. Newman (sec, e.g., US Patent No. 6,045,834). At this stage, YCW is not soluble in water. Subsequent enzymatic and chemical treatment of the primary YCW modifies the structure of polysaccharides and proteins, thereby rendering these YCW components at least partially soluble in water and in dimethyl sulfoxide "DMSO". The composition of the soluble and insoluble parts of the product depends upon the conditions of the process.

Immunoassays comprise sensitive analytical techniques that find use with such complex, non-homogenous mixtures. In some method embodiments of the present invention, antibodies from an animal that has been immunized with an antigen containing yeast $(1\rightarrow6)$-$\beta$-D-glucan which interconnects all of the major structural components of the cell wall (see, Kollár et al. (1997) J. Biol. Chem. 272:17762-17775 recognize this polysaccharide, allowing establishment of robust and sensitive immunoassays. In some embodiments of the present invention, a quantitative immunoassay (ELISA) permits analysis of a YCW derived nutritional additive, MYCOSORB (ALLTECH, Inc.), in animal feed samples. An assay for $(1\rightarrow6)$-$\beta$-D-glucan from YCW is very specific in quantification of YCW, because $(1\rightarrow6)$-$\beta$-D-glucan is very rare among plant carbohydrates. Therefore, antibodies directed against this YCW polysaccharide do not significantly interact with plant polysaccharides present in animal feed, ensuring low assay background.

The application discloses The application disclosures methods for: 1) separation of a soluble $(1\rightarrow4)$-$\alpha$-/$(1\rightarrow6)$-$\beta$-D-glucan from yeast cell wall (see, Kwiatkowski et al. (2009) J. Instit. Brew. 115:1031, and Arvindekar et al. (2002) Yeast 19:131-139 ), e.g., as an antigen for preparation of antibodies recognizing yeast $(1\rightarrow4)$-$\alpha$-/$(1\rightarrow6)$-$\beta$-D-glucan; oxidation of this polysaccharide hydroxyl groups at C-6 position of glucopyranose rings with Pfizner-Moffat reagent (see, Pfizner et al. (1963) J. Am. Chem. Soc. 85:3027), in order to convert them into aldehyde group (see, Zekovic et al. (2006) Chem. Papers 60:243-248); and the use of this oxidized form of $(1\rightarrow4)$-$\alpha$-/$(1\rightarrow6)$-$\beta$-D-glucan in the reductive-amination reaction (see, Abdel-Magid et al. (1996) J. Org. Chem. 61:3849-3862) with the lysine residues of bovine serum albumin (BSA), to produce a polysaccharide-protein conjugate. The application discloses methods for separation and purification (e.g., using ion exchange, DEAE cellulose and $Na_2HPO_4$ buffers of different ionic strength) of $(1\rightarrow4)$-$\alpha$-/$(1\rightarrow6)$-$\beta$-D-glucan-BSA conjugate, which is used as a soluble immunogen in animal (e.g., rabbit) immunization (see, Howard et al. (2001) Basic Methods in Antibody Production and Characterization, CRC Press, pp. 11-18 and 31-50). Further disclosed are methods for preparation of BSA-coated affinity phase resin (see, Matejtschuk (1997) Affinity Separations: a Practical Approach, Oxford University Press) and its use in absorption of BSA-specific antibodies from rabbit sera containing both BSA-specific and $(1\rightarrow4)$-$\alpha$-/$(1\rightarrow6)$-$\beta$-D-glucan-specific antibodies. In some embodiments of the present invention, methods for conjugation of polysaccharides to proteins find use in the production of human and animal vaccines, as such methods preserve the original structure of the polysaccharide and do not modify its reducing end as typical conjugation methods do (see, U.S. Pat. No. 6,596,861);

*Saccharomyces cerevisae* **cell wall glucans**

Glucans are prevalent among *Saccharomyces cerevisiae* cell wall polysaccharides (see, Kwiatkowski et al. (2009) J. Instit. Brew. 115:151-158). The roles of (1→3)-β-D-glucan in maintenance of yeast cell wall shape and rigidity (see, Klis et al. (2006) Yeast 23:185-202; Lesage et al. (2006) Microbiol. Mol. Biol. Rev. 70:317-343) and the (1→6)-β-D-glucan as a polysaccharide that links together all of the cell wall polysaccharides are documented (see, Aimaniada et al. (2009) J. Biol. Chem. 284:13401-13412; Kollár et al. (1997) J. Biol. Chem. 272:17762-1777; Klis et al. (2002) FEMS Microbiology Rev. 26:239-256). The presence of soluble and insoluble glycogen-like (1→4)-α-D-glucan in the cells of *S. cerevisiae* grown aerobically was frequently mentioned in the early yeast literature (see, Gunja-Smith et al. (1974) Biochem. Biophys. Res. Com. 56:588-592; Grba et al. (1975) Appl. Microbiol. Biotechnol. 2:29-37), and two forms of glycogen synthetase have been identified (see, Gunja-Smith et al. (1977) J. Bacteriol. 130:818-825; Rothman-Dcnes et al. (1970) PNAS 66:967-974). Glycogen is the energy reserve carbohydrate accumulated by *S. cerevisiae,* which can be mobilized during periods of yeast starvation. It is a polymer of α-D-glucose with a molecular weight of $\sim 10^8$ and a branched structure with 10-14 residues of α-D-glucose joined by 1→4 linkages (see, Aklujkar et al. (2008) J. Instit. Brew. 114:205-208; Boulton et al. (2001) The Biochemistry of Fermentation. In: Brewing Yeast and Fermentation, Blackwell Science, Iowa, pp. 89-92).

The (1→4)-α-D-glucan content in the yeast cell wall can vary from as little as 1% (see, Lille et al. (1980) J. Bacteriol. 143:1384-1394) to as much as 29% (see, Sedmak et al., U.S. Pat. App. No. 2006/0263415) of the dry weight, depending upon the nutritional status of the cells, the method of isolation, the environmental conditions and the time the cells were harvested (see, Lille et al. (1980) J. Bacteriol. 143:1384-1394). The industrially produced brewer's yeast cells (see, Sedmak et al., U.S. Pat. App. No. 2006/0263415) contained glucans at a 28.9% dry weight (d.w.) concentration, which included 12.4% d.w. of (1→4)-α-D-glucan. When these cells were treated with alkaline protease, the water-insoluble cell wall component contained 54.5% d.w. of glucans and more than half of the weight of (1→4)-α-D-glucan (29.2% d.w.).

In 2002, Arvindekar and Patil (see, Arvindekar et al. (2002) Yeast 19:131-139) proposed an explanation for the presence of the insoluble fraction which has been defined by others as "difficult to wash away" yeast glycogen (see, Fleet et al. (1976) J. Gen. Microbiol. 94:180-192) within the yeast cell wall, based upon their finding that (1→4)-α-D-glucan is covalently bound to (1→6)-β-D-glucan. However, this was in discord with the 1997 paper published by Kollar and co-workers (see, Kollár et al. (1997) J. Biol. Chem. 272:17762-17775) and with work published by Aimaniada and co-workers (see, Aimaniada et al. (2009) J. Biol. Chem. 184:13401-13412) on the role and the structure of the (1→6)-β-D-glucan within the yeast cell wall. Notably, the findings of Arvindekar and Patil required structural verification, as the authors worked with minute fractions from an enzymatic hydrolysis of the yeast cell wall and did not use NMR for the quantification or structural assignments for their separated products. In experiments conducted during the course of developing embodiments of the invention, large scale separation methods were developed for isolation of mixed (1→4)-α-D-glucan/(1→6)-β-D-glucan polysaccharide from *S. cerevisiae* cell wall, and the structure of this product was established using NMR spectroscopy.

**Animal Food and Feed**

Animal feed is any foodstuff that is used specifically to feed domesticated livestock (e.g., cattle, goats, sheep, horses, poultry, buffalo, alpaca, llamas, donkeys, mules, rabbits, chickens, geese, turkeys, and pigs). Animal feeds often include hay, straw, silage, compressed and pelleted feeds, oils and mixed rations, and also sprouted grains and legumes. The worldwide animal feed industry consumed 635 million tons of feed in 2006, with an annual growth rate of about 2%. The use of agricultural land to grow feed rather than human food can be controversial; some types of feed, such as corn (maize), can also serve as human food, while others such as grass cannot. In addition to providing an energy source to animals, animal feeds also provide nutrients (e.g. selenium) utilized by the body. Animal feeds are often mixed with supplements and/or additives (e.g., MYCOSORB) prior to being fed to an animal.

**Immunodetection Methods**

The invention provides methods for detection of substances in samples, such methods comprising immunodetection assays or immunoassays. An immunoassay (immunodetection assay, immunodetection method) involves use of an antibody for the detection (visualization, qualitative identification, quantitative detection) of a substance (e.g., antigen) to which the antibody binds. An antigen recognized by an antibody may be a macromolcculc or a fragment thereof (e.g., polymer, carbohydrate, glycoprotein, protein, moiety or monomer thereof). For example, some methods of the present invention find use in the detection of yeast cell wall glucans (e.g., (1→4)-α-D-glucan, (1→6)-β-D-glucan, and/or (1→4)-α-/(1→6)-β-D-glucan).

Numerous immunoassays find use in the qualitative or quantitative detection of substances (e.g., substances found at low concentration, substances present in complex mixtures, carbohydrate substances, yeast (1→4)-α-D-glucan, yeast (1→6)-β-D-glucan, yeast (1→4)-α-/(1→6)-β-D-glucan). Types of immunoassays include but are not limited to enzyme-linked immunosorbant assays (ELISA), lateral flow tests, western blots, microparticle-based assays (e.g., Luminex® assays), magnetic immunoassays, dot blots, enzyme immunoassays (EIA), radioimmunoassay (RIA), chemiluminescent immunoassays (CLIA), counting immunoassays (CIA), and the like (see, e.g., Wild et al. (2005) "The Immunoassay Handbook, 3rd Ed.", Elsevier Ltd., Oxford, UK). Immunoassays may be competitive or noncompetitive.

**Enzyme-Linked Immunosorbant Assay (ELISA)**

Enzyme-Linked ImmunoSorbent Assay, "ELISA". has been used as a diagnostic tool in medicine and plant pathology, as well as a quality control check in various industries. In one example of a typical ELISA includes a probe molecule that is first immobilized on a polystyrene microplate or other surface. Next a blocking agent such as BSA is applied and incubated. A biological or other sample containing a specific target molecule (often a protein) of unknown concentration is made to come into contact with the immobilized probe molecule. If present, the target molecule is captured by the probe proportionally to the concentration of the target molecule. Next, the surface is typically washed with a mild detergent solution to remove any molecules that are not specifically bound. Next, an additional molecule, such as a second antibody, is applied to form a "sandwich" complex with the capture probe, target molecule, and labeled detector probe. The second molecule is often referred to as a detector probe or detector antibody, and is commonly covalently linked to an enzyme, hapten, or other labeling molecule.

After a final wash step the plate is developed by adding a conjugate that binds to the labeled detector antibody and contains an enzymatic substrate, fluorescently labeled detection reagent, or a variety of other reporters. The reporter produces a detectable signal proportional to the quantity of target antigen in the sample. Typically, ELISAs are read using a colorimetric or fluorescent plate reader and result in a single target analyte measurement per well. Other variants of the ELISA assay include but are not limited to indirect, competitive, or reverse ELISA (see, e.g., Crowther et al. (2008) "The ELISA Guidebook, 2nd Ed.", Humana Press).

In many cases, ELISAs are performed in microplates made to match a standardized format that enables processing via an automated instrument. These standards are established by the Society of Biomolecular Sciences (SBS) and are known as SBS standards. According to SBS standards, the "footprint" for a multiwell plate is approximately 85 mm×125 mm with wells located in a specified positions format depending upon the total number of wells. The American National Standards Institute (ANSI) has published the SBS Standards for microplates as: "Footprint Dimensions" (ANSI/SBS 1-2004), "Height Dimensions" (ANSI/SBS 2-2004), "Bottom Outside Flange Dimensions" (ANSI/SBS 3-2004) and "Well Positions" (ANSI/SBS 4-2004). Most commonly, ELISA users employ 96-wells in a single plate. Alternately, when less than 96-wells are needed in an assay, up to twelve 8-well "strips" can be employed such that only a portion of the 96-wells are used at a time.

**Testing Services**

In some embodiments, a computer-based analysis program is used to translate the raw data generated by an assay (e.g., immunoassay, ELISA) (e.g., the presence, absence, or amount of an antigen) (e.g., yeast (1→4)-α-D-glucan, yeast (1→6)-β-D-glucan, yeast (1→4)-α-/(1→6)-β-D-glucan) into data of predictive value for an end user (e.g., a livestock or companion animal breeder or handler, a veterinarian, a farmer, a consumer). The end user can access the predictive data using any suitable means. Thus, in some embodiments, the present invention provides the further benefit that the end user, who is not likely to be trained in immunoassay analysis, need not understand the raw data. The data is presented directly to the end user in its most useful form. The end user is then able to immediately utilize the information in order to optimize the care of the subject (e.g., livestock or companion animal).

The present invention contemplates any method capable of receiving, processing, and transmitting the information pertaining to samples to and from laboratories conducting the assays. For example, in some embodiments of the present invention, a sample (e.g., a feed sample, an extract of feed sample) is obtained and submitted to a profiling service (e.g., laboratory etc.), located in any part of the world (e.g., in a country matching that of or different than the country where the subject resides or where the information is ultimately used) to generate raw data. The end user may have the sample (e.g., feed extract) obtained by a third party and sent to the profiling center, or subjects may collect the sample themselves and directly send it to a profiling center. Where the sample comprises previously determined information, the information may be directly sent to the profiling service by the end user (e.g., an information card containing the information may be scanned by a computer and the data transmitted to a computer of the profiling center using an electronic communication systems). Once received by the profiling service, the sample is processed and a profile is produced (e.g., antigen content), specific for the diagnostic or prognostic information desired for the end user.

The profile data is then prepared in a format suitable for interpretation by an end user. For example, rather than providing raw data, the prepared format may represent a risk assessment (e.g., likelihood of antigen being present) for the end user, along with recommendations for particular livestock care options. The data may be displayed to the end user by any suitable method. For example, in some embodiments, the profiling service generates a report that can be printed for the end user (e.g., at the point of contact, at the point of livestock care) or displayed to the end user on a computer monitor.

In some embodiments, the information is first analyzed at the point of livestock care or at a regional facility. The raw data is then sent to a central processing facility for further analysis and/or to convert the raw data to information useful for an end user or other interested party. The central processing facility provides the advantage of privacy (all data is stored in a central facility with uniform security protocols), speed, and uniformity of data analysis. The central processing facility can then control the fate of the data following communication to the end user. For example, using an electronic communication system, the central facility can provide data to the end user.

In some embodiments, the end user is able to directly access the data using the electronic communication system. The end user may seek further advice based on the results. In some embodiments, the data is used for research use. For example, the data may be used to further optimize a nutritional regime for livestock or companion animals.

**Compositions & Kits**

Compositions for use (e.g., sufficient for, necessary for, or useful for) in the methods of some embodiments of the present invention include reagents for detecting the presence or absence of specific antigens (e.g., yeast $(1\rightarrow4)$-$\alpha$-D-glucan, yeast $(1\rightarrow6)$-$\beta$-D-glucan, yeast $(1\rightarrow4)$-$\alpha$-/$(1\rightarrow6)$-$\beta$-D-glucan). Any of these compositions, alone or in combination with other disclosed compositions are disclosed in the form of a kit. Kits may further comprise appropriate controls and/or detection reagents.

**Antigen-Carrier Conjugation**

Further disclosed are compositions (e.g., antigens) that find use in the analysis (e.g., immunological analysis; for antibody preparation) of yeast cell wall substances. The application discloses methods for: separation of a soluble $(1\rightarrow4)$-$\alpha$-/$(1\rightarrow6)$-$\beta$-D-glucan from yeast cell wall (see, Kwiatkowski et al. (2009) J. Instit. Brew. 115:1031; Arvindekar et al. (2002) Yeast 19:131-139) as an antigen for yeast $(1\rightarrow6)$-$\beta$-D-glucan; oxidation of this polysaccharide hydroxyl groups at C-6 position of glucopyranose rings with Pfizner-Moffat reagent (see, Pfizner et al. (1963) J. Am. Chem. Soc. 85:3027), in order to convert them into aldehyde groups (see, Zekovic et al. (2006) Chem. Papers 60:243-248); and the use of this oxidized form of $(1\rightarrow4)$-$\alpha$-/$(1\rightarrow6)$-$\beta$-D-glucan in the reductive-amination (see, Abdel-Magid et al. (1996) J. Org. Chem. 61:3849-3862) reaction with the lysine residues of a carrier (e.g., a carrier protein, e.g., BSA), to produce a polysaccharide-carrier conjugate. In some disclosures, the application relates to the application of separation techniques (e.g, ion exchange, DEAE cellulose and $Na_2HPO_4$ buffers of different ionic strength) in separation of pure antigen (e.g., $(1\rightarrow4)$-$\alpha$-/$(1\rightarrow6)$-$\beta$-D-glucan-BSA conjugate), which is used as a soluble immunogen for immunization of an animal (e.g., of a rabbit) (see, Howard et al. (2001) "Basic Methods in Antibody Production and Characterization", CRC Press, pp. 11-18 and 31-50). The application further discloses the preparation of BSA coated affinity phase (see, Matejtschuk (1997) "Affinity Separations: A Practical Approach", Oxford University Press) and its use in absorption of carrier-specific (e.g., BSA-specific) antibodies from antisera (e.g, rabbit sera) containing both antibodies to carrier (e.g., BSA) and antigen (e.g., $(1\rightarrow4)$-$\alpha$-/$(1\rightarrow6)$-$\beta$-D-glucan), thus facilitating specificity of the resulting antibody preparation. Disclosed methods for conjugation of polysaccharides to carriers (e.g., protein carriers, e.g., BSA) find use in the production of human and animal vaccines, as such methods preserve polysaccharide structure, rather than causing reducing end modifications as typical conjugation methods do° (see, Moreau et al., U.S. Pat. No. 6,596,861).

Weak antigenicity of poly- or oligosaccharides often requires modification prior to their use as antigens. In some disclosures, synthetic methods were developed in which polysaccharide antigens (containing $(1\rightarrow6)$-$\beta$-D-glucan) were conjugated to carrier (e.g., protein carrier, e.g., BSA) originating or derived from different animal species than the species used for immunization (see, Howard et al. (2001) "Basic Methods in Antibody Production and Characterization", CRC Press, pp. 11-18 and 31-50; Matejtschuk (1997) "Affinity Separations: A Practical Approach", Oxford University Press). Although the disclosure is not limited by the nature or type of carrier used, BSA is advantageous because of its commercial availability and the presence of five lysine residues, each with a single free amino group that can be conjugated to poly- or oligosaccharides by a variety of chemical methods (see, Abdel-Magid et al. (1996) J. Org. Chem. 61:3849-3862; Lees et al. (1996) Vaccine 14:190-198; Pawlowski et al. (1999) Vaccine 17:1474-1483). In some disclosures, the carbohydrate moiety of the antigen and the carrier to be used in conjugation are water-soluble. In some disclosures, reducing end aldehyde groups of the carbohydrate moiety of the antigen (e.g., $(1\rightarrow4)$-$\alpha$-/$(1\rightarrow6)$-$\beta$-D-glucan polysaccharide) are not utilized for direct conjugation to carrier (e.g., BSA), because to do so would substantially change the structure and binding capacity of the polysaccharide antigen. Further disclosed is a maximally preserved structure of carbohydrate moiety through application of an acetic anhydride/dimethylsulfoxide ($Ac_2O$/DMSO) system (see, Zekovic et al. (2006) Chem. Papers 60:243-248 for oxidation at positions other than C3.

The application discloses methods for use of yeast cell wall components (e.g., soluble (1→4)-α-/(1→6)-β-D-glucan from *Saccharomyces cerevisiae* cell wall) in the synthesis of antigen (e.g., (1→4)-α-/(1→6)-β-D-glucan-BSA (Bovine Serum Albumin)) conjugate to be used in the production of antibodies (e.g., polyclonal antibodies). The application also discloses an immunogenic composition comprising (1→4)-α-/(1→6)-β-D-glucan and/or a conjugate (e.g., protein or sugar conjugate) comprising the same. In some disclosures, antibody compositions find use in detection of yeast cell wall products in samples of interest (e.g., animal feed samples or derivatives, fractions, or extracts thereof). Disclosed, compositions are extracted from glucan-enriched material or glucan-enriched product (GEM, ALLTECH, Inc., Nicholasville, KY, USA), e.g., by acidic extraction (see, Kwiatkowski et al. (2009) J. Instit. Brew. 115:1031). In some embodiments, mild oxidation of polysaccharide (e.g., (1→4)-α-/(1→6)-β-D-glucan) at the C-6 position of the glucopyranose rings using dimethylsulfoxide (DMSO)/acetic anhydride ($Ac_2O$) mixture (see, Pfizner et al. (1963) J. Am. Chem. Soc. 85:3027) converts C-6 hydroxymethylene ($-CH_2OH$) groups into the aldehyde (-CH=O) groups (see, Zekovic et al. (20060 Chem. Papers 60:243-248 ), and makes an oxidized form of antigen (e.g., (1→4)-α-/(1→6)-β-D-glucan able to enter reductive amination with carrier (e.g., BSA) (see, Moreau et al., U.S. Pat. No. 6,596,861; Abdel-Magid et al. (1996) J. Org. Chem. 61:3849-3862). Disclosed conjugation methods, e.g., comprising polysaccharide activation at C6-OH, provide fully conserved original structure of the polysaccharide moiety, and therefore are advantageous for antigen preparation. Alternative methods of poly- or oligosaccharide activation including but not limited to periodate oxidation (see, Hay et al. (1973) Methods Carb. Chem. 5:357-370) or CDAP activation (see, Lees et al. (1996) Vaccine 14:190-198) yield substantially modified polysaccharides, compromising their utility as antigens.

**Antibodies**

Further disclosed is a method of preparing an immune globulin for use in assays to detect yeast cell wall components (e.g., yeast (1→4)-α-D-glucan, yeast (1→6)-β-D-glucan, yeast (1→4)-α-/(1→6)-β-D-glucan) comprising the steps of immunizing a subject with a yeast cell wall component (e.g, yeast (1→4)-α-D-glucan yeast (1→4)-α-β-D-glucan, yeast (1→4)-α-/(1→6)-β-D-glucan, (1→4)-α-/(1→6)-β-D-glucan-BSA, other fragments, variants, or conjugates thereof) and isolating immune globulin from the recipient. An immune globulin prepared by this method is also disclosed.

Inocula for polyclonal antibody production are typically prepared by dispersing the antigenic composition in a physiologically tolerable diluent such as saline or other adjuvants to form an aqueous composition. An immunostimulatory amount of inoculum is administered to a subject and the inoculated subject is then maintained for a time sufficient for the antigenic composition to induce protective antibodies.

The antibodies can be isolated to the extent desired by well known techniques such as affinity chromatography (see, e.g., Harlow and Lane, Antibodies; a Laboratory Manual (1988) Cold Springs Harbor Laboratory Press).

Antibodies include antiserum preparations from a variety of commonly used animals (e.g. goats, primates, rabbits, donkeys, swine, horses, guinea pigs, rats or man). The animals are bled and serum recovered.

An immune globulin produced in accordance with the present disclosure can include whole antibodies, antibody fragments or subfragments. Antibodies can be whole immunoglobulins of any class e.g. IgG, IgM, IgA, IgD or IgE, chimeric antibodies or hybrid antibodies with dual specificity to two or more antigens of the invention. They may also be fragments e.g. F(ab')2, Fab', Fab, Fv and the like including hybrid fragments.

Alternatively, techniques described for the production of single chain antibodies can be adapted using methods known in the art to produce single chain antibodies which specifically bind to a particular antigen. Antibodies with related specificity, but of distinct idiotypic composition, can be generated by chain shuffling from random combinatorial immunoglobin libraries (see, e.g., Burton, Proc. Natl. Acad. Sci. 88, 11120 23, 1991).

Single-chain antibodies also can be constructed using a DNA amplification method, such as PCR, using hybridoma cDNA as a template (see, e.g., Thirion et al., 1996, Eur. J. Cancer Prev. 5, 507-11). Single-chain antibodies can be mono- or bispecific, and can be bivalent or tetravalent. Construction of tetravalent, bispecific single-chain antibodies is taught, for example, in Coloma & Morrison, 1997, Nat. Biotechnol. 15, 159-63. Construction of bivalent, bispecific single-chain antibodies is taught, for example, in Mallender & Voss, 1994, J. Biol. Chem. 269, 199-206.

A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology (see, e.g., Verhaar et al., 1995, Int. J. Cancer 61, 497-501; Nicholls et al., 1993, J. Immunol. Meth. 165, 81-91).

Antibodies which specifically bind to a particular antigen also can be produced by inducing in vivo production in the lymphocyte population or by screening immunoglobulin libraries or panels of highly specific binding reagents as disclosed in the literature (see, e.g., Orlandi et al., Proc. Natl. Acad. Sci. 86, 3833 3837, 1989; Winter et al., Nature 349, 293 299, 1991).

Chimeric antibodies can be constructed as disclosed in WO 93/03151. Binding proteins which are derived from immunoglobulins and which are multivalent and multispecific, such as the "diabodies" described in WO 94/13804, also can be prepared. Antibodies can be purified by methods well known in the art. For example, antibodies can be affinity purified by passage over a column to which the relevant antigen is bound. The bound antibodies can then be eluted from the column using a buffer with a high salt concentration.

An immunogenic composition of the invention can be administered to a subject who then acts as a source of immune globulin, produced in response to challenge from the specific immunogenic composition. A subject thus treated would donate plasma from which hyperimmune globulin would be obtained via conventional plasma fractionation methodology. Disclosed compositions include but are not limited to antibodies (e.g., monoclonal and/or polyclonal antibodies) that can be whole immunoglobulins of any class (e.g. IgG, IgM, IgA, IgD or IgE, chimeric antibodies) or hybrid antibodies with specificity to two or more antigens of the invention. They may also be fragments e.g. F(ab')2, Fab', Fab, Fv and the like including hybrid fragments.

Methods of making monoclonal antibodies are well known in the art and can include the fusion of splenocytes with myeloma cells (See, e.g., Kohler and Milstein 1975 Nature 256; 495; Harlow and Lane, Antibodies; a Laboratory Manual (1988) Cold Springs Harbor Laboratory Press). Alternatively, monoclonal Fv fragments can be obtained by screening a suitable phage display library (See, e.g., Vaughan TJ et al 1998 Nature Biotechnology 16; 535). Monoclonal antibodies may be humanized or part humanized by known methods.

In some disclosures antibody compositions are capable of recognizing yeast cell wall antigens (e.g., monoclonal or polyclonal antibodies capable of recognizing yeast (1→4)-α-D-glucan, yeast (1→6)-β-D-glucan, yeast (1→4)-α-/(1→6)-β-D-glucan, or fragments, variants, or conjugates thereof). Disclosed antibody compositions bind to yeast cell wall (1→4)-α-D-glucan, but not (1→4)-α-D-glucan-containing moieties present in substances other than yeast cell wall (1→4)-α-D-glucan-containing polymers (e.g., yeast cell wall (1-4)-α-D-glucan, yeast cell wall (1→4)-α-/(1→6)-β-D-glucan, or fragments, variants, or conjugates thereof). In some disclosures, antibody compositions bind to yeast cell wall (1→6)-β-D-glucan, but not (1→6)-β-D-glucan-containing moieties present in substances other than yeast cell wall (1→6)-β-D-glucan-containing polymers (e.g., yeast cell wall yeast cell wall (1→6)-β-D-glucan, yeast cell wall (1→4)-α-/(1→6)-β-D-glucan, or fragments, variants, or conjugates thereof). In a further disclosure, antibody compositions bind to yeast cell wall (1→4)-α-/(1→6)-β-D-glucan, but not (1→4)-α-/(1→6)-β-D-glucan-containing moieties present in substances other than yeast cell wall (e.g., yeast cell wall (1→4)-α-/(1→6)-β-D-glucan, or fragments, variants, or conjugates thereof).

## EXAMPLES

The following examples are provided in order to demonstrate and further illustrate certain preferred embodiments and aspects of the present invention and are not to be construed as limiting the scope thereof.

### Example 1

### Extraction of (1→4)-α-/(1→6)-β-D-glucan from food grade *Saccharomyces cerevisiae* yeast cell wall β-glucan

*Yeast cell wall glucan preparation*

An industrial grade (1→6)-β-D-glucan (ALL-BGY, ALLTECH, Inc., Nicholasville, KY, USA) produced from *S. cerevisiae* by an enzymatic/alkaline/thermal treatment was washed four times with deionized water to remove any soluble residues. After freeze-drying, "glucan enriched material" ("GEM") was obtained.

### *Acidic digestion of yeast cell wall glucan*

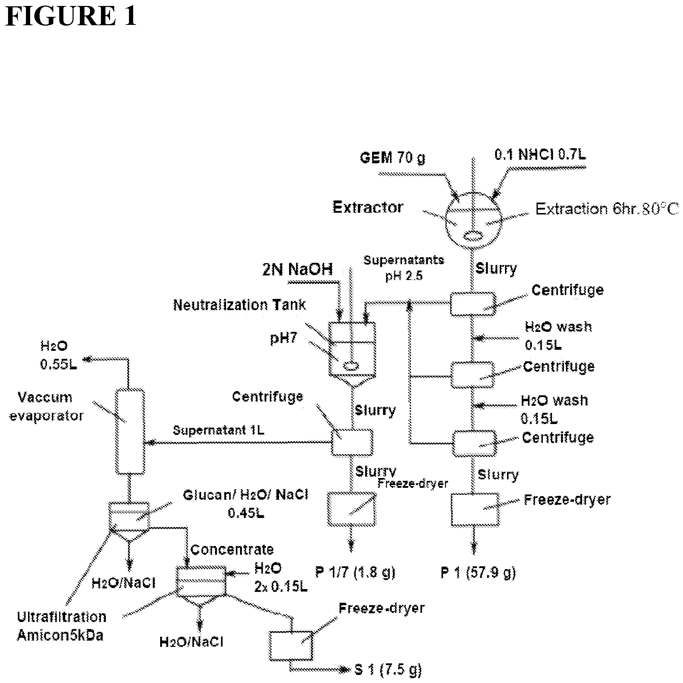

Figure 1 illustrates the preparation of the various fractions. Acidic digestion of yeast cell wall glucan was performed as described in Kwiatkowski et al. (2009) J. Instit. Brew. 115:151-158; herein incorporated by reference in its entirety. "Glucan enriched material" ("GEM") (70 g) was subjected to hydrolysis with 700 mL of 100 mM HCl (pH 2.2) at 80°C for 6 h. After this time, the mixture was centrifuged at $13,500 \times$ g/10°C/10 min and supernatant was collected. The pellet was extracted two times with 150 ml of deionized water and lyophilized, yielding 57.9 g of a white amorphous product (P1).

The two washes were combined with the original supernatant and neutralized to pH 7.0 with 2N NaOH. The precipitate that formed was collected (pellet P1/7) from the centrifugation (1.82 g after lyophilization) and the supernatant was concentrated to a volume of 450 ml using a Buchi vacuum rotary evaporator below a temperature of 37°C. This solution was further concentrated using 5 kDa AMICON 15 ultra-filtration centrifuging devices (Millipore Corporation, Delaware USA). The concentrate (-150 mL) was washed by centrifugation two times with two equal volumes of deionized water, using the same ultra-filtering devices, to remove salt and the low molecular weight components resulting from the acid hydrolysis. The washed concentrate (S1) was lyophilized, yielding 7.5 g of white amorphous product. A part of the pellet P1 was subjected to a second extraction using the same ratio of reagents and conditions as in the first 0.1N HCl extraction. These products were lyophilized to yield P2 and S2. The second extraction with 0.1N HCl did not produce a precipitate after the supernatant had been neutralized. A part of the pellet P2 was subjected to a third extraction using the same ratio of reagents and conditions as in the first 0.1N HCl extraction and the products were lyophilized to yield P3 and S3. The third extraction with 0.1N HCl did not produce a precipitate after the supernatant had been neutralized. The carbohydrate composition and the polysaccharide structure of the soluble and non-soluble fractions from the 0.1N HCl extractions were established by using $^1$H NMR spectroscopy. The mannose and glucose content in the samples was analyzed using $H_2SO_4$-HPLC composition analysis (see, Dallies et al. (1998) Yeast 14:1297-1306; herein incorporated by reference in its entirety). An estimated amount of protein content was calculated using LECO combustion analysis and by multiplying the nitrogen content by a factor of 6.25.

## Example 2

## Antigen Preparation

### $Ac_2O$/DMSO oxidation of (1→4)-α-/(1→6)-β-D-glucan preparation

Five hundred eighty milligrams of 'soluble' glucan were placed in a 22 ml glass vial equipped with a magnetic stirring rod and stopped with a rubber septum. Into this vial, 10ml of anhydrous DMSO and 110μl of acetic anhydride were added using a glass syringe, at room temperature and under agitation. The glucan suspension dissolved completely within 4 h of stirring at 20°C. Stirring of the mixture was continued for 24 h, after which the reaction mixture was diluted with 40ml of water. The resulting clear solution was concentrated using a 30 kDa Amicon®15 ultra-filtration centrifuging device and the residue on the filter was washed five times with 12 ml of deionized water each, to separate and purify the product of oxidation from solvents and reactants. All of the centrifugations were done at 4,750 × g/30 min/10°C. The final residue on the filter was collected and freeze-dried yielding 502mg of the oxidized (1→4)-α-/(1→6)-β-D-glucan in form of a white powder. The product was partially soluble in water and completely soluble in DMSO.

### Elemental analysis

Found for oxidized glucan: C-40.05%; H-6.61%. Calculated for $C_6H_8O_5$ x $H_2O$: C-40.48%; H-5.66%

### Reductive amination of oxidized (1→4)-α-/(1→6)-β-D-glucan with BSA

A sample of 412 mg of oxidized (1→4)-α-/(1→6)-β-D-glucan was dissolved in 20 ml of deionized water and was combined with a solution of 256 mg of BSA in 2 ml of deionized water under magnetic stirring. The mixture was then treated with 60 mg of solid sodium cyanoborohydride. The reaction vial was stopped with a rubber septum, and the mixture was stirred at 20°C for 24 h. After this time, 100 mg of sodium borohydride were added and the mixture was stirred for additional 22 h. The clear solution was then filtered through 30 kDa Amicon®15 ultra-filtration centrifuging device at 4,750 × g/30 min/10°C. The residue was washed on the filter four times with 12 ml of water each and the final concentrate was lyophilized, yielding 573 mg of a white powder.

### Elemental analysis

C 45.01%, H 6.40%, N 2.11 %.

### DEAE Cellulose ion exchange chromatography separation of (1→4)-α-/(1→6)-β-D-glucan-BSA conjugate

A solution of 400 mg of the crude conjugate in 5 ml of 5 mM $Na_2HPO_4$ was introduced on the top of a 2 × 55cm glass chromatography column containing 50 g of DEAE Cellulose in 5mM $Na_2HPO_4$. $Na_2HPO_4$ at various concentrations was used for elution of: 40.1 mg of (1→4)-α-/(1→6)-β-D-glucan (220 ml, 5 mM); 132.3 mg of pure (1→4)-α-/(1→6)-β-D-glucan-BSA conjugate (280 ml, 55 mM); and 96.7 mg of BSA (80 ml, 100 mM). Pure (1→4)-α-/(1→6)-β-D-glucan-BSA conjugate solution was concentrated to a volume of 2 ml using a 30 kDa AMICON15 ultra-filtration centrifuging device, washed with deionized water and lyophilized, yielding 132.3 mg (24.4% total yield) of white powder-like product. Gel electrophoresis produced a wide band revealed by using Comassie Blue and PAS staining, with a center at ~170 kDa. No free BSA or any other impurities were detected in the electrophoregrams.

### Elemental analysis

C 40.11%, H 6.23%, N 3.06%, (20.44w% of BSA). The calculated ratio of β-glucan : BSA is 4:1.

## Example 3

**Polyclonal Antibody Preparation and Purification**

*Antigen preparation and rabbit immunization*

Ten mg of BSA-conjugatcd antigen prepared as described in Example 2 was lyophilized, stored at 4°C, and used for immunization of three rabbits (200 µg antigen per immunization). The immunization protocol was as listed in Table 1.

Table 1. Immunization protocol for generation of anti-(1→4)-α-/(1→6)-β-D-glucan-BSA polyclonal antibodies in rabbits.

| Day | Action |
| --- | --- |
| 0 | Set-up, pre-bleed, initial immunization |
| 21 | Boost |
| 35 | Boost |
| 44-45 | Bleed |
| 49 | Boost |
| 58-59 | Bleed |
| 63 | Bleed |

*Affinity phase purification of polyclonal antibody*

One gram of Epoxy-activated-Agarose (Sigma E 6632) was suspended in a solution of 2.68 g of BSA (SIGMA A2153) in 27 ml of 10 mM $Na_2HPO_4$ and the mixture was stirred for 4 hrs at 4°C in an ice-water bath. After this time, the mixture was centrifuged at 2500 × $g$/5 min/10°C and the pellet was washed and centrifuged four more times with 30 ml of 100 mM $Na_2HPO_4$/0.05w%$NaN_3$ each.

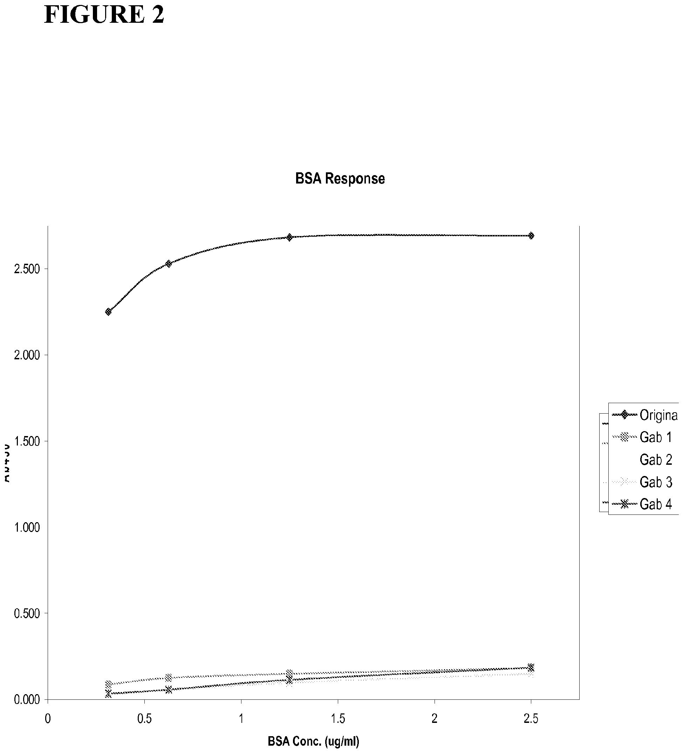

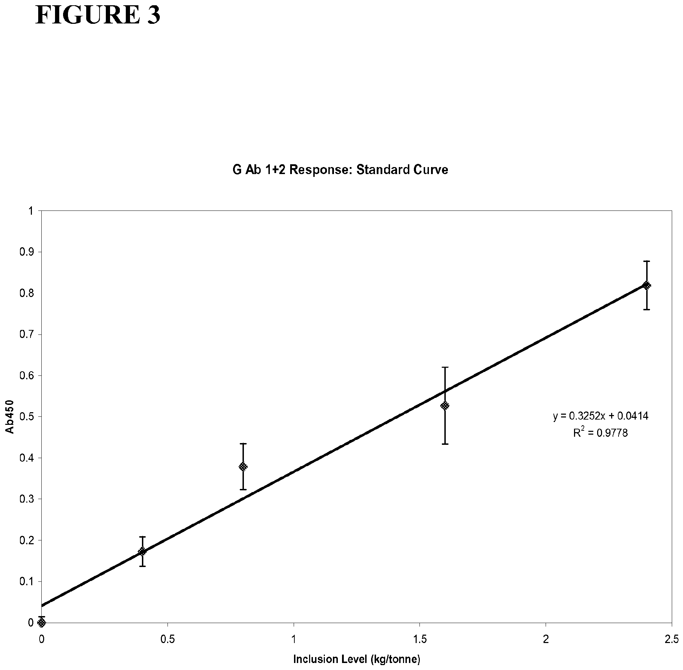

The pellet from the last centrifugation of the BSA coated affinity phase (described *supra*) was suspended in the solution of 750 µl of the sera (collected from the rabbit that has been immunized with the 'soluble' glucan BSA conjugate) in 20 ml of 10mM $Na_2HPO_4$/0.05w%$NaN_3$ and tumbled 2 h at room temperature and then centrifuged at 1500 × $g$/5 min/10°C. The supernatant was collected and the pellet washed three more times with 20 ml of the same buffer, resulting in the collection of three more supernatants. The supernatants were examined for specificity towards BSA and 'soluble' glucan as presented in Figure 2. The first and the second supernatants, which contained significant amount of antibodies, were pooled together and concentrated to a volume of 2.5 ml by centrifugation at 5000 × $g$/5 min/10°C using a 10 kDa AMICON Ultra-15 Centrifugal Filter Device. The concentrate was washed on the filter with 10 mM $Na_2HPO_4$/0.05w%$NaN_3$ and diluted to a volume of 6 ml (6 g) with the same buffer. This solution at 1:800 dilution of 'affinity purified antibodies' was used in the ELISA assay for MYCOSORB in animal feed. The calibration curve obtained is presented in Figure 3.

## Example 4

**Detection of (1→4)-α-/(1→6)-β-D-glucan of Yeast Cell Wall by ELISA**

*Material and Methods*

*Reagents*

Only reagents of recognized analytical grade were used, unless otherwise specified, and deionized or demineralized water or water equivalent purity (18,2 MΩ/cm at 25 °C).°
Reagents and preparation of reagent solutions were as follows:

- Hydrochloric acid, 12.1 N; HCl
- Diluted Hydrochloric acid, 1 N; HCl: 41.3 ml HCl 121 N was slowly added to 400 ml deionized water and mixed with a stir bar on a stir plate until cool. The solution was transferred to a 500 ml volumetric flask and brought to volume with deionized water, then stored at room temperature.

- Dimethyl sulfoxide: DMSO used was high purity, suitable for spectrophotometry, liquid chromatography and gas chromatography.
- Extraction reagent: To prepare extraction reagent, 900 ml of DMSO was mixed with 98.75 ml deionized water and 1.25 ml HCl 12,1N. The solution was mixed with a stir bar on a stir plate at medium speed and room temperature, then stored at 20°C or above.
- Sodium chloride("NaCl")
- Potassium chloride ("KCl")
- Disodium hydrogen phosphate ("$Na_2HPO_4$")
- Potassium dihydrogen phosphate("KH2PO$_4$")
- Phosphate Buffer Saline("PBS") 10X: To prepare 10X PBS, 80.0 g sodium chloride [NaCl], 2.0 g potassium chloride [KCl], 14.2 g disodium hydrogen phosphate [Na2HPO4] and 2.4 g potassium dihydrogen phosphate [KH2PO4] were mixed with approximately 800 ml deionized water. The solution was stirred using a stir bar on a stir plate until solids were fully dissolved. The solution was transferred to a 1000 ml volumetric flask and brought to volume with deionized water, then stored at room temperature or at 2 - 8 °C. The maximum storage time was 6 months at 2 - 8 °C as an undiluted liquid. The solution was warmed to room temperature before use.
- Diluted PBS, IX: To prepare 1X PBS, 1 volume of PBS, 10X was diluted with 9 volumes of deionized water. The dilution was mixed using a stir bar on stir plate at medium speed and room temperature. The maximum storage time was one day.
- Polysorbate 20
- Phosphate Buffered Saline, 0.01 M with 0.05 % Polysorbate 20, pH 7.4: To prepare, the contents of one pouch was dissolved in 1000 ml of deionized water. The solution was stirred on a stir plate until fully dissolved, then stored at room temperature or at 2 - 8 °C. The maximum storage time was 6 months at 2 - 8 °C as an undiluted liquid. The solution was warmed to room temperature before use.
- Dry non-fat milk
- Dry non-fat milk, 3 % (w/v) in PBS, 1X BLOCK reagent: To prepare 1X Block reagent, dry milk was completely dissolved in 1X PBS prior to use. A 10 ml volume was needed for each plate to block. The maximum storage time was one day.
- Rabbit Polyclonal Antibodies; primary antibody (Antibody Solution #1): Affinity-purified antibodies prepared as described herein. Stored at - 80 °C until use.
- Diluted rabbit polyclonal antibody solution: Diluted to 1 :$X_i$ in PBS by adding 1 $\mu$l of antibody solution #1 to $X_i$ $\mu$l of PBS, 1X. A 10 ml volume of solution was needed for each microtiter plate. Diluted antibody solution was mixed well prior use, and excess diluted antibody was discarded. The maximum storage time was one day.
- Peroxidase-conjugated AffiniPure Goat anti-Rabbit IgG (H+L); HRP secondary antibody, Ref. # 111-035-045 (Jackson ImmunoResearch, PA, USA - Antibody Solution #2): Rehydrated with 1.5 ml deionized water and centrifuged until obtaining a completely clear solution. Stored in 10 $\mu$l aliquots at - 80 °C until use. The maximum storage time was 6 weeks at 2 - 8 °C as an undiluted liquid.
- Diluted goat anti-rabbit antibody: Diluted to 1:$Y_i$ in PBS by adding 1 $\mu$l of antibody solution #2 to $Y_i$ $\mu$l PBS, 1X. Diluted antibody solution was mixed well prior use, and excess diluted antibody was discarded. The maximum storage time was one day.
- SureBlue Reserve TMB Microwell Peroxidase Substrate: The needed volume was redispensed in amber Nalgene HDPE and LDPE bottles only. The substrate was warmed to room temperature prior to use. The lid was kept closed tightly, protected from light. Excess substrate was discarded, and the reagent was stored at 2 - 8°C. The maximum storage time was 24 months from date of manufacture when stored at 8°C.
- MYCOSORB stock sample; batch Ref. #285965 (ALLTECH, Inc., KY, USA): A homogeneous sample of MYCOSORB was used.
- Dilution of MYCOSORB in feedstuffs: 20 g of the feed material was mixed with increasing amounts of MYCOSORB product (0.01; 0.02; 0.08; 0.10; 0.12 g) in a 250 ml centrifuge bottle in three replicates to generate a standard curve. The bottle was shaken on an orbital shaker at 400 rpm for 1 h. The precision for weighing the feed materials equaled $\pm$ 0.025 g and for the MYCOSORB product, $\pm$ 0.005 g.
- Large batch of diluted MYCOSORB in feedstuffs: A large sample of diluted MYCOSORB product in feed was prepared using 1.2 g of product and 300 g of the feed material in a 1000 ml container. The bottle was shaken on an orbital shaker at 400 rpm for 1 h. The final concentration of MYCOSORB product in the feed material was 4.0 kg/T. 20.0 g of the homogenized 300.0 g large batch feed-product mixture was transferred into a 250 ml centrifuge bottle. The transfer was repeated 9 more times to obtain 10 replicate samples. The mixture was intermittently shaken in between every 3 aliquots prepared to avoid the product settling to the bottom of the bottle. The precision for weighing the feed materials equaled $\pm$ 0.025 g and the MYCOSORB product, $\pm$ 0.005 g.
- Dilution of unknown MYCOSORB amount in feedstuffs: An unknown sample was prepared by transferring an unknown amount of MYCOSORB product and 20.0 g ($\pm$ 0,025 g) of the feed material to each of five 250 ml centrifuge bottles. The bottle was shaken on an orbital shaker at 400 rpm for 1 h. Sample preparation of the standard curve for the sample(s) was completed simultaneously.

## Sample formulation

Three feed materials (see Table 4, Table 5 , and Table 6) were used to validate the assay. The feed material formulation was selected according to compositions that can be found in the art. The sample size was a total of 15 kg of feed material that was carefully homogenized prior being sent to the laboratory. The sample was stored at 2 - 8°C for the entire study period.

## Sample homogeneity verification

The homogeneity of the dilution of the MYCOSORB product in the feed material was verified by preparing a large batch of MYCOSORB product diluted in feed at an intermediate concentration of product in the feed of 4.0 kg/T. The sample was divided into 10 sub-samples and was further extracted according to the extraction procedure and followed by the determination of the coefficient of variation for the detection of the product by means of the ELISA assay four times for each of the 10 samples.

## Sample preparation

The preparation of standard sample was made as described herein and is presented in Table 2, with the MYCOSORB product to be tested. In this case, the initial stock product was referenced as stated herein. The unknown samples were prepared according as described *supra.* No grinding of the feed sample was required prior carrying out the extraction procedure, (this would have also been the case if the feed material was provided under a pelleted form). It was found that grinding of the feed material may affect the final reliability of the assay.

**Table 2.** Dilution steps of MYCOSORB product for standard curve.

| Dilution factor (kg/T)[a] | Sample group number | Feed (g)[b] | MYCOSORB (g)[c] | Replicates $\times$ Samples |
|---|---|---|---|---|
| 0 | 0 | 20.0 | 0 | 2 $\times$ 10 |
| 0.5 | 1 | 20.0 | 0.01 | 2 $\times$ 3 |
| 1.0 | 2 | 20.0 | 0.02 | 2 $\times$ 3 |
| 4.0 | 3 | 20.0 | 0.08 | 2 $\times$ 3 |
| 5.0 | 4 | 20.0 | 0.10 | 2 $\times$ 3 |
| 6.0 | 5 | 20.0 | 0.12 | 2 $\times$ 3 |
| 0 to 6.0 | C[d] | 20.0 | 0 to 0.12 | 4 $\times$ 10 |
| [a] Dilution factor expressed in kilograms of MYCOSORB product per ton of feed material [b] Feed Range = $\pm$ 0,025 g, [c] MYCOSORB product Range = $\pm$ 0,005 g [d] Where C is an unknown MYCOSORB amount in feedstuffs | | | | |

## Extraction procedure

The entire sets of bottles were shaken on an orbital shaker at 400 rpm for 1 h. Three bottles at a time were removed and a volume of 80 ml of extraction reagent was added to each bottle using a bottle top volumetric dispenser. Bottles were capped. All the samples were shaken by hand to ensure full mixing with the extraction reagent. The bottles were placed in an 80 °C thermostatically maintained water bath for 1 h, making sure that the feed solution was fully submerged. After incubation, the bottles were removed from the water bath and the content of the bottles mixed with rods (NALGENE brand) using a high torque lab stirrer. The mixture was fully homogenized. A clean rod was used for each MYCOSORB inclusion level and for each unknown sample(s). The bottles were replaced in the 80°C thermostatically maintained water bath for 1 h. After incubation, the bottles were removed from the water bath and 80 ml of deionized water was added to each bottle, using a bottle top volumetric dispenser, and then bottles were capped. The bottles were shaken by hand until fully mixed; NALGENE brand rods with the high torque lab stirrer were used as necessary. The bottles were centrifuged at 8,000 g for 10 min. Using glass pipettes, approximately 10 ml of the supernatant was transferred to 15 ml centrifuge sterile tubes. The tubes were centrifuged at 4,000 g for 10 min and transferred to new 15 ml centrifuge sterile tubes, adequately labeled, by decanting. The tubes were stored at - 20°C overnight until used for ELISA assay.

## ELISA procedure

The SureBluc Reserve TMB Microwell Peroxidase Substrate was used to trigger a colorimetric reaction through the reaction with the diluted HRP secondary antibody, further reacting with the diluted primary antibody itself specifically reacting with the substrate of interest extracted from the diluted MYCOSORB product, which is immobilized on the microtiter plate. The colorimetric reaction was ultimately stopped after 20 min using HCl, IN and the absorbance reading was performed on a plate reader at a wavelength of 450 nm. Therefore, blanks made of the feed material only were measured to account for possible colorimetric variation coming from the feed matrix giving a background signal on the spectrophotometer. For calculation, the blank values were subtracted from the absorbance values obtained for each sample.

The previously extracted samples stored overnight were thawed and warmed to room temperature. The samples were vortexed to ensure homogenous sample prior use in ELISA for coating wells of microtiter plates.

For each sample replicate, 100 $\mu$l of the supernatant was transferred to each of 3 wells on a 96-well microtiter plate as presented in Table 3. A randomized distribution of the samples on the plate might be performed in laboratory equipped with a programmed robotized microtiter well dispenser. The standard curve samples and sample replicates were all distributed on one unique plate (i.e. all 0 kg/T sample replicates were plated across row/column E1 to F10). The unknown MYCOSORB replicate samples were distributed on the same plate (i.e. plated across row/column All to C12). The microtiter plates were sealed with microplate adhesive film and incubated at 37°C for 1 h. After incubation, the solution in each well of the microtiter plate was removed and replaced by 250 $\mu$l PBS, 1X for a single wash. The operation was repeated a total of 2 more times. Each well of the microtiter plate was blocked with 100 $\mu$l of diluted non-fat milk in PBS solution and incubated at room temperature for 1 h. After incubation, the solution in each well was removed and replaced by 250 $\mu$l PBS, 1X for a single wash, followed by 250 $\mu$l PBS containing polysorbate 20, 0.05% for 2 washes.

A volume of 100 $\mu$l of the diluted primary antibody was added to each well. The microtiter plate was incubated at room temperature for 1 h. After incubation, the solution in each well of the microtiter plate was removed and replaced by 250 $\mu$l of PBS, 1X containing Polysorbate 20, 0.05% for 3 washes.

A volume of 100 $\mu$l of the diluted secondary antibody was added to each well. The microtiter plate was incubated at room temperature for 1 h. After incubation, the solution in each well of the microtiter plate was removed and replaced by 250 $\mu$l of PBS, 1X containing Polysorbate 20, 0.05% for 3 washes.

A volume of 100 $\mu$l of TMB Substrate, pre-warmed to room temperature, was added to each well and the microtiter plate was incubated for 20 min at room temperature. After exactly 20 min with the TMB substrate, a volume of 100 $\mu$l of HCl, IN was immediately added to each well to stop the colorimetric development. Each microtiter plate was read on a microtiter plate reader at 450 nm.

**Table 3 .** Sample group distribution on the microtiter plate.

| ** | 1 | 2 | 3 | 4 | 5 | 6 | 7 | 8 | 9 | 10 | 11 | 12 |
|---|---|---|---|---|---|---|---|---|---|---|---|---|
| A | $C_{.1}$* | $C_{.2}$ | $C_{.3}$ | $C_{.4}$ | $C_{.5}$ | $C_{.6}$ | $C_{.7}$ | $C_{.8}$ | $C_{.9}$ | $C_{.10}$ | - | - |
| B | $C_{.1}$ | $C_{.2}$ | $C_{.3}$ | $C_{.4}$ | $C_{.5}$ | $C_{.6}$ | $C_{.7}$ | $C_{.8}$ | $C_{.9}$ | $C_{.10}$ | - | - |
| C | $C_{.1}$ | $C_{.2}$ | $C_{.3}$ | $C_{.4}$ | $C_{.5}$ | $C_{.6}$ | $C_{.7}$ | $C_{.8}$ | $C_{.9}$ | $C_{.10}$ | - | - |
| D | $C_{.1}$ | $C_{.2}$ | $C_{.3}$ | $C_{.4}$ | $C_{.5}$ | $C_{.6}$ | $C_{.7}$ | $C_{.8}$ | $C_{.9}$ | $C_{.10}$ | $5_{.1}$ | $5_{.1}$ |
| E | $0_{.1}$ | $0_{.2}$ | $0_{.3}$ | $0_{.4}$ | $0_{.5}$ | $0_{.6}$ | $0_{.7}$ | $0_{.8}$ | $0_{.9}$ | $0_{.10}$ | $5_{.2}$ | $5_{.2}$ |
| F | $0_{.1}$ | $0_{.2}$ | $0_{.3}$ | $0_{.4}$ | $0_{.5}$ | $0_{.6}$ | $0_{.7}$ | $0_{.8}$ | $0_{.9}$ | $0_{.10}$ | $5_{.3}$ | $5_{.3}$ |
| G | $1_{.1}$ | $1_{.2}$ | $1_{.3}$ | $2_{.1}$ | $2_{.2}$ | $2_{.3}$ | $3_{.1}$ | $3_{.2}$ | $3_{.3}$ | $4_{.1}$ | $4_{.2}$ | $4_{.3}$ |
| H | $1_{.1}$ | $1_{.2}$ | $1_{.3}$ | $2_{.1}$ | $2_{.2}$ | $2_{.3}$ | $3_{.1}$ | $3_{.2}$ | $3_{.3}$ | $4_{.1}$ | $4_{.2}$ | $4_{.3}$ |
| * Where *C* is an unknown MYCOSORB product level of inclusion in feedstuffs<br>**Refer to Table 2 for sample group identification | | | | | | | | | | | | |

## Data exclusion from replicates

Statistical data analysis was needed to ensure that all replicates were similar, excluding those that do not pass the Dixon's Q-Test and then consequently identified as outliers according to the definition: $Q_n = |x_a - x_b|/R$, where $R$ is the range of all data points; $x_a$ is the suspected outlier; $x_b$ is the data point closest to $x_a$. If $Q_{calculated} > Q_{table}$ then reject the questionable point at a 95% confidence interval.

### Standard curve formulation

Calibration curves were used for the quantification. They are constructed for each feed matrix using at least five levels (including zero) in the working range of the product as stated in the Council Directive 96/23. The average of the three replicates of well absorbencies at $OD_{450}$ for each sample group was calculated. The average of the ten replicates of well absorbencies at $OD_{450}$ for the blank feed sample was calculated. A standard curve or calibration curve was then made with the difference $\Delta OD_{450}$ ($OD_{450}$ sample group - $OD_{450}$ blank feed) obtained on the ordinate and the different MYCOSORB product concentration ranges, 0.5, 1.0, 4.0, 5.0, 6.0 kg/T on the abscissa.

The coefficient of regression and the equation of the plotted inclusion levels against $OD_{450}$ absorbency were calculated according to a linear regression using the best fitting line, $\Delta OD_{450} = Ax$ (MYCOSORB) after the correction of the values by the averaged blank value. The acceptability range and the linearity of the calibration curve was validated by a coefficient of correlation ($r^2$) value superior to 0.95. The corresponding equation of the curve was then used to determine the inclusion level of MYCOSORB product in the unknown sample(s) by resolving the corresponding equation.

### Interpolation

Should a coefficient of correlation above $r^2 > 0.95$ be found with the standard curve, the corresponding equation of the corrected curve $F(x) = Ax$, (subtracted from the background signal and intercepting the zero) is then used to determine the inclusion level of MYCOSORB product in the unknown sample(s). The average of the ten replicates at $OD_{450}$ for the unknown inclusion level ($C$) of MYCOSORB product is calculated. The obtained average is subtracted from the average of the ten replicates of well absorbencies at $OD_{450}$ for the blank feed sample previously calculated. The $OD_{450}$ average value is then used to resolve the equation of the corrected curve $F^{-1}(yc)$, where $y_c = Ax_c$:

$x_c = y_c/A$, where $y_c$ is the $OD_{450}$ obtained average for the unknown sample ($C$) minus the $OD_{450}$ of the blank, $x_C$ is the corresponding inclusion level of MYCOSORB product, and $A$ is the slope of the standard curve, in kg/T $OD_{450}$.

### Limit of Detection and Quantification

The Detection Limit $(L_D = 3\sigma_{blank})$ and Quantification Limit $(L_Q = 10_{\sigma\ blunk})$ were measured according to the IUPAC nomenclature and given as $\Delta OD_{450}$.

The Limit of Detection ($L_D$) and Limit of Quantification ($L_Q$) were determined from the matrix blanks analysis and calculated into concentration from the liner regression corrected from the mean background $OD_{450}$ value by means of the calibration curve $F(x) = Ax$ resolved as followed according to:

$L_D = 3\sigma_{blank}/A$ and $L_Q = 3\sigma_{blank}/A$, where $L_D$ is the minimum detectable concentration of product or Limit of Detection; $L_Q$ is the minimum quantifiable concentration of product or Limit of Quantification.

### Homogeneity

The coefficient of variation of homogeneity ($RSD_H = CV_H$) was defined as the average coefficient of variation from 10 independent replicates obtained from the same sample at the same day, from the same technician, with the same equipment and method.

### Precision: Repeatability

The within-run repeatability precision ($RSD_{intra} = CV_{intra}$) was defined as the average coefficient of variation from the repetitions obtained from the same sample at the same day for the same run, from the same technician, with the same equipment and method.

The between-run repeatability precision ($RSDI_{nter} = CV_{Inter}$) is the average coefficient of variation from independent results obtained from the same sample at different days during different runs, from the same technician, with the same equipment and method.

### Precision: Overall precision

The precision evaluates the dispersion (or closeness) of the agreement between successive measurements of the same quantity. The dispersion in a set of measurements is usually expressed in terms of the standard deviation.

The precision was thus calculated from the standard deviation of a single individual experiment at a single inclusion level obtained from the same sample at the same day, from the same technician, with the same equipment and method; from the standard deviation comparing each individual run, from the same technician, with the same equipment and method according to the formula: $\sigma_{Total} = \sqrt{\left[ \sigma_{intra}^2 \middle/ n + \sigma_{Inter}^2 \right]}$, where $\sigma_{Total}$ is the standard deviation of the total set of experiments, $\sigma_{intra}$ is the standard deviation of a single individual experiment, $\sigma_{Inter}$ is the standard deviation between multiple experiments, and $n$ is the number of replicates.

The comparison of the standard deviation was achieved from the analysis of variance (one-way ANOVA) using an F-test (p-value $\leq 0.05$) between the variance of the group means and the mean of the within-group variances.

### *Accuracy*

The accuracy is the closeness of agreement that can be found between test results and the accepted reference value of the product being measured. The accuracy was thus evaluated from the measurement of the recovery expressed as percentiles. The recovery represents in this respect, the proportion of the amount of present in or admixed analyte, which is extracted and available for measurement. The bias is the difference between the test results and an accepted reference value (spiked concentration of the analyte). The calculations were performed according to the formulas:

$$Recovery\,(\%) = \frac{(X_c - X_b)}{X_{TH}} \times 100 \; ,$$

where $X_c$ is the concentration measured in spiked sample; $X_b$ is the concentration measured in unspiked sample (blank); $X_{TH}$ is the theoretical concentration.

### *Feed material used for validation tests*

Tests were performed in the Center for Animal Nutrigenomics and Applied Animal Nutrition at ALLTECH, Inc., KY, USA on a dairy feed formulation, a chicken feed formulation and a pig feed formulation of feed material admixed with different levels of inclusion of the MYCOSORB product. The full description of the feed formulation is included *infra.*

### *Dairy feed material*

The feed material was formulated by Coralis Vern (Vern sur Seiche, France, Ref. #111605) under the formula name "Spirit Lait 2L5" (Table 4). The sample size was a total of 15 kg of feed material that was carefully homogenized prior being sent to the laboratory. The sample was stored at 2 - 8 °C for the entire study period. The dairy feed material was provided under pelleted form. No grinding of the feed sample was required prior carrying out the extraction procedure.

**Table 4.** Dairy feed formulation used for the validation of the method.

| Ingredients | Rates |
| --- | --- |
| Wheat standard, % | 20.00 |
| Barley, % | 6.00 |
| Oil palm (experimental cake), % | 12.00 |
| Rape (de-oiled), % | 8.10 |
| Soy Oil Cake 48/Brazil, % | 4.20 |
| Wheat, fine bran, % | 24.94 |
| Wheat amyplus (triticale), % | 20.00 |
| Molasses, % | 1.00 |
| Vinasse (extracts from fermentation), % | 1.50 |
| Calcium Carbonate meal, % | 1.06 |
| Refined salt, % | 0.50 |
| EMROD, % | 0.20 |
| Super 26 Eco aa, % (vitamins and minerals premixture) | 0.50 |
| **Total, %** | 100 |
| **Nutrient** | |
| Dry matter, % | 88.324 |

(continued)

| Nutrient | |
|---|---|
| Crude Protein, % | 17.002 |
| Fat, % | 3.144 |
| Cellulose, % | 8.378 |
| Ashes, % | 6.723 |
| Starch, % | 24.955 |
| Total sugars, % | 4.420 |
| Starch and sugars, % | 29.376 |
| Ruminal Carbohydrate, g/kg | 352,926 |
| PDIN*, g/kg | 117.260 |
| PDIE*, g/kg | 103.028 |
| PDIA*, g/kg | 49.723 |
| Ruminal Nitrogen, g/kg | 124.906 |
| Lysine digestible in the rumen, g/kg | 6.794 |
| Methionine digestible in the rumen, g/kg | 1.873 |
| Calcium, % | 0.800 |
| Total Phosphorous, % | 0.659 |
| Sodium, % | 0.500 |
| Magnesium, % | 0.236 |
| Phosphorus digestible in the rumen, % | 0.466 |
| Structural Value, % | 0.220 |
| Wheat cakes, % | 20.000 |
| Wheat cakes triticale, % | 20.000 |
| Cereal cakes, % | 26.000 |
| Total outcome, wheat bran, % | 44.940 |
| Rape cakes, % | 8.100 |
| Total. MP Granul +, Granule durability % | 20.000 |
| Vitamin A, UI/kg | 4000 |
| Vitamin D3, UI/kg | 2000 |

* Serves to define the protein value of feeds and the animal protein requirements both expressed in terms of true Protein truly Digestible in the small Intestine ("PDI"). The PDI content of a diet is the sum of (*i*) the PDIA fraction, the dietary protein undegraded in the rumen, but truly digestible in the small intestine; (*ii*) the PDIM fraction, the microbial true protein which is truly digestible in the small intestine. Each feed contributes to microbial protein synthesis both by the degradable nitrogen (PDIMN) and the available energy it supplies to the rumen microorganisms (PDIME). The value of each feed is given directly by the sum of PDIA and PDIM considering the two following equations: (1) PDIN = PDIA + PDIMN and (2) PDIE = PDIA + PDIME.

### *Chicken feed material*

The feed material was formulated at the Coldstream University of Kentucky, an Alliance Research Group between the University of Kentucky and ALLTECH, Inc., KY, USA (Table 5). The preparation was made according to compositions found in the art and that can be generalized to the formulations commonly found in the European Union, North America, Latin America, etc. The sample size was a total of 15 kg of feed material that was carefully homogenized prior being sent to the laboratory. The sample was stored at 2 - 8 °C for the entire study period.

**Table 5.** Chicken feed formulation used for the validation of the method.

| Ingredients | Formula for Grower, 22-42 days |
|---|---|
| | Corn-soy basal |
| Corn | 62.15 |
| Soybean meal (48%) | 31.31 |

(continued)

| Ingredients | Formula for Grower, 22-42 days |
| --- | --- |
| | Corn-soy basal |
| Corn oil | 3.10 |
| Limestone | 1.37 |
| Dicalcium phosphate | 1.30 |
| Salt | 0.45 |
| Vitamin-mineral MIX a | 0.25 |
| DL-Methionine | 0.07 |
| L-lysine | - |
| Total | 100 |
| Nutrient | |
| ME, kcal/kg | 3120 |
| Crude Protein, % | 20 |
| Calcium, % | 0.90 |
| Available Phosphorus, % | 0.35 |
| Lysine, % | 1.11 |
| Methionine, % | 0.39 |
| Methionine + Cysteine, % | 0.72 |
| Na, % | 0.20 |

a Vitamin supplied per kg diet: 2200 IU vitamin A; 720 ICU vitamin D3; 27 IU vitamin E; 0,91 mg vitamin K3 (2-methyl-1, 4-naphthoquinone); 2 mg thiamin; 8 mg riboflavin; 55 mg niacin; 18 mg Ca pantothenate; 5 mg vitamin B6 (pyridoxines); 0,221 mg biotin; 1 mg folic acid; 478 mg choline; 28 $\mu$g vitamin B12 (cyanocobalamin).

***Pig feed material***

The feed material was formulated at the Coldstream University of Kentucky, an Alliance Research Group between the University of Kentucky and ALLTECH, Inc., KY, USA (Table 6). The preparation was made according to compositions found in the art and that can be generalized to the formulations commonly found in the European Union, North America, Latin America, etc. The sample size was a total of 15 kg of feed material that was carefully homogenized prior being sent to the laboratory. The sample was stored at 2 - 8°C for the entire study period.

**Table 6.** Pig feed formulation used for the validation of the method.

| Ingredients | Formula for gestating gilts and sows |
| --- | --- |
| | Corn-soy basal |
| Corn (ground, yellow) | 81.540 |
| Dehulled soybean meal (47.5%) | 12.310 |
| Corn oil | 2.000 |
| Limestone (ground) | 0.690 |
| Dicalcium phosphate | 2.630 |
| Salt | 0,500 |
| Vitamin mix (BASF) * | 0.050 |
| Trace mineral mix (Prince) ** | 0.080 |
| Choline chloride (60%) | 0.150 |
| Chromax | 0.050 |
| Total | 100 |
| Nutrient | |
| ME, kcal/kg | 3258 |

(continued)

| Nutrient | | |
|---|---|---|
| CP, % | 12.4 | |
| Ca, % | 0.54 | |
| Available P, % | 0.60 | |
| Lysine, % | 0.54 | |

| * Vitamin Mix composition: | BASF PREMIX (Concentration/kg of mixed diet) |
|---|---|
| | Concentrations/kg mixed diet |
| Vitamin A, IU | 5500 |
| Vitamin D, IU | 550 |
| Vitamin E, IU | 33 |
| Vitamin K, mg | 1.1 |
| Vitamin B12, $\mu$g | 15.125 |
| Niacin, mg | 15.125 |
| Pantothenic acid, mg | 13.75 |
| Riboflavin, mg | 4.125 |
| Biotin, mg | 0.17875 |
| Folic Acid, mg | 0.825 |
| Pyridoxine, mg | 2.475 |
| Thiamin, mg | 0.825 |

| Mineral | % | ppm supplied (1.5 #premix/ton) | Source |
|---|---|---|---|
| Iron | 13.33 | 100 | Sulfate |
| Zinc | 16.67 | 125 | Oxide |
| Manganese | 6.672 | 50 | Oxide |
| Copper | 2.000 | 15 | Sulfate |
| Iodine | 0.1709 | 1.28 | EDDI |
| Selenium | 0.0402 | 0.30 | selenite |
| Calcium | 4.815 | 36 | - |

### Validation using dairy feed material

Tests were performed in the Center for Animal Nutrigenomics and Applied Animal Nutrition at ALLTECH, Inc., KY, USA on a solid European type of dairy feed formulation admixed with five different concentrations of MYCOSORB product.

The dairy feed materials individually admixed with 0.0; 0.5; 1.0; 4.0; 5.0; 6.0 kg/T of MYCOSORB product were analyzed as well as a sample of the same dairy feed material admixed with an unknown level of MYCOSORB product when distributed on a same micro-titer plate.

### Applicability

The validation was performed in order to determine the characteristics of an ELISA assay developed for the specific analysis of the MYCOSORB product and alternates, as a traceability endpoint procedure for the purpose of tracking the product in complex feedstuffs.

Six levels of a MYCOSORB product were used for the validation corresponding to the working range of level of inclusion (0.0; 0.5; 1.0; 4.0; 5.0; 6.0 kg/T) admixed to the feed sample.

Diluted rabbit polyclonal antibody solution: the primary antibody dilution was adapted according to the background levels. The antibodies were thus diluted to 1:2,500 in PBS by adding 1 $\mu$l of antibody solution #1 to 1.2 ml of PBS, 1X. A 10 ml volume of solution was needed for each microtiter plate.

Diluted goat anti-rabbit antibody: the secondary antibody dilution was adapted according to the background levels. The antibodies were thus diluted to 1:20,000 in PBS by adding 1 $\mu$l of antibody solution #2 to 20.0 ml PBS, 1X.

*Homogeneity*

The homogeneity verification of the sample was achieved for a median level of inclusion of MYCOSORB product (4.0 kg/T) in the experimental plan. The assay for this particular level was repeated 5 times on 10 individual samples using 4 replicates each.

According to the calculated values found detailed in Table 7, the $CV_H$ was 4.53%.

**Table 7.** Homogeneity evaluation on a single concentration of 4.0 kg/T of MYCOSORB product admixed to a European type of diet for dairy evaluated through the ELISA procedure for 5 runs on 10 individual sample preparation analyzed in 4 replicates.

| Run # | 4 | 5 | 8 | 9 | 11 | Average |
|---|---|---|---|---|---|---|
| *n* | $4 \times 10$ | $4 \times 10$ | $4 \times 10$ | $4 \times 10$ | $4 \times 10$ | $4 \times 10$ |
| Average OD 450 nm | 1.155 | 1.055 | 0.833 | 1.166 | 1.044 | 1.051 |
| $\sigma_h$ | 0.118 | 0.080 | 0.077 | 0.083 | 0.100 | - |
| % $CV_h$ | 10.21 | 7.58 | 9.27 | 7.09 | 9.59 | - |
| Homogeneity, % $CV_H$ | 8.75 | | | | | |

*Calibration*

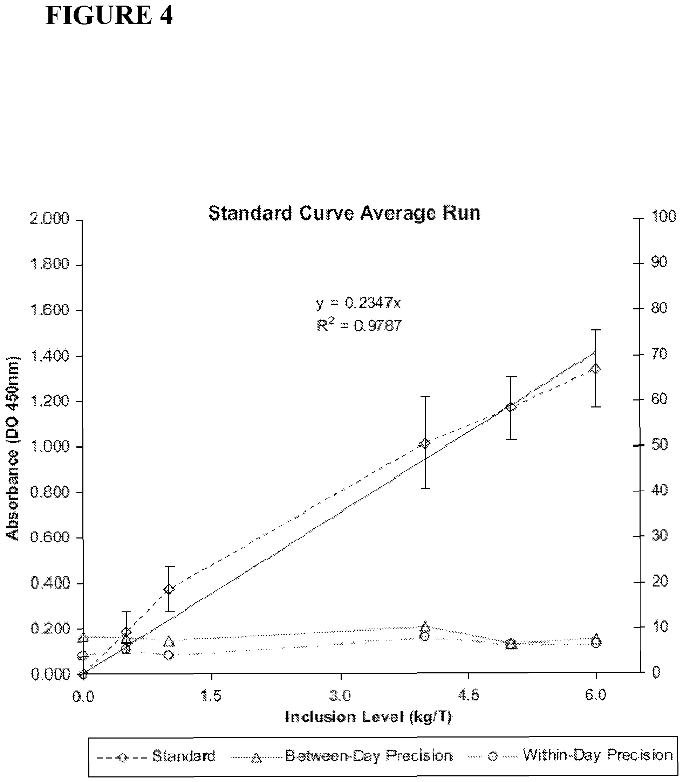

The calibration of the assay was performed on the feed admixed with known standard sample concentration as described herein and in the range 0.0 to 6.0 kg/T of MYCOSORB product. A linear curve fit, *y = Ax*, was used to define the relationship between concentration and response ($OD_{450}$). The average standard curve from 5 runs is shown in Figure 4.

*Linearity*

The linearity of the standard curves was evaluated from the calculation of the coefficient of regression according to the equation of the plotted inclusion levels against $OD_{450}$ absorbency using the best fitting line intercepting at zero, $\Delta OD_{450} = Ax$(MYCOSORB) after subtraction of the blank, The linearity of the standard curve is verified with a coefficient of correlation value found above $r^2 > 0.95$, Table 8.

**Table 8.** Linearity of the standard curves.

| Run # | 4 | 5 | 8 | 9 | 11 | Mean from averaged curve |
|---|---|---|---|---|---|---|
| Date | 6/10/09 | 6/17/09 | 6/30/09 | 7/14/09 | 8/5/09 | 1/6 Outlier |
| *n* | 50 | 50 | 50 | 50 | 50 | - |
| Slope (*A*) | 0.2702 | 0.2249 | 0.2161 | 0.2179 | 0.2444 | 0.2347 |
| Mean Square Error (*MSE*) * | 0.0037 | 0.0005 | 0.0034 | 0.0008 | 0.0009 | - |
| Linearity ($r^2$) | 0.969 | 0.974 | 0.970 | 0.949 | 0.978 | 0.979 |

* The mean square error is define in linear regressions models as: $MSE = \frac{1}{n}\sum_{i=1}^{n} e_i^2$, where $e_i = y_i - \hat{y}_i$ ; $y_i$ is the *y* value experimentally measured; $\hat{y}_i$ is the theoretical value for the expected $x_i$ concentration of MYCOSORB product.

*Repeatability*

The repeatability is a measure of the internal variance by the evaluation of the repeatability standard deviation. The evaluation of the repeatability within the repetition of a run has been obtained from the same sample at the same day for the same run, from the same technician, with the same equipment and method to represent the variation of the detection within a run, $CV_{intra}$ (Table 9). The evaluation of the repeatability between different runs was obtained from the same sample at different days during different runs, from the same technician, with the same equipment and method to represent the variation of the detection between runs, $CV_{Inter}$ Table 10).

The results show that the standard curves obtained on both the same day and on different days has little variability, with an average within-day precision of 5.70% and a between-day precision of 7.86% when applied to a standard curve working range between 0.0 and 6.0 kg/T. The overall precision was 8.28% (Table 11).

**Table 9**. Within-run repeatability precision for standard curve building.

| Concentration (kg/T) | | 0.0 | 0.5 | 1.0 | 4.0 | 5.0 | 6.0 |
|---|---|---|---|---|---|---|---|
| Day 1 Run #4 | $OD_{450\ nm}$ | 1.022 | 1.221 | 1.425 | 2.284 | 2.292 | 2.564 |
| | $\sigma_{intra}$ | 0.033 | 0.056 | 0.117 | 0.071 | 0.062 | 0.132 |
| | % CV | 3.26 | 4.60 | 8.20 | 3.11 | 2.69 | 5.14 |
| Day 2 Run #5 | $OD_{450\ nm}$ | 1.024 | 1.238 | 1.335 | 2.039 | 2.076 | 2.335 |
| | $\sigma_{intra}$ | 0.031 | 0.073 | 0.030 | 0.215 | 0.077 | 0.153 |
| | % CV | 3.03 | 5.91 | 2.21 | 10.54 | 3.72 | 6.55 |
| Day 3 Run #8 | $OD_{450\ nm}$ | 0.871 | 1.066 | 1.231 | 1.807 | 1.953 | 2.088 |
| | $\sigma_{intra}$ | 0.044 | 0.053 | 0.051 | 0.136 | 0.192 | 0.127 |
| | % CV | 5.06 | 4.96 | 4.17 | 7.53 | 9.82 | 6.08 |
| Day 4 Run #9 | $OD_{450\ nm}$ | 1.049 | 1.218 | 1.468 | 2.026 | 2.138 | 2.247 |
| | $\sigma_{intra}$ | 0.041 | 0.059 | 0.035 | 0.266 | 0.095 | 0.141 |
| | % CV | 3.93 | 4.86 | 2.39 | 13.12 | 4.44 | 6.26 |
| Day 5 Run #11 | $OD_{450\ nm}$ | 0.915 | 1.053 | 1.284 | 1.787 | 2.251 | 2.336 |
| | $\sigma_{intra}$ | 0.044 | 0.067 | 0.049 | 0.095 | 0.247 | 0.189 |
| | % CV | 4.79 | 6.34 | 3.85 | 5.30 | 10.98 | 8.09 |
| **% $CV_{intra}$** (within-run precision) | | 4.01 | 5.33 | 4.16 | 7.92 | 6.33 | 6.42 |
| **Average precision, % $CV_{intra}$** | | 5.70 | | | | | |

**Table 10.** Between-run repeatability precision for standard curve building.

| Concentration (kg/T) | 0.0 | 0.5 | 1.0 | 4.0 | 5.0 | 6.0 |
|---|---|---|---|---|---|---|
| $OD_{450\ nm}$ | 0.976 | 1.159 | 1.348 | 1.988 | 2.142 | 2.314 |
| $\sigma_{Inter}$ | 0.078 | 0.091 | 0.098 | 0.203 | 0.136 | 0.172 |
| % $CV_{Inter}$ | 8.01 | 7.88 | 7.27 | 10.22 | 6.36 | 7.45 |
| **Average precision, % $CV_{Inter}$** (between-run precision) | 7.86 | | | | | |

**Table 11.** Overall precision for standard curve building.

| Concentration (kg/T) | 0.0 | 0.5 | 1.0 | 4.0 | 5.0 | 6.0 |
|---|---|---|---|---|---|---|
| $n$ | 30 | 30 | 30 | 30 | 30 | 30 |
| $\sigma_{intra}$ | 0.039 | 0.062 | 0.056 | 0.157 | 0.135 | 0.148 |
| $\sigma_{Inter}$ | 0.078 | 0.091 | 0.098 | 0.203 | 0.136 | 0.172 |
| $\sigma_{Total}$ | 0.080 | 0.095 | 0.101 | 0.215 | 0.149 | 0.185 |
| **Precision, % $CV_{Total}$** | 8.20 | 8.23 | 7.50 | 10.81 | 6.95 | 7.98 |
| **Overall Precision, % $CV_{Total}$** | 8.28 | | | | | |

**$L_D$ and $L_Q$ (sensitivity)**

The $L_D$ and $L_Q$ values are reported in Table 12. The $L_Q$ values found were below the minimum recommended inclusion rate of 2 kg/T of the MYCOSORB product, enabling its quantification in feed material.

Detection Limit $L_D$ = 0.501 $\pm$ 0.103 kg/T

Quantification Limit $L_Q$ = 1.800 $\pm$ 0.389 kg/T

**Table 12.** $L_D$ and $L_Q$ determination.

| Run # | n | OD $_{450}$ blank | | 3 $\sigma$ ($\Delta$ OD $_{450}$) | 10$\sigma$ ($\Delta$ OD $_{450}$) | $L_D$ (kg/T) | $L_Q$ (kg/T) |
|---|---|---|---|---|---|---|---|
| | | $x_{b\ inter}$ - $\overline{x}_{b\ Intra}$ | $\sigma_{blank}$ | | | | |
| Day 1 | 10 | 0.046 | 0.033 | 0.100 | 0.334 | 0.370 | 1.323 |
| Day 2 | 10 | 0.048 | 0.031 | 0.093 | 0.311 | 0.415 | 1.488 |
| Day 3 | 10 | -0.105 | 0.044 | 0.132 | 0.441 | 0.612 | 2.233 |
| Day 4 | 10 | 0.072 | 0.041 | 0.124 | 0.412 | 0.568 | 2.100 |
| Day 5 | 10 | 0.915 | 0.044 | 0.132 | 0.438 | 0.538 | 1.858 |
| Average | | | | 0.116$\pm$0.018 | 0.387$\pm$0.061 | 0.501$\pm$0.103 | 1.800$\pm$0.389 |

*Accuracy*

Accuracy was measured from 5 independent experiments comprising 10 independent samples each and run in 4 replicates each for the 4.0kg/T concentration of MYCOSORB product admixed to the feed material. The final concentration for each sample was calculated according to the equation expressed *supra* using the observed values obtained for the homogeneity calculation (Table 13). Then the average precision was calculated from the averaged accuracy obtained for all of the runs and compared to the spiked concentration amount of 4.0kg/T of MYCOSORB product admixed to the feed material. The calculations were performed according to the equations given *supra.*

The results show that the recovery evaluation accounted for an overestimation of 21 % of the real content of the feed material in spiked concentration of MYCOSORB product (Table 14).

**Table 13** . Accuracy and precision measured from 5 independent experiments comprising 10 independent samples each and run in 4 replicates each for the 4.0kg/T concentration of **MYCOSORB** product admixed to the feed material.

| Run # | $x_{TH,}$ Spike (kg/T) | Replicate #, $x_c$ (OD $_{450}$) | | | | | | | | | | $\overline{x}_c$ | % CV |
|---|---|---|---|---|---|---|---|---|---|---|---|---|---|
| | | 1 | 2 | 3 | 4 | 5 | 6 | 7 | 8 | 9 | 10 | | |
| Day 1 | 4.0 | 5.0 | 4.0 | 5.1 | 3.8 | 4.3 | 4.3 | 3.9 | 4.4 | 3.9 | 4.1 | 4.3 | 9.69 |
| Day 2 | 4.0 | 4.2 | 5.0 | 5.1 | 4.7 | 4.6 | 4.6 | 4.5 | 4.2 | 4.8 | 5.2 | 4.7 | 7.19 |
| Day 3 | 4.0 | 3.6 | 3.6 | 4.1 | 4.4 | 3.8 | 3.9 | 3.7 | 3.9 | 3.3 | 4.3 | 3.9 | 8.79 |
| Day 4 | 4.0 | 5.4 | 5.0 | 5.0 | 4.9 | 5.2 | 5.6 | 5.1 | 5.5 | 5.7 | 6.1 | 5.4 | 6.73 |
| Day 5 | 4.0 | 4.4 | 4.5 | 3.7 | 4.4 | 4.2 | 4.4 | 3.5 | 4.4 | 4.3 | 4.9 | 4.3 | 9.10 |
| % $CV_{intra}$ (within-run precision) | | | | | | | | | | | | | 8.30 |

**Table 14.** Overall recovery measured from the average of 5 independent experiments comprising 10 independent samples each and run in 4 replicates each for the 4.0/kg/T concentration of MYCOSORB product admixed to the feed material.

| $x_{TH,}$ Spike (kg/T) | Average $\overline{x}_c$ | $\sigma_c$ |
|---|---|---|
| 4.0 | 4.5 | 0.6 |
| % $CV_{Inter}$ (between-run precision) | | 12.62 |
| **Overall Recovery** (% theoretical concentration) | | 112.21 |

*Validation using chicken feed material*

Tests were performed in the Center for Animal Nutrigenomics and Applied Animal Nutrition at ALLTECH, Inc., KY, USA on a solid chicken feed formulation admixed with five different concentrations of MYCOSORB product.

The chicken feed materials individually admixed with 0.0; 0.5; 1.0; 4.0; 5.0; 6.0 kg/T of MYCOSORB product was analyzed as well as a sample of the same chicken feed material admixed with an unknown level of MYCOSORB product when distributed on a same micro-titer plate.

*Applicability*

The validation was performed in order to determine the characteristics of an ELISA assay developed for the analysis of the MYCOSORB product and alternates, as a traceability endpoint procedure for the purpose of tracking the earlier mentioned product in complex feedstuffs.

Six levels of a MYCOSORB product (Ref. #285965) were used for the validation corresponding to the working range of level of inclusion (0.0; 0.5; 1.0; 4.0; 5.0; 6.0 kg/T) admixed to feed sample.

Diluted rabbit polyclonal antibody solution: the primary antibody dilution was adapted according to the background levels. The antibodies were thus diluted to 1:7,500 in PBS by adding 1 $\mu$l of antibody solution #1 (4.15) to 7.5 ml of PBS, 1X. A 10 ml volume of solution was needed for each microtiter plate.

Diluted goat anti-rabbit antibody: the secondary antibody dilution was adapted according to the background levels. The antibodies were thus diluted to 1:30,000 in PBS by adding 1 $\mu$l of antibody solution #2 to 30.0 ml PBS, 1X.

*Homogeneity*

The homogeneity verification of the sample was achieved for a median level of inclusion of MYCOSORB product (4.0 kg/T) in the experimental plan. The assay for this particular level was repeated 5 times on 10 individual samples using 4 replicates each (Table 15).

**Table15.** Homogeneity evaluation on a single concentration of 4.0 kg/T of MYCOSORB product admixed to chicken feed material evaluated through the ELISA procedure for 5 runs on 10 individual sample preparation analyzed in 4 replicates.

| Run # | 2 | 3 | 4 | 5 | 6 | Average |
|---|---|---|---|---|---|---|
| $n$ | 4 × 10 | 4 × 10 | 4 × 10 | 4 × 10 | 4 × 10 | 4 × 10 |
| Average OD 450 nm | 1.419 | 0.908 | 0.777 | 0.991 | 1.290 | 1.034 |
| $\sigma_h$ | 0.048 | 0.060 | 0.081 | 0.060 | 0.069 | - |
| % $CV_h$ | 3.08 | 6.11 | 10.38 | 5.65 | 6.39 | - |
| Homogeneity, % $CV_H$ | 6.32 | | | | | |

*Calibration*

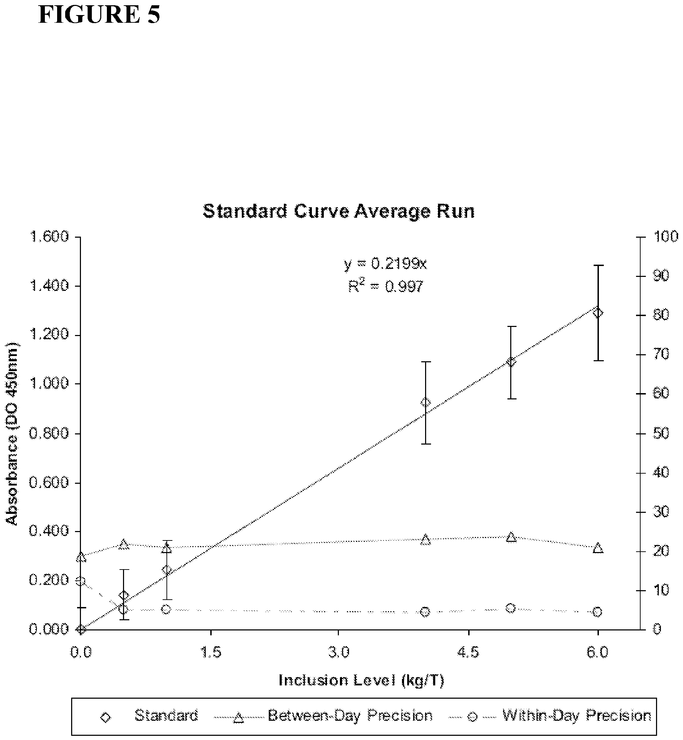

The calibration of the assay was performed on the feed admixed with known standard sample concentration and in the range 0.0 to 6.0 kg/T of MYCOSORB product, A linear curve fit, $y = Ax$, was used to define the relationship between concentration and response ($OD_{450}$), The average standard curve from 5 runs was compiled in Figure 5.

*Linearity*

The linearity of the standard curves was evaluated from the calculation of the coefficient of regression according to the equation of the plotted inclusion levels against $OD_{450}$ absorbency using the best fitting line intercepting at zero, $\Delta OD_{450} = Ax$ (MYCOSORB) after subtraction of the blank, The linearity of the standard curve is verified with a coefficient of correlation value found above $r^2 > 0.95$. The coefficient of regression calculated for each standard run curves and for the averaged standard run curve is reported in Table 16.

**Table 16.** Linearity of the standard curves in the chicken feed material.

| Run # | 2 | 3 | 4 | 5 | 6 | Mean from averaged curve |
|---|---|---|---|---|---|---|
| Date | 4/21/09 | 4/22/09 | 4/23/09 | 4/24/09 | 4/28/09 | 1/6 Outlier |
| n | 50 | 50 | 50 | 50 | 50 | - |
| Slope (*A*) | 0.2954 | 0.1904 | 0.1555 | 0.2282 | 0.2302 | 0.2199 |
| Mean Square Error (*MSE*) * | 0.0123 | 0.0018 | 0.0118 | 0.0003 | 0.0006 | - |
| Linearity (r$^2$) | 0.995 | 0.999 | 0.991 | 0.992 | 0.992 | 0.997 |

* The mean square error is define in linear regressions models as: $MSE = \frac{1}{n}\sum_{i=1}^{n} e_i^2$, where $e_i^2 = y_i - \hat{y}_i$; $y_i$ is the y value experimentally measured; $\hat{y}_i$ is the theoretical value for the expected $x_i$ concentration of MYCOSORB product.

### *Repeatability*

The repeatability is a measure of the internal variance by the evaluation of the repeatability standard deviation. The evaluation of the repeatability within the repetition of a run was obtained from the same sample at the same day for the same run, from the same technician, with the same equipment and method to represent the variation of the detection within a run, $CV_{intra}$ (Table 17). The evaluation of the repeatability between different runs was obtained from the same sample at different days during different runs, from the same technician, with the same equipment and method to represent the variation of the detection between runs, $CV_{Inter}$ (Table 18).

The results show that the standard curves obtained on both the same day and on different days has little variability, with an average within-day precision of 6.00%. However, the difference between samples was more pronounced with a between-day precision of 21.52% and an overall precision of 21.75% (Table 19) when applied to a standard curve working range between 0.0 and 6.0 kg/T.

**Table 17.** Within-run repeatability precision for standard curve building.

| Concentration (kg/T) | | 0.0 | 0.5 | 1.0 | 4.0 | 5.0 | 6.0 |
|---|---|---|---|---|---|---|---|
| Day 1 Run #2 | OD$_{450\,nm}$ | 0.380 | 0.606 | 0.728 | 1.622 | 1.837 | 2.114 |
| | $\sigma_{intra}$ | 0.031 | 0.018 | 0.041 | 0.104 | 0.070 | 0.098 |
| | % CV | 8.17 | 3.01 | 5.59 | 6.40 | 3.82 | 4.62 |
| Day 2 Run #3 | OD$_{450\,nm}$ | 0.310 | 0.401 | 0.492 | 1.092 | 1.237 | 1.463 |
| | $\sigma_{intra}$ | 0.030 | 0.034 | 0.021 | 0.030 | 0.016 | 0.050 |
| | % CV | 9.55 | 8.44 | 4.30 | 2.77 | 1.32 | 3.40 |
| Day 3 Run #4 | OD$_{450\,nm}$ | 0.227 | 0.344 | 0.418 | 0.856 | 0.950 | 1.192 |
| | $\sigma_{intra}$ | 0.019 | 0.017 | 0.020 | 0.025 | 0.041 | 0.035 |
| | % CV | 8.14 | 5.08 | 4.86 | 2.89 | 4.31 | 2.97 |
| Day 4 Run #5 | OD$_{456\,nm}$ | 0.343 | 0.457 | 0.617 | 1.338 | 1.491 | 1.644 |
| | $\sigma_{intra}$ | 0.058 | 0.032 | 0.029 | 0.057 | 0.140 | 0.124 |
| | % CV | 17.01 | 6.96 | 4.70 | 4.23 | 9.42 | 7.52 |
| Day 5 Run #6 | OD$_{450\,nm}$ | 0.361 | 0.517 | 0.588 | 1.341 | 1.561 | 1.661 |
| | $\sigma_{intra}$ | 0.064 | 0.011 | 0.035 | 0.069 | 0.109 | 0.047 |
| | % CV | 17.61 | 2.09 | 5.99 | 5.12 | 6.99 | 2.84 |
| % $CV_{intra}$ (within-run precision) | | 12.10 | 5.12 | 5.09 | 4.28 | 5.17 | 4.27 |
| Average precision, % $CV_{intra}$ | | 6.00 | | | | | |

**Table 18.** Between-run repeatability precision for standard curve building.

| Concentration (kg/T) | 0.0 | 0.5 | 1.0 | 4.0 | 5.0 | 6.0 |
|---|---|---|---|---|---|---|
| $OD_{450\,nm}$ | 0.324 | 0.465 | 0.568 | 1.250 | 1.415 | 1.615 |
| $\sigma_{Inter}$ | 0.060 | 0.102 | 0.119 | 0.289 | 0.337 | 0.337 |
| % $CV_{Inter}$ | 18.54 | 21.88 | 20.93 | 23.14 | 23.78 | 20.86 |
| **Average precision, % $CV_{Inter}$** (between-run precision) | 21.52 | | | | | |

**Table 19.** Overall precision for standard curve building.

| Concentration (kg/T) | 0.0 | 0.5 | 1.0 | 4.0 | 5.0 | 6.0 |
|---|---|---|---|---|---|---|
| $n$ | 30 | 30 | 30 | 30 | 30 | 30 |
| $\sigma_{intra}$ | 0.059 | 0.034 | 0.034 | 0.071 | 0.060 | 0.060 |
| $\sigma_{Inter}$ | 0.060 | 0.102 | 0.119 | 0.289 | 0.337 | 0.337 |
| $\sigma_{Total}$ | 0.063 | 0.102 | 0.120 | 0.290 | 0.338 | 0.338 |
| **Precision, % $CV_{Total}$** | 19.35 | 21.99 | 21.05 | 23.22 | 23.90 | 20.96 |
| **Overall Precision, % $CV_{Total}$** | 21.75 | | | | | |

### $L_D$ and $L_Q$ (sensitivity)

The $L_D$ and $L_Q$ values are reported in Table 20. The $L_Q$ values found are below the recommended inclusion rate of the MYCOSORB product enabling its quantification in feed material.

Detection Limit       $L_D = 0.547 \pm 0.237$ kg/T.

Quantification Limit       $L_Q = 1.864 \pm 0.806$ kg/T.

**Table 20.** $L_D$ and $L_Q$ determination in Chicken feed material.

| | n | $OD_{450}$ blank | | $L_D(\triangle OD_{450})$ | $L_Q(\triangle OD_{450})$ | $x_D$ (kg/T) | $x_Q$ (kg/T) |
|---|---|---|---|---|---|---|---|
| | | $\overline{x}_{b\,inter}$-$\overline{x}_{b\,Intra}$ | $\sigma_{blank}$ | | | | |
| Day 1 | 10 | 0.056 | 0.031 | 0.093 | 0.311 | 0.316 | 1.093 |
| Day 2 | 10 | -0.014 | 0.030 | 0.089 | 0.297 | 0.467 | 1.552 |
| Day 3 | 10 | -0.097 | 0.019 | 0.056 | 0.185 | 0.357 | 1.228 |
| Day 4 | 10 | 0.018 | 0.058 | 0.175 | 0.583 | 0.767 | 2.622 |
| Day 5 | 10 | 0.037 | 0.064 | 0.191 | 0.636 | 0.829 | 2.827 |
| **Average** | | | | | 0.121±0.059 | 0.402±0.196 | 0.547±0.237 |

### *Accuracy*

Accuracy was measured from 5 independent experiments comprising 10 independent samples each and run in 4 replicates each for the 4.0kg/T concentration of MYCOSORB product admixed to the feed material. The final concentration for each sample was calculated as described herein using the observed values obtained for the homogeneity calculation (Table 21). Then the average precision was calculated from the averaged accuracy obtained for all of the runs and compared to the spiked concentration amount of 4.0kg/T of MYCOSORB product admixed to the feed material. The calculations were performed according to the equations given herein.

The results show that the recovery accounted for an overestimation of 18% of the real content of the feed material in spiked concentration of MYCOSORB product (Table 22).

**Table 21.** Accuracy and precision measured from 5 independent experiments comprising 10 independent samples each and run in 4 replicates each for the 4.0 kg/T concentration of MYCOSORB product admixed to the feed material.

| Run # | $x_{TH}$, Spike (kg/T) | Replicate #, $x_c$(OD $_{450}$) | | | | | | | | | | $\overline{x}_c$ | % CV |
|---|---|---|---|---|---|---|---|---|---|---|---|---|---|
| | | 1 | 2 | 3 | 4 | 5 | 6 | 7 | 8 | 9 | 10 | | |
| Day 1 | 4.0 | 4.8 | 5.2 | 4.8 | 4.7 | 4.8 | 4.6 | 5.0 | 4.8 | 4.7 | 4.7 | 4.8 | 2.92 |
| Day 2 | 4.0 | 5.0 | 4.6 | 4.9 | 4.5 | 4.9 | 4.2 | 4.6 | 5.0 | 5.0 | 4.9 | 4.8 | 5.80 |
| Day 3 | 4.0 | 4.9 | 6.2 | 4.6 | 5.5 | 4.9 | 5.0 | 4.7 | 5.0 | 5.0 | 4.3 | 5.0 | 9.84 |
| Day 4 | 4.0 | 4.7 | 4.4 | 4.0 | 4.2 | 3.9 | 4.7 | 4.4 | 4.4 | 4.3 | 4.3 | 4.3 | 5.36 |
| Day 5 | 4.0 | 4.1 | 4.3 | 4.8 | 4.4 | 4.8 | 4.6 | 4.8 | 5.0 | 5.0 | 5.0 | 4.7 | 6.060 |
| **% CV$_{intra}$** (within-run precision) | | | | | | | | | | | | | 6.00 |

**Table 22.** Overall recovery measured from the average of 5 independent experiments comprising 10 independent samples each and run in 4 replicates each for the 4.0 kg/T concentration of MYCOSORB product admixed to the feed material.

| $x_{TH}$, Spike(kg/T) | Average $\overline{x}_c$ | $\sigma_c$ |
|---|---|---|
| 4.0 | 4.7 | 0.2 |
| **% CV$_{inter}$**(between-run precision) | | 5.07 |
| **Overall Recovery** (% theoretical concentration) | | 117.95 |

### *Validation using pig feed material*

Tests were performed in the Center for Animal Nutrigenomics and Applied Animal Nutrition of ALLTECH, Inc., KY, USA on a solid pig feed formulation admixed with five different concentrations of MYCOSORB product.

The pig feed materials individually admixed with 0.0; 0.5; 1.0; 4.0; 5.0; 6.0 kg/T of MYCOSORB product was analyzed as well as a sample of the same pig feed material admixed with an unknown level of MYCOSORB product when distributed on a same micro-titer plate.

### *Applicability*

The validation was performed in order to determine the characteristics of an ELISA assay developed for the analysis of the MYCOSORB product and alternates, as a traceability endpoint procedure for the purpose of tracking the product in complex feedstuffs.

Six levels of a MYCOSORB product were used for the validation corresponding to the working range of level of inclusion (with 0.0; 0.5; 1.0; 4.0; 5.0; 6.0 kg/T) admixed to feed sample.

Diluted rabbit polyclonal antibody solution: the primary antibody dilution was adapted according to the background levels. The antibodies were thus diluted to 1:3,500 in PBS by adding 1 μl of antibody solution #1 to 3.5 ml of PBS, 1X. A 10 ml volume of solution was needed for each microtiter plate.

Diluted goat anti-rabbit antibody: the secondary antibody dilution was adapted according to the background levels. The antibodies were thus diluted to 1:30,000 in PBS by adding 1 μl of antibody solution #2 to 30.0 ml PBS, 1X.

### *Homogeneity*

The homogeneity verification of the sample was achieved for a median level of inclusion of MYCOSORB product (4.0 kg/T) in the experimental plan. The assay for this particular level was repeated 5 times on 10 individual samples using 4 replicates each (Table 23).

**Table 23.** Homogeneity evaluation on a single concentration of 4.0 kg/T of MYCOSORB product admixed to pig feed material evaluated through the ELISA procedure for 5 runs on 10 individual sample preparation analyzed in 4 replicates.

| Run # | 1 | 2 | 3 | 4 | 5 | Average |
|---|---|---|---|---|---|---|
| $n$ | $4 \times 10$ | $4 \times 10$ | $4 \times 10$ | $4 \times 10$ | $4 \times 10$ | $4 \times 10$ |
| Average OD 450 nm | 1.091 | 1.533 | 1.486 | 1.845 | 0.856 | 1.362 |
| $\sigma_{intra}$ | 0.109 | 0.161 | 0.077 | 0.199 | 0.048 | - |
| % $CV_h$ | 10.02 | 10.51 | 5.15 | 10.76 | 5.57 | - |
| Homogeneity, % $CV_H$ | 8.40 | | | | | |

### Calibration

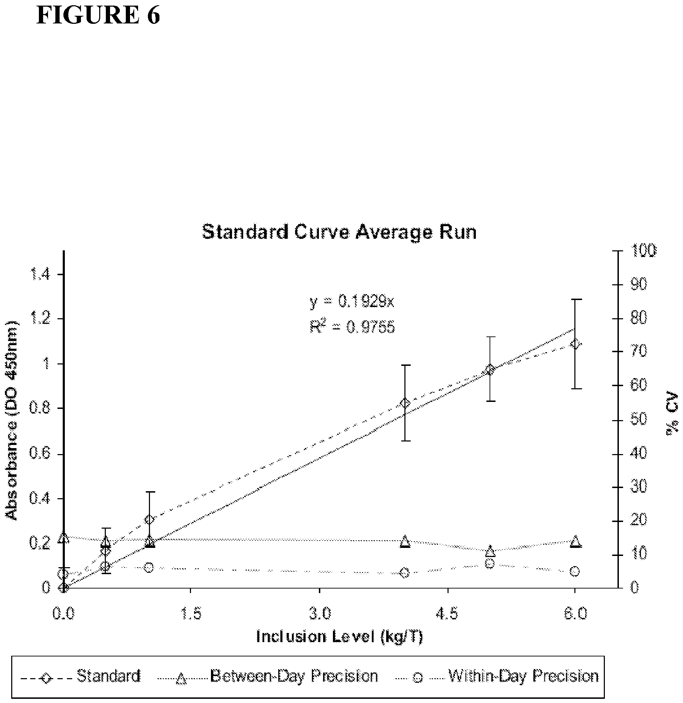

The calibration of the assay was performed on the feed admixed with known standard sample concentration and in the range 0.0 to 6.0 kg/T of MYCOSORB product, A linear curve fit, $y = Ax$ with intercept set to zero, was used to define the relationship between concentration and response ($OD_{450}$), The average standard curve from 5 runs was compiled in Figure 6.

### Linearity

The linearity of the standard curves was evaluated from the calculation of the coefficient of regression according to the equation of the plotted inclusion levels against $OD_{450}$ absorbency using the best fitting line, the best fitting line intercepting at zero, $\Delta OD_{450} = Ax$(MYCOSORB) after subtraction of the blank. The linearity of the standard curve is verified with a coefficient of correlation value found above $r^2 > 0.95$, The coefficient of regression calculated for each standard run curves and for the averaged standard run curve is reported in Table 24.

**Table 24.** Linearity of the standard curves in the pig feed material.

| Run # | 1 | 2 | 3 | 4 | 5 | Mean from averaged curve |
|---|---|---|---|---|---|---|
| Date | 5/7/09 | 5/12/09 | 5/13/09 | 5/22/09 | 8/18/09 | 1/6 Outlier |
| $n$ | 50 | 50 | 50 | 50 | 50 | - |
| Slope ($A$) | 0.2522 | 0.2593 | 0.2620 | 0.2856 | 0.1818 | 0.1929 |
| Mean Square Error ($MSE$)* | 0.0017 | 0.0021 | 0.0020 | 0.0073 | 0.0181 | - |
| Linearity ($r^2$) | 0.996 | 0.995 | 0.990 | 0.974 | 0.987 | 0.997 |

The mean square error is define in linear regressions models as: $$MSE = \frac{1}{n}\sum_{i=1}^{n} e_i^2, \qquad e_i^2 = y_i - \hat{y}_i;$$ where $y_i$ is the $y$ value experimentally measured; $\hat{y}_i$ is the theoretical value for the expected $x_i$ concentration of MYCOSORB product.

### Repeatability

The repeatability is a measure of the internal variance by the evaluation of the repeatability standard deviation. The evaluation of the repeatability within the repetition of a run has been obtained from the same sample at the same day for the same run, from the same technician, with the same equipment and method to represent the variation of the detection within a run, $C_{intra}$ (Table 25). The evaluation of the repeatability between different runs was obtained from the same sample at different days during different runs, from the same technician, with the same equipment and method to represent the variation of the detection between runs, $CV_{Inter}$ (Table 26).

The results show that the standard curves obtained on both the same day and on different days has little variability, with an average within-day precision of 7% and a between-day precision of 20% when applied to a standard curve working range between 0.0 and 6.0 kg/T.

**Table 25.** Within-run repeatability precision for standard curve building.

| Concentration (kg/T) | | 0.0 | 0.5 | 1.0 | 4.0 | 5.0 | 6.0 |
|---|---|---|---|---|---|---|---|
| Day 1 Run #1 | $OD_{450\,nm}$ | 0.284 | 0.430 | 0.540 | 1.372 | 1.542 | 1.757 |
| | $\sigma_{intra}$ | 0.012 | 0.040 | 0.055 | 0.051 | 0.111 | 0.121 |
| | % CV | 4.29 | 9.35 | 10.19 | 3.70 | 7.22 | 6.90 |
| Day 2 Run #2 | $OD_{450\,nm}$ | 0.279 | 0.419 | 0.598 | 1.376 | 1.510 | 1.839 |
| | $\sigma_{intra}$ | 0.035 | 0.028 | 0.011 | 0.040 | 0.201 | 0.137 |
| | % CV | 12.59 | 6.61 | 1.79 | 2.92 | 13.35 | 7.46 |
| Day 3 Run #3 | $OD_{450\,nm}$ | 0.259 | 0.432 | 0.490 | 1.417 | 1.590 | 1.742 |
| | $\sigma_{intra}$ | 0.020 | 0.007 | 0.028 | 0.118 | 0.044 | 0.174 |
| | % CV | 7.91 | 1.56 | 5.67 | 8.34 | 2.75 | 10.02 |
| Day 4 Run #4 | $OD_{450\,nm}$ | 0.305 | 0.455 | 0.737 | 1.447 | 1.556 | 2.142 |
| | $\sigma_{intra}$ | 0.027 | 0.024 | 0.101 | 0.271 | 0.104 | 0.060 |
| | % CV | 9.01 | 5.17 | 13.70 | 18.73 | 6.70 | 2.81 |
| Day 5 Run #5 | $OD_{450\,nm}$ | 0.236 | 0.308 | 0.374 | 0.735 | 0.876 | 0.984 |
| | $\sigma_{intra}$ | 0.020 | 0.018 | 0.017 | 0.029 | 0.048 | 0.023 |
| | % CV | 8.39 | 5.87 | 4.58 | 3.99 | 5.52 | 2.31 |
| % $CV_{intra}$ (within-run precision) | | 8.44 | 5.71 | 7.18 | 7.53 | 7.10 | 5.90 |
| Overall % ($CV_{intra}$) | | 6.98 | | | | | |

**Table 26.** Between-run repeatability precision for standard curve building.

| Concentration (kg/T) | 0.0 | 0.5 | 1.0 | 4.0 | 5.0 | 6.0 |
|---|---|---|---|---|---|---|
| $OD_{450\,nm}$ | 0.273 | 0.409 | 0.548 | 1.269 | 1.415 | 1.693 |
| $\sigma_{Inter}$ | 0.026 | 0.058 | 0.134 | 0.300 | 0.302 | 0.428 |
| % $CV_{Inter}$ | 9.62 | 14.16 | 24.45 | 23.67 | 21.38 | 25.28 |
| *Average precision, % $CV_{Inter}$* (between-run precision) | 19.76 | | | | | |

**Table 27.** Overall precision for standard curve building.

| Concentration (kg/T) | 0.0 | 0.5 | 1.0 | 4.0 | 5.0 | 6.0 |
|---|---|---|---|---|---|---|
| n | 30 | 30 | 30 | 30 | 30 | 30 |
| $\sigma_{intra}$ | 0.023 | 0.023 | 0.042 | 0.102 | 0.102 | 0.103 |
| $\sigma_{Inter}$ | 0.026 | 0.058 | 0.134 | 0.300 | 0.302 | 0.428 |
| $\sigma_{Total}$ | 0.028 | 0.059 | 0.135 | 0.304 | 0.306 | 0.430 |
| *Precision, % $CV_{Total}$* | 10.33 | 14.39 | 24.70 | 23.94 | 21.63 | 25.42 |
| *Overall Precision, % $CV_{Total}$* | 20.07 | | | | | |

## $L_D$ and $L_Q$ (sensitivity)

The $L_D$ and $L_Q$ values are reported in Table 28. The $L_Q$ values found arc below the recommended inclusion rate of the MYCOSORB product, enabling its quantification in feed material.

Detection Limit $\quad L_D = 0.403 \pm 0.150$ kg/T
Quantification Limit $\quad L_Q = 1.363 \pm 0.498$ kg/T

**Table 28.** $L_D$ and $L_Q$ determination in Pig feed material.

| | $n$ | OD$_{450}$ blank | | 3 $\sigma$ ($\Delta$ OD$_{450}$) | 10$\sigma$ ($\Delta$ OD$_{450}$) | $L_D$ (kg/T) | $L_Q$ (kg/T) |
|---|---|---|---|---|---|---|---|
| | | $x_{b\,inter}$-$\overline{x}_{b\,Intra}$ | $\sigma_{blank}$ | | | | |
| Day 1 | 10 | 0.064 | 0.071 | 0.111 | 0.371 | 0.613 | 2.054 |
| Day 2 | 10 | -0.006 | 0.035 | 0.105 | 0.351 | 0.407 | 1.386 |
| Day 3 | 10 | -0.027 | 0.020 | 0.061 | 0.205 | 0.234 | 0.795 |
| Day 4 | 10 | 0.019 | 0.027 | 0.082 | 0.275 | 0.289 | 0.991 |
| Day 5 | 10 | -0.050 | 0.020 | 0.059 | 0.198 | 0.471 | 1.591 |
| **Average** | | | | 0.084±0.024 | 0.280±0.080 | 0.403±0.150 | 1.363±0.498 |

*Accuracy*

Accuracy was measured from 5 independent experiments comprising 10 independent samples each and run in 4 replicates each for the 4.0 kg/T concentration of MYCOSORB product admixed to the feed material. The final concentration for each sample was calculated as described herein using the observed values obtained for the homogeneity calculation (Table 29). Then the average precision was calculated from the averaged accuracy obtained for all of the runs and compared to the spiked concentration amount of 4.0 kg/T of MYCOSORB product admixed to the feed material. The calculations were performed according to the equations described herein.

The results show that the recovery evaluation accounted for an overestimation of 20% of the real content of the feed material in spiked concentration of MYCOSORB product (Table 30).

**Table 29.** Accuracy and precision measured from 5 independent experiments comprising 10 independent samples each and run in 4 replicates each for the 4.0 kg/T concentration of MYCOSORB product admixed to the feed material.

| Run # | $x_{TH}$, Spike (kg/T) | Replicate #, $x_c$ (OD$_{450}$) | | | | | | | | | | $\overline{x}_c$ | % CV |
|---|---|---|---|---|---|---|---|---|---|---|---|---|---|
| | | 1 | 2 | 3 | 4 | 5 | 6 | 7 | 8 | 9 | 10 | | |
| Day 1 | 4.0 | 4.3 | 4.6 | 3.6 | 3.8 | 4.1 | 3.4 | 5.2 | 4.4 | 5.0 | 4.2 | 4.3 | 12.80 |
| Day 2 | 4.0 | 6.1 | 4.9 | 4.1 | 4.5 | 4.6 | 4.5 | 4.7 | 4.2 | 5.2 | 5.7 | 4.8 | 12.50 |
| Day 3 | 4.0 | 4.9 | 4.4 | 4.3 | 5.1 | 4.6 | 4.9 | 4.6 | 4.4 | 4.7 | 5.1 | 4.7 | 5.91 |
| Day 4 | 4.0 | 5.0 | 4.2 | 5.9 | 4.3 | 5.6 | 5.4 | 5.4 | 5.6 | 6.6 | 5.6 | 5.4 | 12.64 |
| Day 5 | 4.0 | 4.6 | 4.8 | 4.9 | 4.9 | 4.8 | 5.5 | 4.4 | 5.3 | 4.6 | 5.3 | 4.9 | 6.97 |
| **% CV$_{intra}$** (within-run precision) | | | | | | | | | | | | | 10.16 |

**Table 30**. Overall recovery measured from the average of 5 independent experiments comprising 10 independent samples each and run in 4 replicates each for the 4.0 kg/T concentration of MYCOSORB product admixed to the feed material.

| $x_{TH}$, Spike (kg/T) | Average $\overline{x}_c$ | $\sigma_c$ |
|---|---|---|
| 4.0 | 4.4 | 0.3 |
| **% CV$_{Inter}$** (between-run precision) | | 8.47 |
| **Overall Recovery** (% theoretical concentration) | | 120.46 |

***Verification using chicken and pig feed material***

The ELISA in feed assay for MYCOSORB detection was performed in chicken and pig matrices, and verified in an independent laboratory and compared to the validation results. The assay was verified at Alimetrics, Ltd., Koskelontic 19B, Espoo, Finland.

The calibration of the assay was performed on 2 different days on the feed admixed with known standard sample concentration in the range 0.0 to 6.0 kg/T of MYCOSORB product. A linear curve fit with y = Ax with intercept set to zero was used to define the relationship between concentration and response ($OD_{450}$) in pig feedstuffs and in chicken feedstuffs. Absorbance and resulting calibration differed significantly between different runs; however, the absolute concentrations for the known and blind samples remain the same.

*Linearity*

The linearity of the standard curves was evaluated from the calculation of the coefficient of regression according to the equation of the plotted inclusion levels against $OD_{450}$ absorbency using the best fitting line, the best fitting line intercepting at zero, $\Delta OD_{450} = Ax$(MYCOSORB) after subtraction of the blank. The linearity of the standard curve is verified with a coefficient of correlation value found above $r^2 > 0.95$, The coefficient of correlation ranged from 0.991 to 0.994 (Table 31).

**Table 31.** Linearity of the standard curves in chicken and pig feed material.

| Run # | 1 | 2 | 3 | 4 | Mean from averaged curve |
|---|---|---|---|---|---|
| **Matrix** | Chicken feed | | Pig feed | | - |
| **Date** | 4/6/10 | 4/7/10 | 4/6/10 | 4/6/10 | - |
| **n** | 15 | 15 | 15 | 15 | - |
| **Slope (A)** | 0.0954 | 0.0775 | 0.0828 | 0.0809 | - |
| **Linearity (r²)** | 0.991 | 0.994 | 0.993 | 0.992 | 0.997 |

The mean square error is define in linear regressions models as:

$$MSE = \frac{1}{n}\sum_{i=1}^{n} e_i^2 , \qquad e_i^2 = y_i - \hat{y}_i ;$$

where $y_i$ is the y value experimentally measured; $\hat{y}_i$ is the theoretical value for the expected $x_i$ concentration of MYCOSORB product.

**$L_D$ and $L_Q$ (sensitivity)**

The $L_D$ and $L_Q$ values were determined in a 40% hydrochloric acid matrix. Estimate of the respective $L_D$ and $L_Q$ values were respectively of 0.35 and 1.20 kg/T of MYCOSORB admixed to the chicken feedstuffs and of 0.27 and 0.91 kg/T of MYCOSORB admixed to the pig feedstuffs (Table 32 and Table 33, respectively).

**Table 32.** Results obtained on the blank samples in two separate runs carried out at one day of interval and performed by the verification laboratory on the chicken feedstuffs.

| | Date | Sample ID | Sample Intake (g) | Result (kg/T) |
|---|---|---|---|---|
| **Day 1** | 4/6/2010 | 0.3 | 20 | 0.230 |
| | | 0.4 | 20 | 0.150 |
| | | 0.6 | 20 | 0.010 |
| **Day 2** | 4/7/2010 | 0.1 | 20 | 0.320 |
| | | 0.4 | 20 | 0.110 |
| | | 0.10 | 20 | 0.020 |
| **Average** | | | | 0.140 |

**Table 33.** Results obtained on the blank samples in two separate runs carried out at one day of interval and performed by the verification laboratory on the pig feedstuffs.

| | Date | Sample ID | Sample Intake (g) | Result (kg/T) |
|---|---|---|---|---|
| **Day 1** | 4/6/2010 | 0.2 | 20 | 0.230 |
| | | 0.3 | 20 | 0.290 |
| | | 0.4 | 20 | 0.082 |
| **Day 2** | 4/7/2010 | 0.1 | 20 | 0.220 |
| | | 0.3 | 20 | 0.110 |
| | | 0.7 | 20 | 0.064 |
| **Average** | | | | 0.170 |

### Accuracy and precision for "known" samples

Accuracy was measured from 2 independent experiments comprising 6 replicates each on a 4.0 kg/T concentration of MYCOSORB product admixed to the feed material. The average precision was calculated from the averaged accuracy obtained for all of the runs and compared to the spiked concentration amount of 4.0 kg/T of MYCOSORB product admixed to the feed material.

The values obtained met the criteria for both accuracy and precision, as follows.

The evaluation of the repeatability within the repetition of a run has been obtained from the same sample at the same day for the same run, from the same technician, with the same equipment and method to represent the variation of the detection within a run, $CV_{intra}$. The evaluation of the repeatability between different runs was obtained from the same sample at different days during different runs, from the same technician, with the same equipment and method to represent the variation of the detection between runs, $CV_{Inter}$.

The values obtained met the criteria for both accuracy and precision, as follows for the chicken and pig matrices (Table 34 and Table 35).

**Table 34.** Accuracy and precision measured from 6 replicate tests performed on 2 separate days using known samples (4.0 kg/T) of MYCOSORB product admixed to the chicken feed material.

| | Date | Sample ID | Sample Intake (g) | Results (kg/T) |
|---|---|---|---|---|
| **Day 1** | 3/4/2010 | H.1 | 20 | 4.4 |
| | | H.2 | 20 | 4.4 |
| | | H.3 | 20 | 3.9 |
| | | H.4 | 20 | 4.1 |
| | | H.5 | 20 | 4.4 |
| | | H.6 | 20 | 4.2 |
| **Day 2** | 3/5/2010 | H.7 | 20 | 3.8 |
| | | H.8 | 20 | 3.3 |
| | | H.9 | 20 | 3.7 |
| | | H.10 | 20 | 3.4 |
| | | H.11 | 20 | 3.8 |
| | | H.12 | 20 | 4.1 |
| **Average** | | | | 4.0 |
| **% $CV_{intra}$** (within-run precision) | | | 6.6 | |
| **% $CV_{Inter}$** (between-run precision) | | | 9.7 | |
| **Overall Recovery** (% theoretical concentration) | | | 99% | |

**Table 35.** Accuracy and precision measured from 6 replicate tests performed on 2 separate days using known samples (4.0 kg/T) of MYCOSORB product admixed to the pig feed material.

| | Date | Sample ID | Sample Intake (g) | Results (kg/T) |
|---|---|---|---|---|
| Day 1 | 3/4/2010 | H.1 | 20 | 4.7 |
| | | H.2 | 20 | 4.1 |
| | | H.3 | 20 | 4.4 |
| | | H.4 | 20 | 4.6 |
| | | H.5 | 20 | 3.8 |
| | | H.6 | 20 | 4.3 |
| Day 2 | 3/5/2010 | H.7 | 20 | 3.5 |
| | | H.8 | 20 | 3.7 |
| | | H.9 | 20 | 3.1 |
| | | H.10 | 20 | 3.2 |
| | | H.11 | 20 | 3.6 |
| | | H.12 | 20 | 3.5 |
| Average | | | | 3.9 |
| % CV$_{Intra}$ (within-run precision) | | | | 7.5 |
| % CV$_{Inter}$ (between-run precision) | | | | 14.0 |
| Overall Recovery (% theoretical concentration) | | | | 96% |

### Accuracy and precision for "unknown" samples

Accuracy was measured from 2 independent experiments comprising 3 replicates each on a 4.0 kg/T concentration of MYCOSORB product admixed to the feed material in a blind test. The values obtained met the criteria for both accuracy and precision, as follows (Table 36 and Table 37).

**Table 36.** Accuracy and precision measured from 3 replicate tests performed on unknown samples (4.0 kg/T) of MYCOSORB product admixed to the chicken feed material.

| Measurement Date | Sample ID (mL) | Sample Intake (g) | Results (kg/T) |
|---|---|---|---|
| 4/6/2010 | C.6 | 20 | 4.0 |
| | C.7 | 20 | 3.8 |
| | C.8 | 20 | 4.1 |
| Average | | | 4.0 |
| Repeatability Standard Deviation | | | 0.11 |
| RSD (%) | | | 3.0% |

**Table 37.** Accuracy and precision measured from 3 replicate tests performed on unknown samples (4.0 kg/T) of MYCOSORB product admixed to the pig feed material.

| Measurement Date | Sample ID (mL) | Sample Intake (g) | Results (kg/T) |
|---|---|---|---|
| 4/6/2010 | C.6 | 20 | 4.5 |
| | C.7 | 20 | 3.8 |
| | C.8 | 20 | 4.4 |

(continued)

| Measurement Date | Sample ID (mL) | Sample Intake (g) | Results (kg/T) |
|---|---|---|---|
| Average | | 4.2 | |
| Repeatability Standard Deviation | | 0.36 | |
| RSD (%) | | 8.5% | |

### *Ruggedness*

The ruggedness analysis evaluates the ability of the measurement method to resist changes in results when subjected to minor changes in environmental and procedure variables.

The stability of the ELISA assay was investigated by changing the primary and secondary antibody dilution, the stability of the primary antibody, by changing the distribution of the different samples to be tested on the microtiter plates (single plate containing all the samples to be measured and comprising the blank, the standards and the unknown sample vs. multiple microtiter plates comprising more repetitions per sample analyzed but where blank, standards and unknown are on different microtiter plates). Table 38 reports the variations and shows that results are consistent, even with the changes performed, indicating a strong ruggedness of the test especially to variation in antibody dilutions.

**Table 38 .** Ruggedness test. Standard conditions are shown in bold. Ten percent of alterations are also highlighted if found.

| Condition altered | Value of condition | Feed Sample | $x_c$ | Recovery | $CV_{intra}$ | $CV_{Inter}$ | CV Total |
|---|---|---|---|---|---|---|---|
| Primary Antibody, 2ndary Ab **1:20 000** | 1:2000 | Dairy | 4.45 | 120.98 | 7.90 | 10.85 | 12.06 |
| | 1:2500 | | 4.47 | 121.06 | 8.98 | 13.86 | 15.13 |
| | 1:4000 | | 4.44 | 121.01 | 10.54 | 10.18 | 14.12 |
| | 1:5000 | | 4.49 | 123.30 | 9.57 | 13.42 | 16.14 |
| Primary Antibody, 2ndary Ab 1:30 000 | 1:2000 | Dairy | 4.37 | 119.56 | 10.03 | 9.39 | 12.70 |
| | 1:2500 | | 4.55 | 121.75 | 8.86 | 14.58 | 15.73 |
| | 1:4000 | | 4.30 | 115.85 | 10.66 | 11.11 | 14.10 |
| | 1:5000 | | 4.35 | 117.29 | 9.91 | 14.55 | 16.33 |
| Primary Antibody stability | 2 - 8 °C | Chicken | 4.8 | 134.80 | 5.59 | - | - |
| | 1 week, RT | | 4.9 | 138.10 | 6.85 | - | - |
| Plate distribution | **Single plate** | Chicken | 4.72 | 120.68 | 6.13 | 5.19 | 8.07 |
| | Multiple plates | | 4.82 | 124.23 | 5.99 | 5.89 | 8.11 |

### Example 5

### Monoclonal Antibody Specificity Testing

### *Methods*

Seventeen compounds were tested at a concentration of 50 $\mu$g/ml in PBS to determine cross-reactivity by monoclonal antibodies raised against yeast (1→4)-$\alpha$-D-glucan/(1→6)-($\beta$-D-glucan-BSA conjugate.

Compounds tested were: Soluble starch-potato, starch-rice, starch-wheat, starch-corn, BSA, glycogen (from rabbit), glycogen (from oyster), glycogen (bovine), Mannan, Laminarin, Zymosin A, Maltrin QD, glucan from Baker's yeast, (1→3)-$\beta$-D-glucan from *Eug. gracilis,* Torula yeast, Ycw-02 bead beater (yeast cell wall fraction - bead beater preparation), $\beta$ glucan from barley.

Yeast cell wall extract (MYCOSORB batch #08FS001) and the (I→4)-$\alpha$-D-glucan/(1→6)-$\beta$-D-glucan-BSA antigen used to raise monoclonal antibody were also tested at concentrations of 1 $\mu$g/ml, 2 $\mu$g/ml and 5 $\mu$g/ml. Both monoclonal antibodies (513A161.1 and 513A431.1) were assayed at the following range of dilutions: 1:100, 1:200, 1:500, 1:1000, 1:1500 and 1:2000. Ninety-six well Nunc microtiter plates with the Lot#0702013 were used. Plates were coated

with 100 μl/well of compound and agitated by tapping 8-10 times with fingertips. The plates were covered with sealing tape and incubated for 1 h at 37° C. Solution was removed and plates were washed 3 times with 200 μl of PBS per wash using a multi-channel pipette. Plates were tapped 8-10 times with fingertips prior to removing each wash solution. Between washes, excess fluid was removed by tapping the plate on paper towels. For blocking, 100μl/well 3% milk (not centrifuged) was added. Plates were incubated 1 h at room temperature. Blocking solution was removed and wells were washed with 1 x 200 μl of PBS, 2 x 200 μl PBS + 0.05% polysorbate 20. Plates were agitated by tapping 8-10 times with fingertips prior to removal of each wash solution. Between washes, excess fluid was removed by tapping the plate on paper towels.

100μl of each dilution of appropriate sera was added per well. PBS (100 μl/well) was used for blank wells. The plate was incubated, covered, for 1 h at room temperature. Solution was removed and wells were washed 3 times with 200 μl PBS + 0.05% polysorbate 20 each. The plate was tapped 8-10 times with fingertips to agitate prior to removal of each wash. Between washes, excess fluid was removed by tapping the plate on paper towels. One hundred μl of the secondary antibody (1:10,000 goat anti-mouse, IgG-peroxidase conjugated) was added per well. The plate was incubated, covered, for 1h at room temperature. Solution was removed and wells were washed 3 times with 200 μl per wash PBS + 0.05% polysorbate 20. The plate was tapped 8-10 times with fingertips to agitate prior to removal of each wash. Between washes, excess fluid was removed by tapping the plate on paper towels.

100 μl room-temperature TMB substrate was added per well. After 5 minutes, the reaction was halted with 100 μl per well of IN HCl. Plate bottoms were wiped clean of fingerprints, and after a 10 second shake, absorbance at 450 nm was read using microtiter plate reader.

### Results

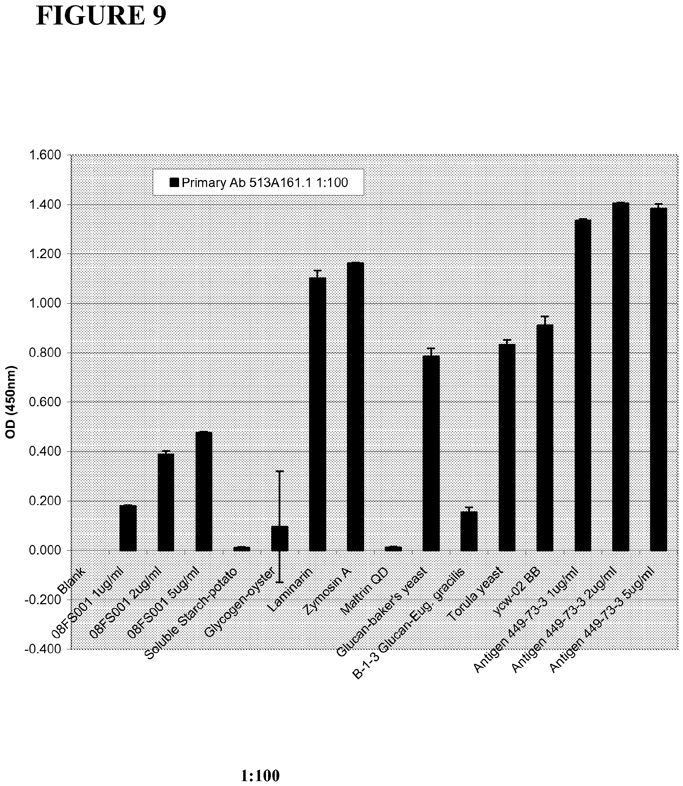

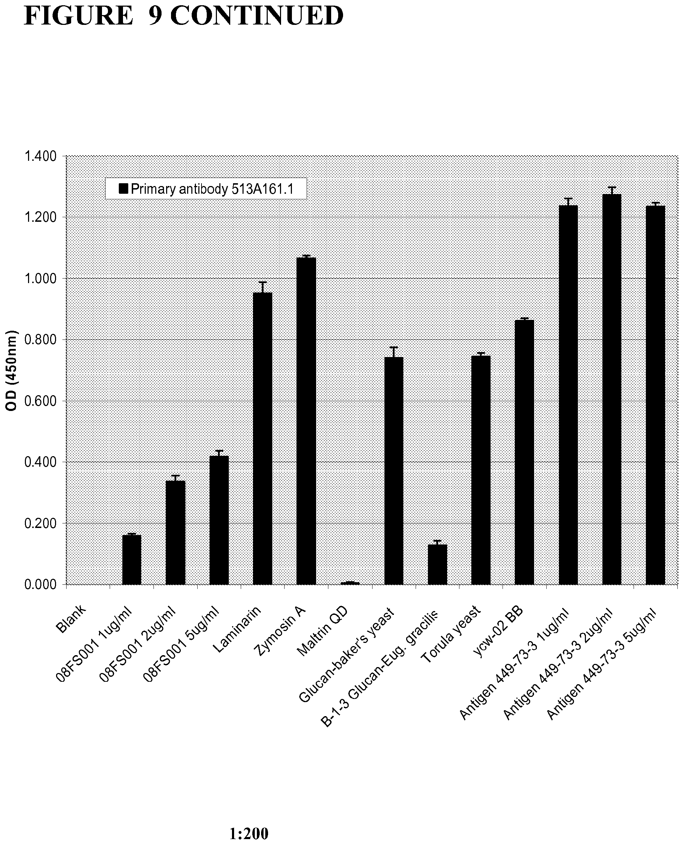

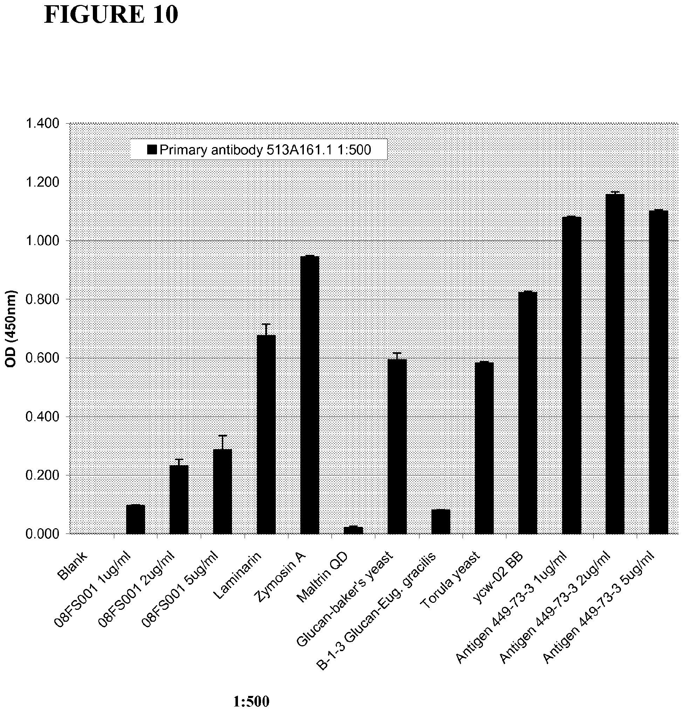

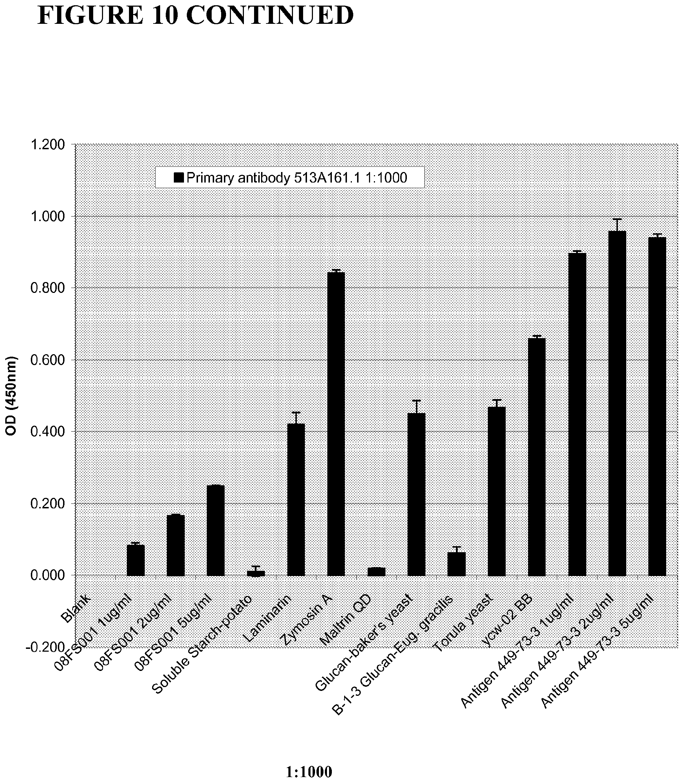

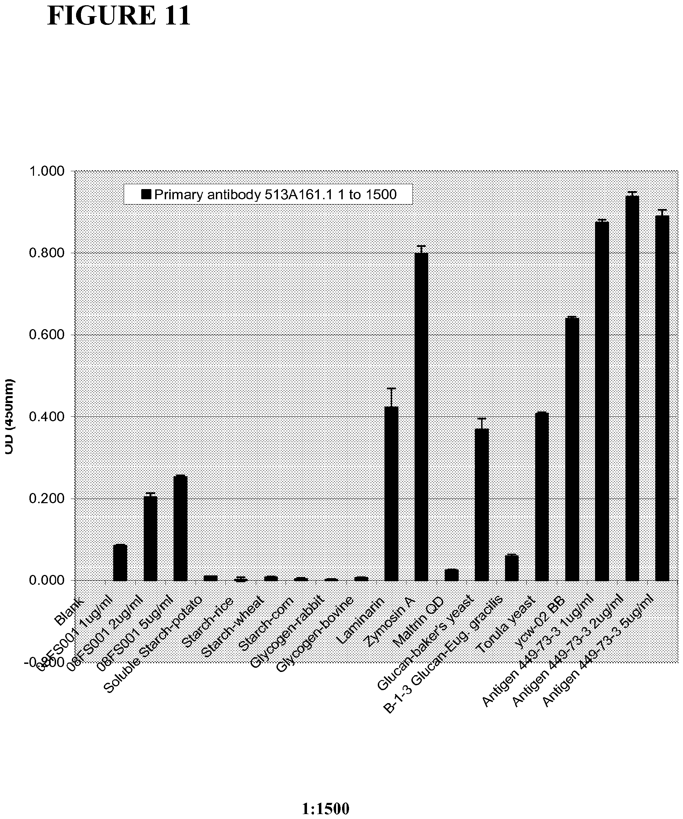

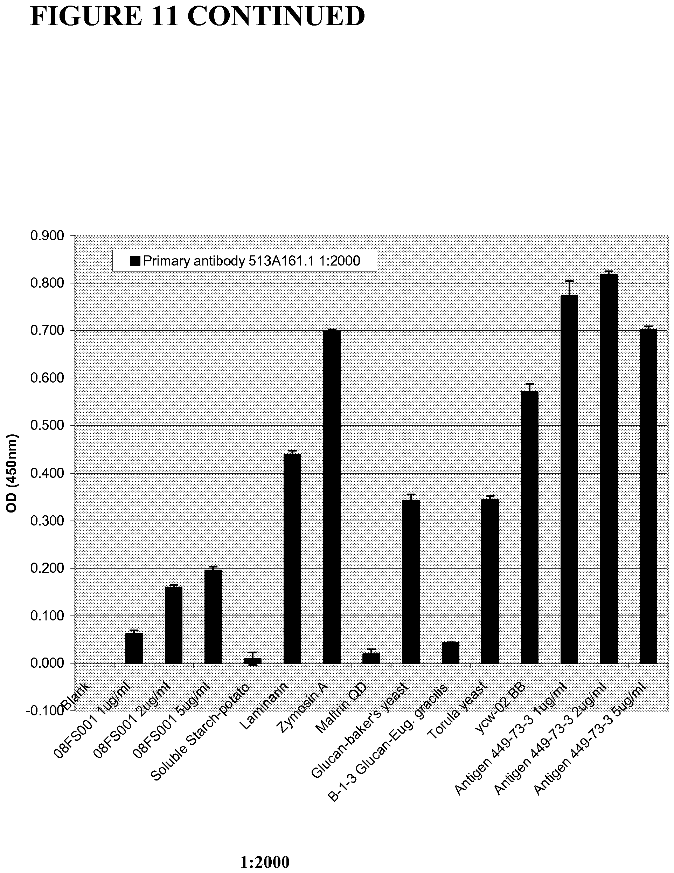

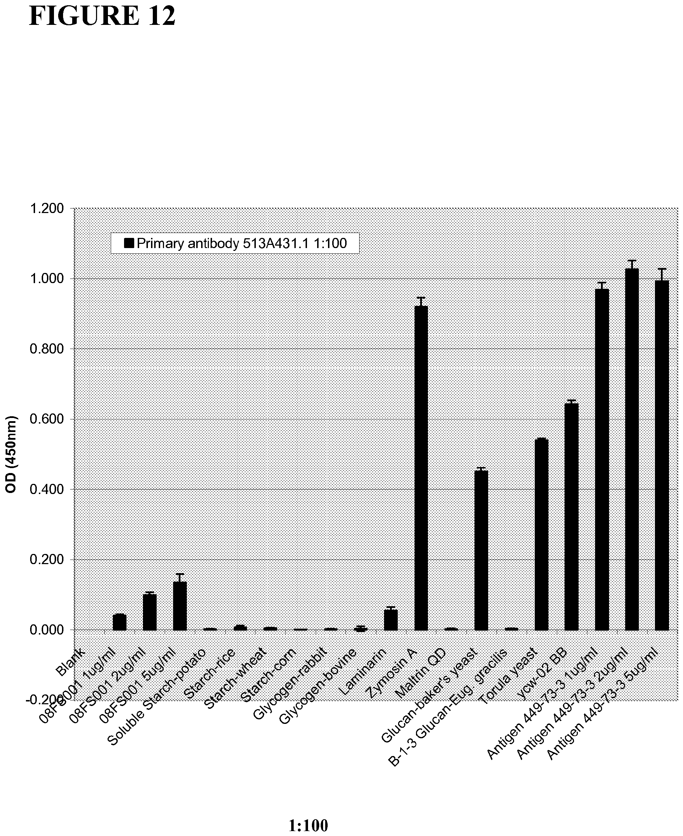

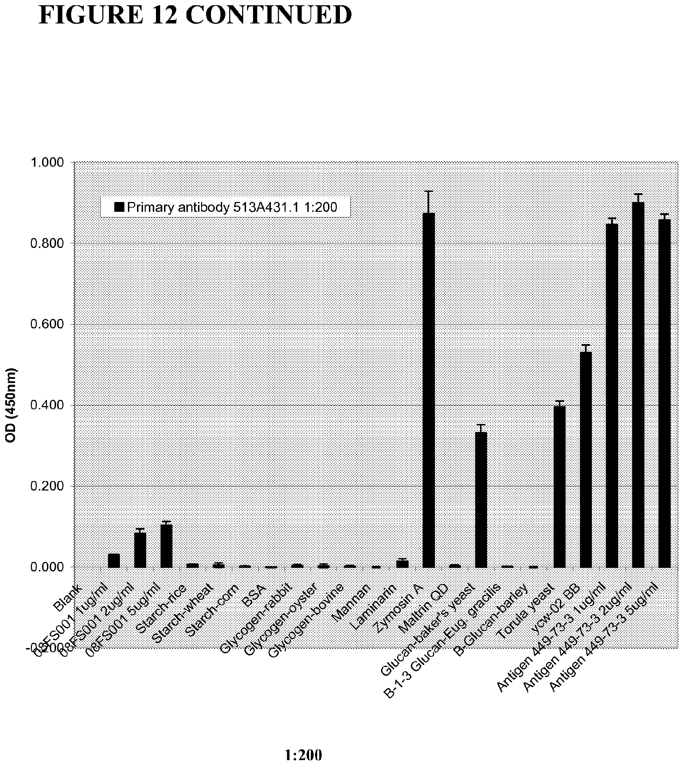

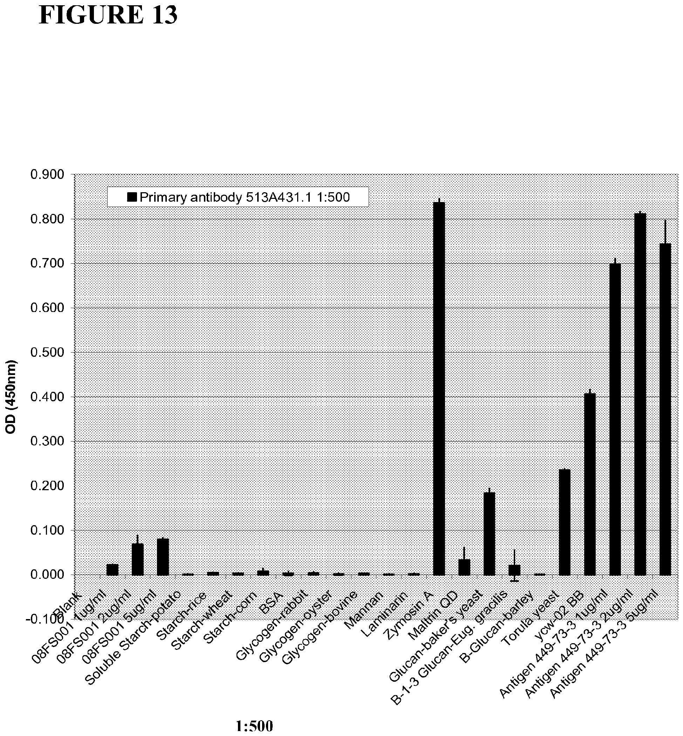

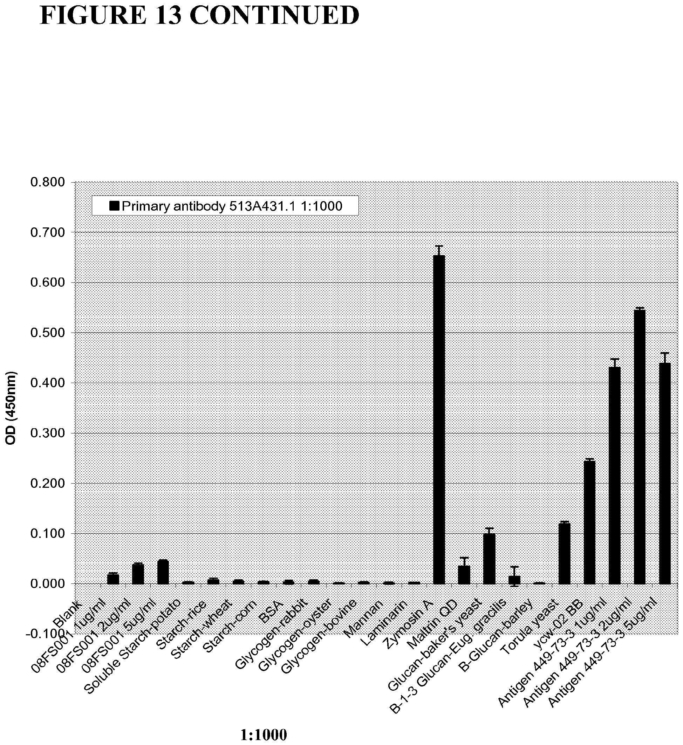

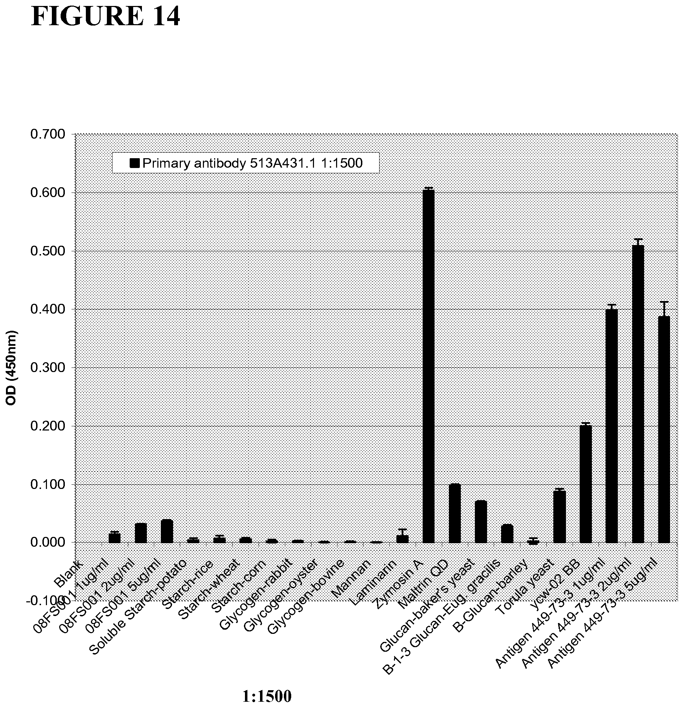

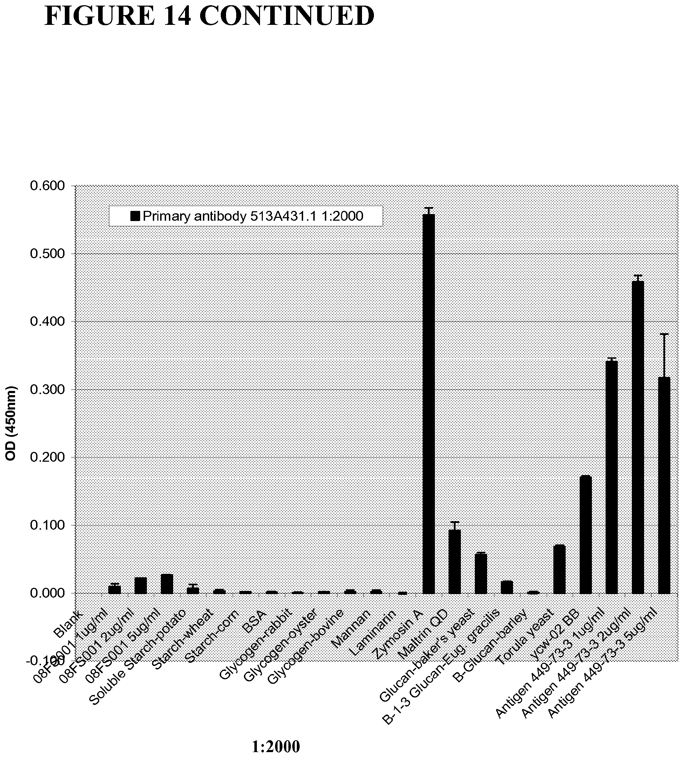

Bar graphs of ELISA data are shown in Figures 9-14. Both 513A161.1 and 513A431.1 reacted very robustly with the antigen 499-73-3 at a concentration as low as 1 μg/ml, with 2 μg/ml producing the strongest absorbance reading. The antibodies were also able to recognize 08FS001 at concentrations ranging from 1 μg/ml to 5 μg/ml, with the strongest absorbance reading at 5 ug/ml. However, the absorbance was significantly less for 08FS001 than for antigen 499-73-3. Antibody 513A161.1 recognized Laminarin and reacted with it significantly, but 513A431.1 did not. Both antibodies had a very strong absorbance reading for Zymosin A which was similar and sometimes stronger than the absorbance seen with the antigen 499-73-3. Surprisingly, the antibodies showed significant cross-reaction with Zymosin A, Maltrin QD, glucan from baker's yeast, (1→3)-β-D-glucan from *Eug. gracilis,* Torula yeast and ycw-02 bead beater pellet, limiting their use for quantitative ELISA assays.

### Example 6

### Attempts to Optimize Monoclonal Antibody ELISA Assay Conditions to Detect Feed-Extracted Antigen

#### Dilution in PBS versus PBS+3% nonfat dry milk

Attempts to use monoclonal antibody 513A161.1 (Example 5) to detect feed-extracted antigen in quantitative ELISA assays resulted in low absorbance readings. To determine whether diluting the antibodies in 3% milk as opposed to PBS affected absorbance readings (e.g., by causing excessive blocking when in combination with feed extract components), the following protocol was performed:

Yeast cell wall extract (MYCOSORB batch #08FS001) extracts was submitted to chemical extraction process as discussed in example 4 by means of a solution containing 0.5% HCl. ELISA assays were conducted as described in Example 5, using monoclonal antibody 513A161 at a dilution of 1:400 as primary antibody and goat anti mouse IgG-HRP antibody (1:10,000) diluted in either PBS or 3% milk as secondary antibody. Microtiter plates were coated with yeast cell wall extract at 100 μl/well or with extract diluted 1:1 in PBS at 200 μl/well.

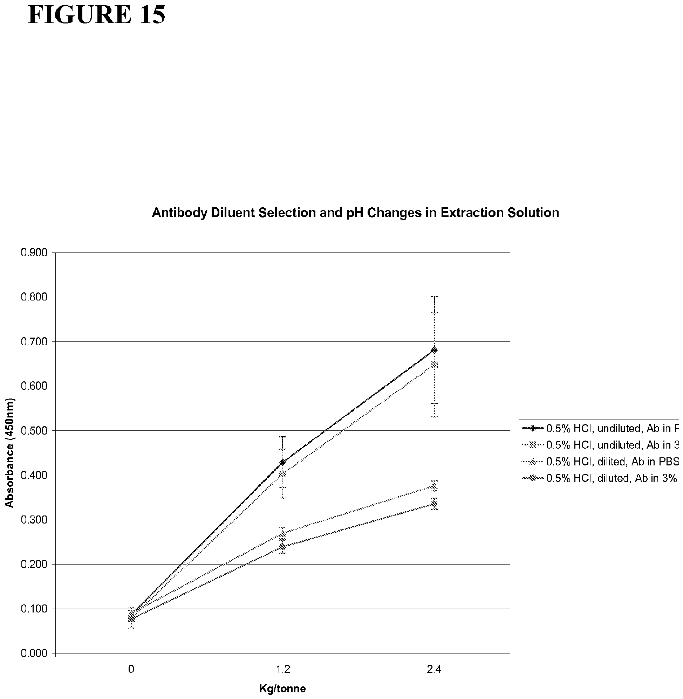

Results shown in Figure 15 established that a slight but statistically insignificant effect of antibody diluents was observed. Additionally, attempts to use the monoclonal antibody to detect antigen in feed standards extracted with 0.5% HCl the triplicates resulted in great variability in readings between replicates, large standard deviations, and inflated absorbance readings.

#### Antigen coating step - temperature and time

Attempts to use monoclonal antibody 513A161.1 (Example 5) to detect feed-cxtractcd antigen in quantitative ELISA assays resulted in low absorbance readings. To increase the absorbance readings of the ELISA using mouse monoclonal antibody 513A161.1, the following experiment was performed to determine whether changing the time and temperature of the antigen coating incubation step would improve feed-extracted antigen detection without increasing background/nonspecific binding.

The protocol consisted of conducting feed extraction as described above to generate analysis samples. The sample aliquots were either coated on the plate immediately or stored overnight at -25°C. Sample preparation was repeated 3 times to generate triplicate extractions. A six-point standard curve (0, 0.4, 0.8, 1.2, 1.8, 2.4 kg/T) was prepared using antigen in chicken feed preparations. Plates were coated with feed extract standard curve and left to incubate at either 4°C overnight (stationary), 4°C overnight (rocking), or 37°C for 1 hour (stationary). ELISA assays were conducted as described in Example 5 using monoclonal antibody 513A161.1 at a dilution of 1:400 prepared in 3% milk as primary antibody and

goat anti mouse IgG-HRP antibody (1:10,000) diluted in 3% milk as secondary antibody. Substrate incubation occurred for 30 minutes, and absorbance at 450 nm was determined.

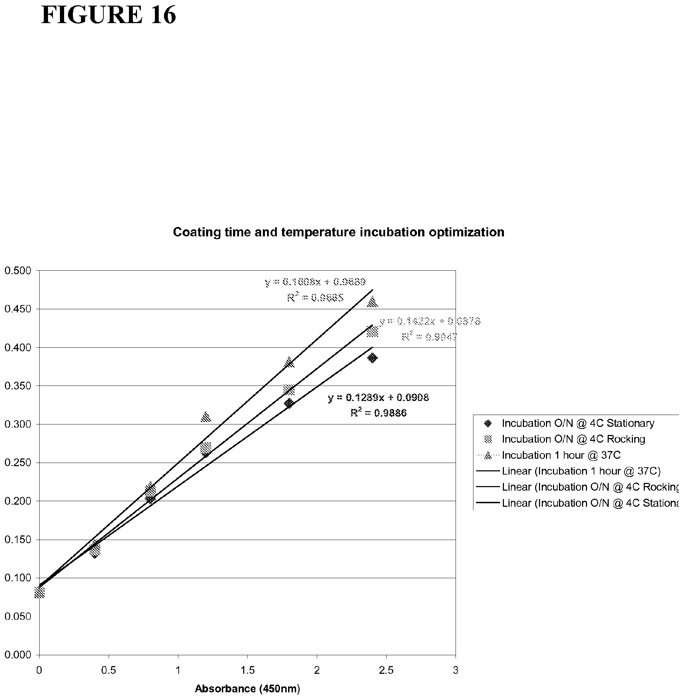

Results shown in Figure 16 indicate that changing the time and temperature at which the antigen was coated onto the plate did not increase the absorbance to an optimal range and also did not affect the linearity of the standard curve. In addition, background was increased independently of incubation time.

**Example 7**

**Polyclonal Antibody Selectivity (Interference) Testing**

In order to determine the degree of interference, a set of assays was performed using the chicken feed material described herein without and with MYCOSORB product (Ref. #285965) present at 1 kg/T, this latter being without or with several proportions of possible interfering products (50%, 100%, 200% compare to MYCOSORB product level of inclusion, w/w) belonging to carbohydrates or side products found in feed formulations. In this respect, the following products have been investigated and were tested to evaluate their impact on the detection of the MYCOSORB product through the herein exact methodology:

- Amylose (potato starch): $(1\rightarrow4){:}(1\rightarrow6){-}\alpha{-}$D-glucans with a ratio 30:1.
- Maltodextrins and corn syrup solids: easily digestible carbohydrates made from natural corn starch, polymer of dextrose.
- Glycogen (from bovine liver, type IX): $(1\rightarrow4){:}(1\rightarrow6){-}\alpha{-}$D-glucans with a ratio 10:1.
- Laminarin (from *Laminaria digitata*): $(1\rightarrow3){:}\beta(1\rightarrow6){-}\beta{-}$D-glucans with a ratio 3:1.
- Distiller Dry Grains; byproducts of the bioethanol production from corn.
- Red Star® Pasteur Champagne Active Dry Wine Yeast (from *Saccharomyces bayanus)*: yeast and yeast cell wall (complex carbohydrates made of $(1\rightarrow3){:}\beta(1\rightarrow6){-}\beta{-}$D-glucans, $(1\rightarrow4){:}(1\rightarrow6){-}\alpha{-}$D-polymannose linked to proteins, $(1\rightarrow2){:}(1\rightarrow4){-}\beta{-}$N-acetylglucosmamine).

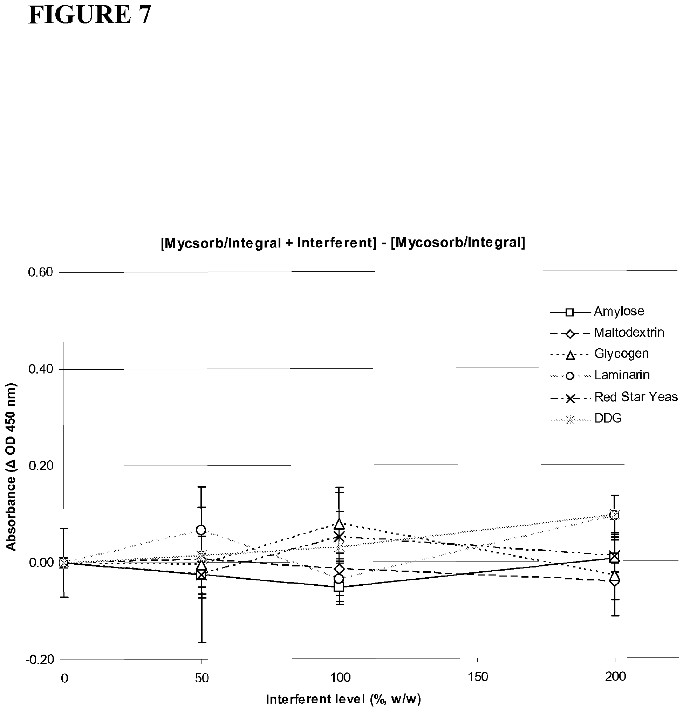

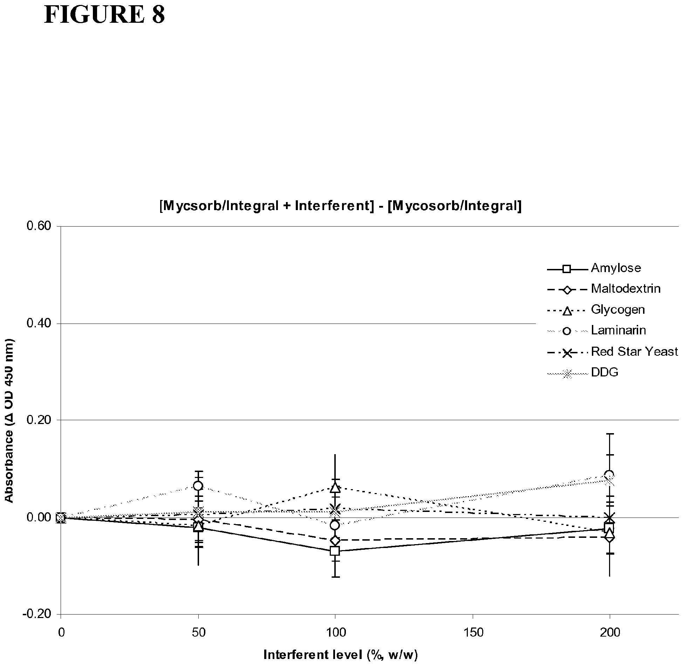

As shown in Figure 7 and Figure 8, the summation of the differences between the signals obtained with the MYCOSORB product and the MYCOSORB product admixed with 50, 100, 200% (w/w) of interferent, were approximately a $OD_{450}$ value of 0. The results account for a lack of interference and thus the specific properties of the assay for the detection of MYCOSORB product in complex feed matrix consisting in different carbohydrates interferent.

Detailed results for each individual interferent and limits of the one-way ANOVA test, for a 95% interval of confidence, are given in Table 39 through Table 44 where the optical density (450 nm) average values and differences between samples were calculated, as well as the standard deviation and coefficient of variation. The homogeneity of the variances within each interferent was evaluated using Levene's, O'Brien's, and Brown and Forsythe tests. In the case of non-constant variance, the nonparametric Kruskal-Willis one-way ANOVA method was used, with a rank transformation that resulted in more robust tests to non-normality, and resistance to outliers. The analyses were performed on separate plates for each concentration of interferent tested or on a single individual plate. Identical results were obtained for the two plate preparations.

Table 39. $OD_{450}$ average values obtained for MYCOSORB product when admixed with 0, 50, 100, 200% (w/w) of amylose in chicken feed. Differences between values were analyzed by means of one-way ANOVA and mean comparison statistic tests with an interval of confidence of 95%.

| MYCOSORB 1 kg/T | | MYCOSORB / Interferent ratios (w/w) | | |
|---|---|---|---|---|
| | | + 50% | + 100% | + 200% |
| ANOVA | | Kruskal-Wallis one-way nonparametric | | |
| | Blank (Feed only) *N* | 24 | 24 | 24 |

(continued)

| MYCOSORB 1 kg/T | | MYCOSORB / Interferent ratios (w/w) | | |
|---|---|---|---|---|
| | | + 50% | + 100% | + 200% |
| ANOVA | | Kruskal-Wallis one-way nonparametric | | |
| Amylose | Mean (OD$_{450}$)<br>Mean Rank<br>$\sigma_{intra}$<br>% CV$_{intra}$ | 0.3317<br>20.396[C]<br>0.0157<br>4.7471 | 0.3538<br>40.479[C]<br>0.0234<br>6.6116 | 0.3638<br>48.625[C]<br>0.0242<br>6.6448 |
| | Control<br>N<br>Mean (OD$_{450}$)<br>Mean Rank<br>$\sigma_{intra}$<br>% CV$_{intra}$ | 24<br>0.6601<br>154.25[AB]<br>0.0348<br>5.2709 | 24<br>0.7073<br>196.48[A]<br>0.0351<br>4.9578 | 24<br>0.6294<br>113.17[B]<br>0.0206<br>3.2661 |
| | Control + Amylose<br>N<br>Mean (OD$_{450}$)<br>Mean Rank<br>$\sigma_{intra}$<br>% CV$_{intra}$ | 24<br>0.6352[B]<br>122.40[B]<br>0.0200<br>3.1418 | 24<br>0.6561[AB]<br>151.13[AB]<br>0.0221<br>3.3738 | 24<br>0.6365[B]<br>129.58[B]<br>0.0346<br>5.4299 |
| | Difference Mean (OD$_{450}$)<br>Difference Mean Rank | -0.0249<br>-31.85 | -0.0512<br>-45.35 | 0.071<br>16.41 |
| | Critical Z value | 3.197 | | |
| | Critical value for comparison | 57.678 | | |

**Table 40.** OD$_{450}$ average values obtained for MYCOSORB product when admixed with 0, 50, 100, 200% (w/w) of maltodextrins in chicken feed. Differences between values were analyzed by means of one-way ANOVA and mean comparison statistic tests with an interval of confidence of 95%.

| MYCOSORB 1 kg/T | | MYCOSORB / Interferent ratios (w/w) | | |
|---|---|---|---|---|
| | | +50% | + 100 % | + 200 % |
| ANOVA | | One-way parametric and Tukey comparison test | | |
| Maltodextrins | Blank (Feed only)<br>N<br>Mean (OD$_{450}$)<br>$\sigma_{intra}$<br>% CV$_{intra}$ | 24<br>0.3628[C]<br>0.0274<br>7.5505 | 24<br>0.3818[B]<br>0.0288<br>7.5414 | 24<br>0.3587[C]<br>0.0367<br>10.218 |
| | Control<br>N<br>Mean (OD$_{450}$)<br>$\sigma_{intra}$<br>% CV$_{intra}$ | 24<br>0.7022[A]<br>0.0276<br>3.9264 | 24<br>0.6426[B]<br>0.0247<br>3.8422 | 24<br>0.6822[A]<br>0.0276<br>4.0480 |
| | Control + Maltodextrins<br>N<br>Mean (OD$_{450}$)<br>$\sigma_{intra}$<br>% CV$_{intra}$ | 24<br>0.7088[A]<br>0.0490<br>6.9145 | 24<br>0.6271[B]<br>0.0449<br>7.1518 | 24<br>0.6407[B]<br>0.0342<br>5.3306 |

(continued)

| MYCOSORB 1 kg/T | | MYCOSORB / Interferent ratios (w/w) | | |
|---|---|---|---|---|
| | | +50% | + 100 % | + 200 % |
| ANOVA | | One-way parametric and Tukey comparison test | | |
| | Difference | 0.0066 | -0.0155 | -0.0415* |
| | Critical value | 0.0308 | | |
| | $\sigma$ for comparison | 0.0099 | | |

**Table 41.** $OD_{450}$ average values obtained for MYCOSORB product when admixed with 0, 50, 100, 200% (w/w) of glycogen in chicken feed. Differences between values were analyzed by means of one-way ANOVA and mean comparison statistic tests with an interval of confidence of 95%.

| MYCOSORB 1 kg/T | | MYCOSORB / Interferent ratios (w/w) | | |
|---|---|---|---|---|
| | | + 50 % | + 100 % | + 200 % |
| ANOVA | | Kruskal-Wallis one-way nonparametric | | |
| Glycogen | Blank (Feed only) N Mean ($OD_{450}$) Mean Rank $\sigma_{intra}$ % $CV_{intra}$ | 24 0.4452 35.604[B] 0.0311 6.9773 | 24 0.4414 33.458[B] 0.0235 5.3259 | 16 0.4495 40.437[B] 0.0270 6.0070 |
| | Control N Mean ($OD_{450}$) Mean Rank $\sigma_{intra}$ % $CV_{intra}$ | 24 0.8522 150.33[A] 0.0610 7.1611 | 24 0.8423 143.69[A] 0.0433 5.1412 | 24 0.8339 137.54[A] 0.0312 3.7443 |
| | Control + Glycogen N Mean ($OD_{450}$) Mean Rank $\sigma_{intra}$ % $CV_{intra}$ | 24 0.8474 149.94[A] 0.0358 4.2302 | 24 0.8367 140.06[A] 0.0576 6.8847 | 24 0.8051 124.58[A] 0.0584 7.2482 |
| | Difference Mean ($OD_{450}$) Difference Mean Rank | -0.0048 0.39 | -0.0056 -3.63 | -0.0288 -12.96 |
| | Critical Z value | 3.197 | | |
| | Critical value for comparison | 55.547 to 62.103 | | |

**Table 42.** OD$_{450}$ average values obtained for MYCOSORB product when admixed with 0, 50, 100, 200% (w/w) of laminarin in chicken feed. Differences between values were analyzed by means of one-way ANOVA and mean comparison statistic tests with an interval of confidence of 95%.

| MYCOSORB **1 kg/T** | | MYCOSORB / **Interferent ratios (w/w)** | | |
| --- | --- | --- | --- | --- |
| | | + 50 % | + 100 % | + 200 % |
| **ANOVA** | | Kruskal-Wallis one-way nonparametric | | |
| **Laminarin** | Blank (Feed only) | | | |
| | $N$ | 24 | 24 | 24 |
| | Mean (OD$_{450}$) | 0.3580 | 0.3478 | 0.3673 |
| | Mean Rank | 37.88[DE] | 31.15[E] | 40.48[DE] |
| | $\sigma_{intra}$ | 0.0428 | 0.0522 | 0.0553 |
| | % CV$_{intra}$ | 11.951 | 14.998 | 15.063 |
| | Control | | | |
| | $N$ | 24 | 24 | 24 |
| | Mean (OD$_{450}$) | 0.5618 | 0.5981 | 0.5224 |
| | Mean Rank | 126.96[BC] | 157.79[AB] | 94.708[CD] |
| | $\sigma_{intra}$ | 0.0488 | 0.0229 | 0.0233 |
| | % CV$_{intra}$ | 8.6916 | 3.8358 | 4.4521 |
| | Control + Laminarin | | | |
| | $N$ | 24 | 24 | 24 |
| | Mean (OD$_{450}$) | 0.6288 | 0.5630 | 0.6155 |
| | Mean Rank | 187.33[A] | 125.50[BC] | 174.21[AB] |
| | $\sigma_{intra}$ | 0.0245 | 0.0341 | 0.0312 |
| | % CV$_{intra}$ | 3.8924 | 6.0501 | 5.0624 |
| | Difference Mean (OD$_{450}$) | -0.0670 | -0.0351 | 0.0931 |
| | Difference Mean Rank | 60.37* | -32.29 | 79.502* |
| | Critical Z value | 3.197 | | |
| | Critical value for comparison | 57.68 | | |

**Table 43 .** OD$_{450}$ average values obtained for MYCOSORB product when admixed with 0, 50, 100, 200% (w/w) of RED STAR wine yeast in chicken feed. Differences between values were analyzed by means of one-way ANOVA and mean comparison statistic tests with an interval of confidence of 95%.

| MYCOSORB **1 kg/T** | | MYCOSORB / **Interferent ratios (w/w)** | | |
| --- | --- | --- | --- | --- |
| | | + 50 % | + 100 % | + 200 % |
| **ANOVA** | | Kruskal-Wallis one-way nonparametric | | |
| | Blank (Feed only) | | | |
| | $N$ | 24 | 24 | 24 |
| | Mean (OD$_{450}$) | 0.3533 | 0.3396 | 0.3490 |
| | Mean Rank | 41.75[B] | 29.90[B] | 37.85[B] |
| | $\sigma_{intra}$ | 0.0240 | 0.0205 | 0.0261 |
| | % CV$_{intra}$ | 6.7991 | 6.0454 | 7.4902 |
| **Wine Yeast** | Control | | | |
| | $N$ | 24 | 24 | 24 |
| | Mean (OD$_{450}$) | 0.6223 | 0.5566 | 0.5846 |
| | Mean Rank | 169.90[A] | 117.38[A] | 135.13[A] |
| | $\sigma_{intra}$ | 0.0534 | 0.0546 | 0.0413 |

(continued)

| MYCOSORB **1 kg/T** | | MYCOSORB / **Interferent ratios (w/w)** | | |
| --- | --- | --- | --- | --- |
| | | + 50 % | + 100 % | + 200 % |
| **ANOVA** | | Kruskal-Wallis one-way nonparametric | | |
| | % $CV_{intra}$ | 8.5726 | 9.8003 | 7.0725 |
| | Control + Red Star® Yeast<br>N<br>Mean (OD$_{450}$)<br>Mean Rank<br>$\sigma_{intra}$<br>% $CV_{intra}$ | 24<br>0.5977<br>145.65[A]<br>0.0257<br>4.2986 | 24<br>0.6098<br>156.17[A]<br>0.0253<br>4.1468 | 24<br>0.5950<br>142.79[A]<br>0.0274<br>4.6038 |
| | Difference Mean (OD$_{450}$)<br>Difference Mean Rank | -0.0246<br>-24.25 | 0.0532<br>38.79 | 0.0104<br>7.66 |
| | Critical Z value | 3.197 | | |
| | Critical value for comparison | 57.68 | | |

**Table 44.** OD$_{450}$ average values obtained for MYCOSORB product when admixed with 0, 50, 100, 200% (w/w) of distilled dry grains in chicken feed. Differences between values were analyzed by means of one-way ANOVA and mean comparison statistic tests with an interval of confidence of 95%.

| MYCOSORB **1 kg/T** | | MYCOSORB / **Interferent ratios (w/w)** | | |
| --- | --- | --- | --- | --- |
| | | + 50 % | + 100 % | + 200 % |
| **ANOVA** | | Kruskal-Wallis one-way nonparametric | | |
| **DDG** | Blank (Feed only)<br>N<br>Mean (OD$_{450}$)<br>Mean Rank<br>$\sigma_{intra}$<br>% $CV_{intra}$ | 24<br>0.3504<br>38.81[C]<br>0.0464<br>13.228 | 24<br>0.3465<br>35.85[C]<br>0.0515<br>14.858 | 24<br>0.3458<br>34.83[C]<br>0.0510<br>14.744 |
| | Control<br>N<br>Mean (OD$_{450}$)<br>Mean Rank<br>$\sigma_{intra}$<br>% $CV_{intra}$ | 24<br>0.8338<br>151.46[AB]<br>0.0411<br>4.9341 | 24<br>0.7825<br>108.19[B]<br>0.0240<br>3.0669 | 24<br>0.7793<br>129.58[AB]<br>0.0934<br>11.990 |
| | Control + DDG<br>N<br>Mean (OD$_{450}$)<br>Mean Rank<br>$\sigma_{intra}$<br>% $CV_{intra}$ | 24<br>0.8476<br>162.73[AB]<br>0.0382<br>4.5019 | 24<br>0.8136<br>134.40[AB]<br>0.0489<br>6.0098 | 24<br>0.8741<br>180.65[A]<br>0.0465<br>5.3191 |
| | Difference | 0.0138<br>11.27 | 0.0311<br>26.21 | 0.0948<br>51.07 |
| | Critical value | 3.197 | | |
| | $\sigma$ for comparison | 57.68 | | |

Various modifications and variations of the described method and system of the invention will be apparent to those skilled in the art Although the invention has been described in connection with specific preferred embodiments, it should be understood that the invention as claimed should not be unduly limited to such specific embodiments. Indeed, various modifications of the described modes for carrying out the invention that are obvious to those skilled in carbohydrate chemistry, microbiology, animal feed and nutrition, immunology, or related fields are intended to be within the scope of the following claims.

**Claims**

1. A method for detecting a yeast cell wall component in a sample, comprising:

   - providing a sample of food or feed selected from the group consisting of livestock feed, companion animal feed, Total Mixed Ration (TMR), forage, feed pellet, feed concentrate, feed premix coproduct, grain, distiller grain, molasses, fiber, fodder, grass, hay, kernel, leaves, meal, soluble feed, and extracts thereof;
   - conducting an assay comprising contacting a primary antibody capable of binding to an antigen selected from the group consisting of $(1\rightarrow4)$-$\alpha$-D-glucan, $(1\rightarrow6)$-$\beta$-D-glucan, and $(1\rightarrow4)$-$\alpha$-/$(1\rightarrow6)$-$\beta$-D-glucan to the sample under conditions such that the primary antibody forms a complex with the antigen, if present; and using a secondary antibody to detect the primary antibody-antigen complex, wherein detecting the primary antibody-antigen complex provides an indication of the presence of the yeast cell wall component in the sample.

2. The method of claim 1, wherein the method comprises obtaining the extract from the sample using organic solvent and acid.

3. The method of claim 1, wherein the method comprises obtaining the extract from the sample using dimethylsulfoxide and hydrochloric acid.

**Patentansprüche**

1. Verfahren zum Nachweisen einer Hefezellwandkomponente in einer Probe, umfassend:

   - das Bereitstellen einer Probe von Nahrungsmittel oder Futter, ausgewählt aus der Gruppe bestehend aus Viehfutter, Haustierfutter, Totaler Mischration (TMR), Futtermittel bzw. Grünfutter, Futterpellet, Futterkonzentrat, Futtervormischungsnebenprodukt, Getreide, Getreideschlempe, Melasse, Faser, Trockenfutter, Gras, Heu, Samenkern bzw. Korn, Blättern, Mehl, löslichem Futter, und Extrakten von diesen;
   - das Durchführen eines Assays, umfassend das Inkontaktbringen eines primären Antikörpers, der an ein Antigen, ausgewählt aus der Gruppe bestehend aus $(1\rightarrow4)$-$\alpha$-D-Glucan, $(1\rightarrow6)$-$\beta$-D-Glucan, $(1\rightarrow4)$-$\alpha$-/$(1\rightarrow6)$-$\beta$-D-Glucan, binden kann, mit der Probe unter solchen Bedingungen, dass der primäre Antikörper einen Komplex mit dem Antigen, falls vorhanden, bildet; und das Verwenden eines zweiten Antikörpers, um den primären Antikörper-Antigen-Komplex nachzuweisen, wobei das Nachweisen des primären Antikörper-Antigen-Komplexes ein Anzeichen für das Vorhandensein der Hefezellwandkomponente in der Probe ergibt.

2. Verfahren nach Anspruch 1, wobei das Verfahren das Erhalten des Extrakts aus der Probe unter Verwendung von organischem Lösungsmittel und Säure umfasst.

3. Verfahren nach Anspruch 1, wobei das Verfahren das Erhalten des Extrakts aus der Probe unter Verwendung von Dimethylsulfoxid und Chlorwasserstoffsäure umfasst.

**Revendications**

1. Procédé de détection d'un composant de paroi cellulaire de levures dans un échantillon, consistant à:

   - fournir un échantillon de nourriture ou d'aliments choisis dans le groupe composé d'aliments pour bétail, d'aliments pour animaux de compagnie, de ration totale mélangée (TMR), de fourrage, de granulés alimentaires, de concentré alimentaire, de coproduit de prémélange alimentaire, de grain, de grain de distillerie, de mélasse, de fibre, de plante fourragère, d'herbe, de foin, de cerneau, de feuilles, de farine, d'aliment soluble et de leurs

EP 2 569 632 B1

extraits;

- réaliser un test comprenant la mise en contact d'un anticorps primaire susceptible de se lier à un antigène choisi dans le groupe composé de (1→4)-α-D-glucane, (1→6)-β-D-glucane, et (1→4)-α-/(1→6)-β-D-glucane à l'échantillon dans des conditions telles que l'anticorps forme un complexe avec l'antigène, s'il est présent; et en utilisant un anticorps secondaire pour détecter le complexe anticorps-antigène primaire, dans lequel la détection du complexe anticorps-antigène primaire fournit une indication de la présence du composant de paroi cellulaire de levures dans l'échantillon.

2. Procédé selon la revendication 1, dans lequel le procédé consiste à obtenir l'extrait de l'échantillon en utilisant un solvant organique et un acide.

3. Procédé selon la revendication 1, dans lequel le procédé consiste à obtenir l'extrait de l'échantillon en utilisant du diméthylsulfoxyde et de l'acide chlorhydrique.

# FIGURE 1

# FIGURE 2

BSA Response

# FIGURE 3

**G Ab 1+2 Response: Standard Curve**

$y = 0.3252x + 0.0414$
$R^2 = 0.9778$

# FIGURE 4

Standard Curve Average Run

$y = 0.2347x$
$R^2 = 0.9787$

Absorbance (DO 450nm)

Inclusion Level (kg/T)

--◇-- Standard    --△-- Between-Day Precision    --○-- Within-Day Precision

# FIGURE 5

Standard Curve Average Run

$y = 0.2199x$
$R^2 = 0.997$

Absorbance (DO 450nm)

Inclusion Level (kg/T)

◇ Standard    △ Between-Day Precision    ○ Within-Day Precision

# FIGURE 6

Standard Curve Average Run

$y = 0.1929x$
$R^2 = 0.9755$

- - ◇ - - Standard   ⋯⋯△⋯⋯ Between-Day Precision   ⋯⋯○⋯⋯ Within-Day Precision

# FIGURE 7

[Mycsorb/Integral + Interferent] - [Mycosorb/Integral]

# FIGURE 8

[Mycsorb/Integral + Interferent] - [Mycosorb/Integral]

# FIGURE 9

1:100

# FIGURE 9 CONTINUED

1:200

# FIGURE 10

# FIGURE 10 CONTINUED

**1:1000**

# FIGURE 11

1:1500

# FIGURE 11 CONTINUED

**1:2000**

# FIGURE 12

**1:100**

# FIGURE 12 CONTINUED

1:200

# FIGURE 13

# FIGURE 13 CONTINUED

# FIGURE 14

# FIGURE 14 CONTINUED

1:2000

# FIGURE 15

Antibody Diluent Selection and pH Changes in Extraction Solution

# FIGURE 16

**Coating time and temperature incubation optimization**

$y = 0.1608x + 0.0688$
$R^2 = 0.9686$

$y = 0.1422x + 0.0878$
$R^2 = 0.9947$

$y = 0.1289x + 0.0908$
$R^2 = 0.9886$

- Incubation O/N @ 4C Stationary
- Incubation O/N @ 4C Rocking
- Incubation 1 hour @ 37C
- Linear (Incubation 1 hour @ 37C)
- Linear (Incubation O/N @ 4C Rocking)
- Linear (Incubation O/N @ 4C Stationa

**Absorbance (450nm)**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 61334995 B
- US 6045834 A
- US 6596861 B
- US 20060263415 A, Sedmak
- WO 9303151 A
- WO 9413804 A

### Non-patent literature cited in the description

- Mycotoxins: Economics and Health Risks. Council for Agricultural Science and Technology. Task Force Report, 1989
- KWIATKOWSKI et al. *Journal of the Institute of Brewing,* 2009, vol. 115 (2), 151-158
- GATHURU et al. *Vaccine,* 2005, vol. 23, 4727-4733
- Current Protocols in Immunology. John Wiley and Sons, Inc, 1998
- Enzyme-Linked Immunosorbent Assay (ELISA). CROWTHER et al. Molecular Biomethods Handbook. Humana Press, Inc, 1998, 595-617
- Antibodies: A Laboratory Manual. Cold Spring Harbor Laboratory Press, 1988
- Current Protocols in Molecular Biology. John Wiley & Sons, Inc, 1994
- LESAGE et al. *Microbiol. Mol. Biol. Rev.,* 2006, vol. 70, 317-343
- KOLLÁR et al. *J. Biol. Chem.,* 1997, vol. 272, 17762-17775
- KWIATKOWSKI et al. *J. Instit. Brew.,* 2009, vol. 115, 1031
- ARVINDEKAR et al. *Yeast,* 2002, vol. 19, 131-139
- PFIZNER et al. *J. Am. Chem. Soc.,* 1963, vol. 85, 3027
- ZEKOVIC et al. *Chem. Papers,* 2006, vol. 60, 243-248
- ABDEL-MAGID et al. *J. Org. Chem.,* 1996, vol. 61, 3849-3862
- HOWARD et al. Basic Methods in Antibody Production and Characterization. CRC Press, 2001, 11-18, 31-50
- MATEJTSCHUK. Affinity Separations: a Practical Approach. Oxford University Press, 1997
- KWIATKOWSKI et al. *J. Instit. Brew.,* 2009, vol. 115, 151-158
- KLIS et al. *Yeast,* 2006, vol. 23, 185-202
- AIMANIADA et al. *J. Biol. Chem.,* 2009, vol. 284, 13401-13412
- KOLLÁR et al. *J. Biol. Chem.,* 1997, vol. 272, 17762-1777
- KLIS et al. *FEMS Microbiology Rev.,* 2002, vol. 26, 239-256
- GUNJA-SMITH et al. *Biochem. Biophys. Res. Com.,* 1974, vol. 56, 588-592
- GRBA et al. *Appl. Microbiol. Biotechnol.,* 1975, vol. 2, 29-37
- GUNJA-SMITH et al. *J. Bacteriol.,* 1977, vol. 130, 818-825
- ROTHMAN-DCNES et al. *PNAS,* 1970, vol. 66, 967-974
- AKLUJKAR et al. *J. Instit. Brew.,* 2008, vol. 114, 205-208
- In: Brewing Yeast and Fermentation. BOULTON et al. The Biochemistry of Fermentation. Blackwell Science, 2001, 89-92
- LILLE et al. *J. Bacteriol.,* 1980, vol. 143, 1384-1394
- FLEET et al. *J. Gen. Microbiol.,* 1976, vol. 94, 180-192
- AIMANIADA et al. *J. Biol. Chem.,* 2009, vol. 184, 13401-13412
- WILD et al. The Immunoassay Handbook. Elsevier Ltd, 2005
- CROWTHER et al. The ELISA Guidebook. Humana Press, 2008
- HOWARD et al. Basic Methods in Antibody Production and Characterization. CRC Press, 2001, 11-18, 31-50
- MATEJTSCHUK. Affinity Separations: A Practical Approach. Oxford University Press, 1997
- LEES et al. *Vaccine,* 1996, vol. 14, 190-198
- PAWLOWSKI et al. *Vaccine,* 1999, vol. 17, 1474-1483
- ZEKOVIC et al. *20060 Chem. Papers,* vol. 60, 243-248
- HAY et al. *Methods Carb. Chem.,* 1973, vol. 5, 357-370
- HARLOW ; LANE. Antibodies; a Laboratory Manual. Cold Springs Harbor Laboratory Press, 1988

- **BURTON.** *Proc. Natl. Acad. Sci.,* 1991, vol. 88, 11120-23
- **THIRION et al.** *Eur. J. Cancer Prev.,* 1996, vol. 5, 507-11
- **COLOMA ; MORRISON.** *Nat. Biotechnol.,* 1997, vol. 15, 159-63
- **MALLENDER ; VOSS.** *J. Biol. Chem.,* 1994, vol. 269, 199-206
- **VERHAAR et al.** *Int. J. Cancer,* 1995, vol. 61, 497-501
- **NICHOLLS et al.** *J. Immunol. Meth.,* 1993, vol. 165, 81-91
- **ORLANDI et al.** *Proc. Natl. Acad. Sci.,* 1989, vol. 86, 3833-3837
- **WINTER et al.** *Nature,* 1991, vol. 349, 293-299
- **KOHLER ; MILSTEIN.** *Nature,* 1975, vol. 256, 495
- **VAUGHAN TJ et al.** *Nature Biotechnology,* 1998, vol. 16, 535
- **DALLIES et al.** *Yeast,* 1998, vol. 14, 1297-1306